# EUROPEAN PATENT APPLICATION

(11) **EP 3 670 500 A1**
(43) Date of publication of application: **24.06.2020**
(21) Application number: 18847012.4
(22) Date of filing: 17.08.2018
(51) Int. Cl.: C07D 239/48, C07D 413/12, C07D 413/14, C07D 498/00, A61K 31/506, A61P 35/00, A61P 29/00, A61P 37/00

(54) **CHEMICAL COMPOUND, PHARMACEUTICAL COMPOSITION THEREOF, AND USE AND APPLICATION THEREOF**

(30) Priority: 18.08.2017 CN 201710713389
(71) Applicant: Beijing Hanmi Pharmaceutical Co., Ltd., Beijing 101312 (CN)
(72) Inventor: ZHAO, Tao, Beijing 101312 (CN); WEI, Dong, Beijing 101312 (CN); LI, Min, Beijing 101312 (CN); KIM, Maeng Sup, Beijing 101312 (CN); CHUNG, Chul Woong, Beijing 101312 (CN)
(74) Representative: Maiwald Patent- und Rechtsanwaltsgesellschaft mbH
(86) International application number: PCT/CN2018/101128
(87) International publication number: WO 2019/034153

(57) **Abstract**

Provided are a pyrimidinyl amino compound having protein kinase inhibitor activity, and a pharmaceutical composition containing said compound; also provided are a use and application of said compound. What is provided is selected from a group consisting of the compound represented by formula (I), a stereoisomer thereof, a tautomer thereof, a pharmacologically acceptable salt thereof, a solvate thereof, and a prodrug thereof. The described compound has good inhibitory activity against the JAK family of kinases and the SYK family of kinases, and therefore may serve as a JAK inhibitor and SYK inhibitor, and is effective in use for the prevention or treatment of diseases related to the JAK and SYK families of kinases.

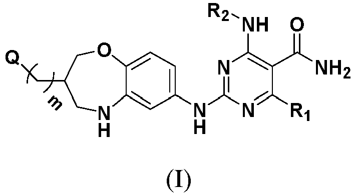

## Description

The present application claims the priority of the Chinese patent application No. 201710713389.8, titled "Chemical Compound, Pharmaceutical Composition Thereof, and Use and Application Thereof' and filed on August 18, 2017 with the Chinese Patent Office, which is incorporated herein by reference in its entirety.

### TECHNICAL FIELD

The present disclosure relates to a pyrimidinylamide compound having a protein kinase inhibitory activity and a pharmaceutical composition containing the same. The present disclosure also relates to the use and application of the compound.

### BACKGROUND OF THE INVENTION

There is a close relationship between cancer, autoimmune disease, and anti-infection-associated disease and dysregulation of protein kinase activity. This is mainly due to mutation or overexpression of the gene that expresses the kinase, or a decrease in the activity of the kinase itself. The existing literatures show that the development of drugs that selectively inhibit kinases will have positive economic and social benefits in the future.

Kinases catalyze the phosphorylation of proteins, lipids, carbohydrates, nucleosides and other cell metabolites and play a key role in all aspects of eukaryotic physiology. Firstly, protein kinases and lipid kinases have an impact on the processes of cell differentiation, activation, and growth in which they are involved; secondly, they play an important role in the survival of cells in the conditions wherein external stimulating factors such as growth factors are involved. In general, protein kinases are divided into two categories: one is biased towards phosphorylation of tyrosine residues; and the other is biased towards phosphorylation of serine or threonine residues. Tyrosine kinases mainly refer to cell membrane growth factor receptors, including endothelial growth factor receptor (EGFR) and intercellular non-receptor kinases such as JAKs and SYK

The JAKs kinase family belongs to an intracellular non-receptor tyrosine kinase series. It was discovered in 1981 and consists of 120-140 kd amino acids. It regulates intracellular signal transduction through the JAK-STAT pathway. The JAKs kinase family plays a vital role in the relevant cellular functions involved in an immune response and in an intracellular factor-dependent regulation and expansion. As far as is known, the JAKs family mainly includes four categories of members, namely JAK1, JAK2, JAK3 and TYK2. JAK1, JAK2 and TYK2 exist in a very wide range, but JAK3 only exists in bone marrow and lymph, etc. Each kinase has a catalytically active region and a falsely active region that must be inactive without catalytic function. Proteins of the JAKs family bind to cell-activating factors through their terminal FERM domain. Through the mediation of intracellular cell-activating factors, they in turn bind to intracellular receptors, thereby exerting their due role. In this way, a signaling molecule finds out an anchoring site, and plays an important role especially in signal transduction and transcription activation of a cell membrane.

With a biological experiment using g a mutant human organelle, it was for the first time discovered that TYK2 has resistant effect to the function of type I interferon (IFNα). A further in vitro experiment showed that the TYK2 kinase is involved in a variety of related cell-activating factor signaling pathways associated with autonomic or adaptive immunity. TYK2 knockout mice showed severe immunodeficiency in the experiment. Surprisingly, TYK2-deficient mice reduced their immune response to interferon IFNα/β, and these intracellular interferons can activate the kinase TYK2 in vitro. Deletion of the TYK2 kinase can result in defects in STAT4 activation and inability of a mouse T-cell to differentiate into a Th1 cell with a TNFr factor. TYK2 interacts with many receptor chains, including tyrosine phosphorylation, and can activate downstream STATs pathways through this pathway, resulting in phosphorylation of recruited STAT homodimers and heterodimers; with the accumulation of phosphorylated products in the nucleus, DNA transcription is caused.

Many cytokines are related to the activation of TYK2, the most important of which are IL-12 and IL-23. These two cytokines share the same subunit p40, and their corresponding receptors are dimers IL-12Rβ1/IL-12β2 and IL-12Rβ1/IL-23R. IL-12Rβ1 binds to TYK2, while IL-12Rβ2 and IL-23R bind to JAK2, and then cooperate with TYK2 to produce physiological functions. In T cells, IL-12 can induce the production of IFNy, and this process is highly dependent on the role of TYK2. Under the synergistic action of TYK2 and JAK2, IL-23 can activate T cells to promote their differentiation into Th17 cells. Therefore, TYK2 plays an important role in the process of regulating the human immune system. More importantly, TYK2 can participate in IFNα/β-mediated signaling pathways, thereby inhibiting the growth of hematopoietic cells, and has an important role in the pathogenesis of blood diseases.

Psoriasis is a common chronic inflammatory skin disease. Ustekinumab, which is clinically used to treat psoriasis, is a monoclonal antibody against IL-12/23 p40, and its effect is better than that of monoclonal antibody etanercept for anti-TNF-a treatment. Therefore, the cell signaling pathway of the IL-12/IL-23 axis mediated by TYK2 kinase is highly relevant to the prevention and treatment of psoriasis. In addition, TYK2 is involved in the pathogenesis of disease in an anti-type II collagen antibody-induced arthritis (CAIA) model in vivo. Hind limbs of wild-type mice swelled with a large amount of erythema and inflammation of joints after injection of a gene antibody, while TYK2-deficient mice did not show any signs of arthritis. Experiments have shown that no inflammatory cell infiltration and articular cartilage lesion are seen in TYK2-deficient mice, and the production of Th1/Th17 pathway-associated cytokines is also reduced in TYK2-deficient mice, and the expression of other inflammatory cytokines and matrix metalloproteinases also did not increase. The results of studies have shown that TYK2 has an important contribution to the development of arthritis.

A JAK1 gene knockout experiment in a mouse model indicates that this enzyme plays a key role in regulating the biological effects of the above-mentioned cytokine receptors.

Knockout of JAK2 in a mouse model can lead to animal death caused by anemia. A base mutation JAK2V617F in the JAK2 gene in humans is closely associated with the occurrence of polycythemia vera (PV), essential thrombocythemia (ET), idiopathic myelofibrosis (IMF), chronic myelogenous leukemia (CML), etc. in myeloproliferative diseases. JAK2 inhibitors have been described as suitable for myeloproliferative diseases.

JAK3 deficiency is identified in people with autosomal recessive severe combined immunodeficiency (SCID) but does not show non-immune defects, suggesting that JAK3 inhibitors as immunosuppressants will have relevant effects in the body and thus become promising drugs for immunosuppression.

Spleen tyrosine kinase (SYK) is an intracellular tyrosine protein kinase that belongs to the ZAP70 protein kinase family. SYK plays a key role in the early development of B cells, the development of individual lymphocytes, and the function of mature B cells. In this process, it participates in a variety of signal transduction pathways and can play a role without the need for phosphorylation of Src kinases. In addition to being universally expressed in hematopoietic stem cells, SYK is expressed in non-hematopoietic cells such as epithelial cells, liver cells, fibroblasts, nerve cells, and breast tissue and has multiple functions. The signal transduction pathway of SYK in B cells can be briefly summarized as follows: B cell receptor (BCR) binds to an antigen, and then activates and phosphorylates the cytoplasmic tail of ITAM motif receptor through LynPTK (belonging to a Src tyrosine kinase family). The subsequent association of phosphorylated ITAM with the SH2 domain of SYK protein leads to the autophosphorylation of SYK. The autophosphorylation of SYK is very important for immune receptor-mediated SYK activation and downstream signaling. The destruction of SYK can prevent most of the downstream signaling of BCR and severely hinder the development of B cells. Dysfunction of SYK or PTK exists in many diseases in humans, such as allergic reaction, asthma, inflammation and autoimmune disease. Numerous studies have shown that SYK is an important mediator of acute or chronic inflammation. SYK activation exists in several common B-cell malignancies. For example, antigen-independent phosphorylated SYK can be detected in follicular lymphoma, diffuse large B-cell lymphoma, mantle cell lymphoma, and B-cell chronic lymphocytic leukemia. Researchers have found that inhibition of SYK in cells of follicular lymphoma and diffuse large B-cell lymphoma can reduce the level of phosphorylation of downstream signaling molecules, thereby inhibiting tumor cell proliferation and survival. In addition, SYK translocations have been found in myelodysplastic syndromes and peripheral T-cell lymphomas, further suggesting that the kinase can act as a proto-oncogene.

### SUMMARY OF THE INVENTION

In view of the above, the present disclosure discloses a pyrimidinylamide compound having a protein kinase inhibitory activity, a pharmaceutical composition containing the compound, and the use and application of the compound. By replacing substituents, a single and multiple kinases can be selectively inhibited to achieve the treatment of corresponding diseases.

In the first aspect, the present disclosure provides a compound selected from the group consisting of a compound represented by the following general formula I, a stereoisomer thereof, a tautomer thereof, a pharmaceutically acceptable salt thereof, a solvate thereof and a prodrug thereof, 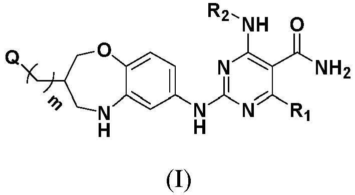 wherein,
Q is selected from hydrogen, carboxyl, cyano, ORₐ, NHR_{b} or SO₂R_{c};
R₁ is selected from hydrogen or C₁-C₃ alkyl;
R₂ is selected from substituted or unsubstituted linear or branched alkyl, substituted or unsubstituted cycloalkyl, substituted or unsubstituted heterocyclyl, substituted or unsubstituted aryl, or substituted or unsubstituted heteroaryl;
wherein the substituent is selected from halogen, trifluoromethyl, cyano, hydroxyl, carboxyl, linear or branched C₁-C₆ alkyl, C₃-C₁₀ cycloalkyl, C₁-C₆ alkoxyalkyl, C₁-C₆ alkylaminoalkyl, C₁-C₆ alkylsulfonylalkyl, C₃-C₈ heterocyclyl, C₆-C₁₂ aryl or C₄-C₁₂ heteroaryl, C₁-C₆ alkylsulfonamidoalkyl, C₁-C₆ alkenylalkyl, or C₁-C₆ alkynylalkyl;
Rₐ, R_{b}, and R_{c} are each independently selected from hydrogen, C₃-C₈ cycloalkyl, trifluoromethyl, trideuterated methyl, linear or branched C₁-C₆ alkyl, C₁-C₆ alkylaminoalkyl, C₁-C₆ alkoxyalkyl, C₃-C₈ heterocyclylalkyl, C₁-C₆ alkoxycarbonylalkyl, C₁-C₆ alkylaminocarbonylalkyl, C₁-C₆ hydroxyalkyl, or C₁-C₆ carboxylalkyl;
m is 0 or 1.

In the second aspect, the present disclosure provides a pharmaceutical composition comprising the compound of the first aspect and pharmaceutically acceptable excipient(s).

In the third aspect, the present disclosure provides a use of the compound of the first aspect as an inhibitor of TYK2 kinase inhibitor, a JAK1 kinase inhibitor, a JAK2 kinase inhibitor, a JAK3 kinase inhibitor, or a SYK kinase.

In the fourth aspect, the present disclosure provides a use of the compound of the first aspect in the manufacture of a medicament for prevention or treatment of a disease associated with TYK2 kinase, JAK1 kinase, JAK2 kinase, JAK3 kinase, or SYK kinase.

In the fifth aspect, the present disclosure provides a method for treating a disease by administering the compound of the first aspect or the pharmaceutical composition of the second aspect to a patient to inhibit the activity of TYK2 kinase, JAK1 kinase, JAK2 kinase, JAK3 kinase, or SYK kinase.

The technical solution disclosed herein at least has the following beneficial effects: the novel pyrimidinylamide compound disclosed herein has good inhibitory activity against TYK2 kinase, JAK1 kinase, JAK2 kinase, JAK3 kinase, or SYK kinase, and thus can be used as a TYK2 kinase inhibitor, a JAK1 kinase inhibitor, a JAK2 kinase inhibitor, a JAK3 kinase inhibitor, or a SYK kinase inhibitor, thereby effectively preventing or treating a disease associated with TYK2 kinase, JAK1 kinase, JAK2 kinase, JAK3 kinase, or SYK kinase.

### DETAILED DESCRIPTION OF THE EMBODIMENTS

Unless defined otherwise, all technical and scientific terms used herein have the same meaning as commonly understood by one of ordinary skill in the art to which the subject matter of the claims pertains.

It should be understood that the foregoing brief description and the following detailed description are exemplary and are intended for illustration purpose only, without placing any limitation on the subject matter disclosed herein.

Some chemical groups defined herein are preceded by a simplified symbol to indicate the total number of carbon atoms or ring atoms present in the group. For example, C₁-C₆ alkyl refers to an alkyl group, as defined below, having a total of 1 to 6 carbon atoms; and C₃-C₁₀ cycloalkyl refers to a cycloalkyl group, as defined below, having a total of 3 to 10 carbon atoms; in the following, "membered" as used herein refers to the number of ring atoms of a group, for example, "5-membered ring" means that the number of ring atoms is five.

Unless otherwise specified in this specification, all combined groups described in the present disclosure (that is, a group combined by two or more groups) are connected to the rest of the molecule through the last described group as a connection site. For example, the group "alkylaminoalkyl" means that an alkylamino group is connected to the rest of the molecule through an alkyl group; the group "alkoxyalkyl" means that an alkoxy group is connected to the rest of the molecule through an alkyl group, etc.

In addition to the foregoing, when used in the description and claims of the present disclosure, the following terms have the following meanings unless specifically stated otherwise:
"Amino" refers to a -NH₂ group.
"Cyano" refers to a -CN group.
"Hydroxy" refers to a -OH group.
"Acyl" refers to a -C(=O)- group.
"Carboxyl" refers to a -C(=O)OH group.
"Sulfonyl" refers to a -S(=O)₂- group.
"Aminoacyl" refers to a -C(=O)-NH₂ group.

In the present disclosure, the term "halogen" refers to fluorine, chlorine, bromine and iodine, preferably fluorine.

In the present disclosure, as a separate group or part of another group, the term "alkyl" refers to a linear or branched group consisting only of carbon and hydrogen atoms, containing no unsaturated bonds, and connecting to the rest of the molecule through a single bond. Alkyl may have, for example, 1 to 18, preferably 1 to 8, more preferably 1 to 6, more preferably 1 to 4 carbon atoms. Examples of alkyl include, but are not limited to, methyl, ethyl, propyl, isopropyl, n-butyl, isobutyl, sec-butyl, tert-butyl, n-pentyl, n-hexyl, 2-pentyl, heptyl, 2-methylhexyl, 3-methylhexyl, octyl, nonyl, decyl, etc., preferably methyl, ethyl, and isopropyl. The hydrogen on an alkyl group can optionally be replaced by any suitable group, such as halogen, hydroxyl, amino, mono-substituted amino, di-substituted amino, alkoxy, aminoacyl, heterocyclyl, etc.

In the present disclosure, as a separate group or part of another group, the term "alkoxyalkyl" means an alkoxy group that is connected to the rest of the molecule through an alkyl group. Alkylaminocarbonylalkyl means an alkylaminocarbonyl group that is connected to the rest of the molecule through an alkyl group.

In the present disclosure, as a separate group or part of another group, the term "heterocyclyl" refers to a stable 3- to 18-membered non-aromatic cyclic group consisting of 2 to 12 carbon atoms and 1 to 6 heteroatoms selected from nitrogen, oxygen, and sulfur. Unless otherwise specified in this specification, a heterocyclyl group may be a monocyclic, bicyclic, tricyclic or more cyclic ring system, which may include a fused ring system or a bridged ring system. For the purpose of the present disclosure, a heterocyclyl group is preferably a stable 3- to 8-membered non-aromatic monocyclic or bicyclic group containing 1 to 3 heteroatoms selected from nitrogen, oxygen, and sulfur, more preferably a stable 5- to 8-membered non-aromatic monocyclic group containing 1 to 3 heteroatoms selected from nitrogen, oxygen, and sulfur, and more preferably a stable 5-membered to 6-membered non-aromatic monocyclic group containing 1 to 2 heteroatoms selected from nitrogen, oxygen, and sulfur. The nitrogen, carbon or sulfur atom in the heterocyclyl group may be optionally oxidized; the nitrogen atom may be optionally quaternized; and the heterocyclyl group can be partially or fully saturated. A heterocyclyl group can be connected to the rest of the molecule via a carbon atom or a heteroatom with a single bond. In a heterocyclyl group containing a fused ring, one or more rings may be aryl or heteroaryl, provided that the point of attachment to the rest of the molecule is a non-aromatic ring atom. Examples of a heterocyclyl group include, but are not limited to: pyranyl, tetrahydropyranyl, thiopyranyl, tetrahydrofuranyl, morpholinyl, thiomorpholinyl, piperazinyl, piperidinyl, oxazinyl, dioxolanyl, tetrahydroisoquinolinyl, decahydroisoquinolinyl, imidazolinyl, imidazolidinyl, quinolizinyl, thiazolidinyl, isothiazolidinyl, isoxazolidinyl, dihydroindolyl, octahydroindolyl, octahydroisoindolyl, pyrrolidinyl, pyrazolidinyl, phthalimidyl, etc, preferably tetrahydrofuranyl, tetrahydropyranyl, morpholinyl, piperazinyl, piperidinyl, and pyrrolidinyl, and more preferably tetrahydropyranyl, morpholinyl, and piperidinyl. The hydrogen in a heterocyclyl group can be optionally replaced by any suitable group, such as halogen, hydroxyl, amino, mono-substituted amino, di-substituted amino, alkyl, haloalkyl, alkoxy, cycloalkyl, heterocyclyl, alkylcarbonyl, aminoacyl, etc.

In the present disclosure, as a separate group or part of another group, the term "aryl" refers to a system having 6 to 18 (preferably 6 to 10) carbon atoms and at least one aromatic ring. For the purpose of the present disclosure, an aryl group may be a monocyclic, bicyclic, tricyclic, or more cyclic ring system, which can include a fused or bridged ring system. An aryl group is connected to the rest of the molecule with a single bond via an aromatic ring atom. An aryl group can be substituted at any suitable position with one or more substituents selected from halogen, hydroxyl, amino, alkyl, cycloalkyl, haloalkyl, hydroxyalkyl, alkoxy, heterocyclyl, aryl, heteroaryl, substituted aminoacyl, heterocyclylalkylacyl, heterocyclylcarbonyl, etc. Examples of aryl include, but are not limited to, phenyl, naphthalenyl, anthryl, phenanthryl, fluorenyl, 2-benzoxazolinone, 2H-1,4-benzoxazin-3(4H)-on-7-yl, etc., preferably phenyl.

In the present disclosure, as a group or part of another group, the term "heteroaryl" refers to a 5- to 16-membered ring system group having 1 to 4 heteroatoms selected from nitrogen, oxygen, and sulfur in the ring, and at least one aromatic ring. Unless specifically stated otherwise in this specification, a heteroaryl group may be a monocyclic, bicyclic, tricyclic, or more cyclic ring system, which may include a fused ring system or a bridged ring system, provided that the point of attachment is an aromatic ring atom. The nitrogen, carbon or sulfur atom in the heteroaryl group can be optionally oxidized; and the nitrogen atom can be optionally quaternized. For the purpose of the present disclosure, a heteroaryl group is preferably a stable 5- to 10-membered aromatic monocyclic or bicyclic group containing 1 to 3 heteroatoms selected from nitrogen, oxygen, and sulfur, and more preferably a stable 5- to 9-membered aromatic monocyclic group containing 1 to 2 heteroatoms selected from nitrogen, oxygen, and sulfur. Examples of heteroaryl include, but are not limited to, thienyl, furyl, pyrrolyl, imidazolyl, benzimidazolyl, pyrazolyl, benzopyrazolyl, triazolyl, tetrazolyl, pyridyl, pyrazinyl, triazinyl, pyrimidinyl, pyridazinyl, indazinyl, indolyl, isoindolyl, indazolyl, isoindazolyl, purinyl, quinolinyl, isoquinolinyl, diazanaphthalenyl, naphthyridinyl, quinoxalinyl, pteridinyl, carbazolyl, carbolinyl, phenanthridinyl, phenanthrolinyl, acridinyl, phenazinyl, thiazolyl, isothiazolyl, benzothiazolyl, thiophenyl, oxazolyl, isoxazolyl, oxadiazolyl, oxatriazolyl, cinnolinyl, quinazolinyl, phenylsulfenyl, indolizinyl, o-phenanthrolinyl, phenoxazinyl, phenothiazinyl, 4,5,6,7-tetrahydrobenzo[b]thienyl, naphthopyridyl, imidazolo[1,2-a]pyridyl, etc., preferably pyrazolyl, pyridyl, pyrimidinyl, isoxazolyl, isothiazolyl, thienyl, and indazolyl, and more preferably pyrazolyl, pyridyl, isothiazolyl, and indazolyl. A heteroaryl group can be substituted at any suitable position with one or more substituents selected from halogen, hydroxyl, amino, alkyl, cycloalkyl, haloalkyl, hydroxyalkyl, alkoxy, heterocyclyl, aryl, heteroaryl, substituted or unsubstituted aminoacyl, heterocyclylalkylacyl, heterocyclylcarbonyl, etc.

In the present disclosure, a "stereoisomer" refers to a compound that consists of the same atoms and bonded by the same bond, but has a different three-dimensional structure. A stereoisomer includes an enantiomer and a diastereomer, in which the diastereomer includes a cis-trans isomer (i.e., a geometric isomer) and a conformer. The present disclosure covers various stereoisomers and mixtures thereof.

A "tautomer" refers to an isomer formed by the transfer of a proton from one atom of a molecule to another atom of the same molecule. All tautomeric forms of a compound of formula I disclosed herein are also included in the scope of the present disclosure.

In the present disclosure, the term "pharmaceutically acceptable salt" includes a pharmaceutically acceptable acid addition salt and a pharmaceutically acceptable base addition salt.

A "pharmaceutically acceptable acid addition salt" refers to a salt formed with an inorganic or organic acid that is capable of retaining the biological effectiveness of a free base without other side effects. The inorganic acid includes, but is not limited to, hydrochloric acid, hydrobromic acid, sulfuric acid, nitric acid, phosphoric acid, etc.; the organic acid includes, but is not limited to, formic acid, acetic acid, trifluoroacetic acid, propionic acid, caprylic acid, caproic acid, capric acid, undecylenic acid, glycolic acid, gluconic acid, lactic acid, oxalic acid, sebacic acid, adipic acid, glutaric acid, malonic acid, maleic acid, succinic acid, fumaric acid, tartaric acid, citric acid, palmitic acid, stearic acid, oleic acid, cinnamic acid, lauric acid, malic acid, glutamic acid, pyroglutamic acid, aspartic acid, benzoic acid, methanesulfonic acid, p-toluenesulfonic acid, alginic acid, ascorbic acid, salicylic acid, 4-aminosalicylic acid, naphthalene disulfonic acid, etc. These salts can be prepared by methods known in the art.

A "pharmaceutically acceptable base addition salt" refers to a salt that is capable of maintaining the biological effectiveness of a free acid without other side effects. These salts are prepared by adding an inorganic or organic base to a free acid. A salt derived from an inorganic base includes, but is not limited to, salt of sodium, potassium, lithium, ammonium, calcium, magnesium, iron, zinc, copper, manganese, and aluminum, etc. A preferred inorganic salt is ammonium, sodium salt, potassium salt, calcium salt, and magnesium salt. A salt derived from an organic base includes, but is not limited to, a salt of the following base: primary, secondary, and tertiary amines, substituted amines, including natural substituted amines, cyclic amines and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, choline, betaine, ethylenediamine, glucosamine, methylglucosamine, theobromine, trometamol, purine, piperazine, piperidine, N-ethylpiperidine, polyamine resin, etc.

Depending on the number of charged functional groups and the valence of a cation or anion, the compound disclosed herein may contain multiple cations or anions.

Generally, crystallization results in a solvate of a compound disclosed herein. In the present disclosure, a "solvate" refers to an aggregate comprising one or more molecules of a compound disclosed herein and one or more solvent molecules. They are either reacted in the solvent, or precipitated or crystallized from the solvent. The solvent may be water, and the solvate in this case is a hydrate. Alternatively, the solvent may be an organic solvent. A solvate of the compound disclosed herein is also within the scope disclosed herein.

In the present disclosure, a "pharmaceutical composition" refers to a preparation of a compound disclosed herein and a medium generally accepted in the art for delivering a biologically active compound to a mammal (e.g., a human). The medium includes a pharmaceutically acceptable excipient. A pharmaceutical composition disclosed herein may be a single preparation or a combination of multiple preparations.

In the present disclosure, a "pharmaceutically acceptable excipient" includes, but is not limited to, any adjuvant, carrier, auxiliary, glidant, sweetener, diluent, preservative, dye/colorant, flavoring agent, surfactant, wetting agent, dispersant, suspending agent, stabilizer, isotonizing agent, solvent or emulsifier that is approved by the relevant governmental authority as acceptable for human or livestock use.

The compounds of the first aspect in embodiments disclosed herein are specifically described below.

The compound in examples disclosed herein is selected from the group consisting of a compound represented by the following general formula I, a stereoisomer thereof, a tautomer thereof, a pharmaceutically acceptable salt thereof, a solvate thereof, and a prodrug thereof, 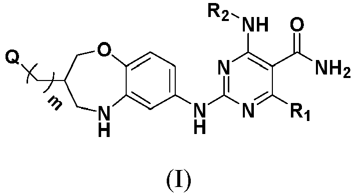 wherein,
Q is selected from carboxyl, cyano, ORₐ, NHR_{b} or SO₂R_{c};
R₁ is selected from hydrogen or C₁-C₃ alkyl;
R₂ is selected from substituted or unsubstituted linear or branched alkyl, substituted or unsubstituted cycloalkyl, substituted or unsubstituted heterocyclyl, substituted or unsubstituted aryl, or substituted or unsubstituted heteroaryl;
wherein the substituent is selected from halogen, trifluoromethyl, cyano, hydroxyl, carboxyl, linear or branched C₁-C₆ alkyl, C₃-C₁₀ cycloalkyl, C₁-C₆ alkoxyalkyl, C₁-C₆ alkylaminoalkyl, C₁-C₆ alkylsulfonylalkyl, C₃-C₈ heterocyclyl, C₆-C₁₂ aryl or C₄-C₁₂ heteroaryl;
Rₐ, R_{b}, and R_{c} are each independently selected from hydrogen, linear or branched C₁-C₄ alkyl, or C₃-C₈ cycloalkyl;
m is 0 or 1.

In some embodiments of the compound described herein, Q is selected from ORₐ, or NHR_{b};
R₁ is selected from hydrogen or C₁-C₃ alkyl;
R₂ is selected from substituted or unsubstituted linear or branched C₁-C₄ alkyl, substituted or unsubstituted C₃-C₁₀ cycloalkyl, substituted or unsubstituted C₃-C₆ heterocyclyl, substituted or unsubstituted C₆-C₁₀ aryl, or substituted or unsubstituted C₄-C₁₀ heteroaryl; wherein the substituent is selected from halogen, trifluoromethyl, cyano, hydroxyl, carboxyl, linear or branched C₁-C₆ alkyl, C₃-C₁₀ cycloalkyl, C₁-C₆ alkoxyalkyl, C₁-C₆ alkylaminoalkyl, C₁-C₆ alkylsulfonylalkyl, C₃-C₈ heterocyclyl, C₆-C₁₀ aryl or C₄-C₁₀ heteroaryl;
Rₐ, and R_{b} are each independently selected from hydrogen, linear or branched C₁-C₄ alkyl, or C₃-C₆ cycloalkyl;
m is 0 or 1.

In some further embodiments of the compound disclosed herein, Q is selected from ORₐ, or NHR_{b};
Rₐ, and R_{b} are each independently selected from hydrogen, linear or branched C₁-C₄ alkyl, or C₃-C₆ cycloalkyl; R₁ is selected from hydrogen or methyl; m is 0.

In some embodiments of the compound disclosed herein, the compound has a structure represented by formula (I): 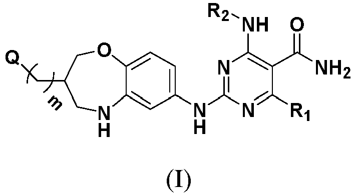
wherein Q is selected from hydrogen, carboxyl, cyano, ORₐ, NHR_{b}, or SO₂R_{c};
R₁ is selected from hydrogen or C₁-C₃ alkyl;
R₂ is selected from substituted or unsubstituted linear or branched alkyl, substituted or unsubstituted cycloalkyl, substituted or unsubstituted heterocyclyl, substituted or unsubstituted aryl, or substituted or unsubstituted heteroaryl;
wherein the substituent is selected from halogen, trifluoromethyl, cyano, hydroxyl, carboxyl, linear or branched C₁-C₆ alkyl, C₃-C₁₀ cycloalkyl, C₁-C₆ alkoxyalkyl, C₁-C₆ alkylaminoalkyl, C₁-C₆ alkylsulfonylalkyl, C₃-C₈ heterocyclyl, C₆-C₁₂ aryl or C₄-C₁₂ heteroaryl, C₁-C₆ alkylsulfonylaminoalkyl, C₁-C₆ alkenylalkyl, or C₁-C₆ alkynylalkyl;
Rₐ, R_{b}, and R_{c} are each independently selected from hydrogen, C₃-C₈ cycloalkyl, trifluoromethyl, trideuterated methyl, linear or branched C₁-C₆ alkyl, C₁-C₆ alkylaminoalkyl, C₁-C₆ alkoxyalkyl, C₃-C₈ heterocyclylalkyl, C₁-C₆ alkoxycarbonylalkyl, C₁-C₆ hydroxyalkyl, or C₁-C₆ carboxylalkyl;
m is 0 or 1.

In some embodiments of the compound described herein, Q is selected from hydrogen, ORₐ, or NHR_{b};
R₁ is selected from hydrogen or C₁-C₃ alkyl;
R₂ is selected from substituted or unsubstituted linear or branched C₁-C₄ alkyl, substituted or unsubstituted C₃-C₁₀ cycloalkyl, substituted or unsubstituted C₃-C₆ heterocyclyl, substituted or unsubstituted C₆-C₁₀ aryl, or substituted or unsubstituted C₄-C₁₀ heteroaryl; wherein a substituent is selected from halogen, trifluoromethyl, cyano, hydroxyl, carboxyl, linear or branched C₁-C₆ alkyl, C₃-C₁₀ cycloalkyl, C₁-C₆ alkoxyalkyl, C₁-C₆ alkylaminoalkyl, C₁-C₆ alkylsulfonylalkyl, C₃-C₈ heterocyclyl, C₆-C₁₀ aryl or C₄-C₁₀ heteroaryl, C₁-C₆ alkylsulfonylaminoalkyl, C₁-C₆ alkenylalkyl, or C₁-C₆ alkynylalkyl;
Rₐ and R_{b} are each independently selected from hydrogen, C₃-C₆ cycloalkyl, trifluoromethyl, trideuterated methyl, linear or branched C₁-C₆ alkyl, C₁-C₆ alkylaminoalkyl, C₁-C₆ alkoxyalkyl, C₃-C₈ heterocyclylalkyl, C₁-C₆ alkoxycarbonylalkyl, C₁-C₆ alkylaminocarbonylalkyl, C₁-C₆ hydroxyalkyl, or C₁-C₆ carboxylalkyl; m is 0 or 1.

In a further embodiment of the compound disclosed herein, Q is selected from hydrogen, ORₐ, or NHR_{b};
Rₐ and R_{b} are each independently selected from hydrogen, C₃-C₆ cycloalkyl, trifluoromethyl, trideuterated methyl, linear or branched C₁-C₆ alkyl, C₁-C₆ alkylaminoalkyl, C₁-C₆ alkoxyalkyl, C₃-C₈ heterocyclylalkyl, C₁-C₆ alkoxycarbonylalkyl, C₁-C₆ alkylaminocarbonylalkyl, C₁-C₆ hydroxyalkyl, or C₁-C₆ carboxylalkyl;
R₁ is selected from hydrogen or methyl; m is 0.

In some embodiments of the compound described herein, in the general formula I: Q is selected from hydrogen, carboxyl, cyano, ORₐ, NHR_{b}, or SO₂R_{c}; further optionally, Q is selected from hydrogen, ORₐ, or NHR_{b};
Rₐ, R_{b}, and R_{c} are each independently selected from hydrogen, C₃-C₈ cycloalkyl, trifluoromethyl, trideuterated methyl, linear or branched C₁-C₆ alkyl, C₁-C₆ alkylaminoalkyl, C₁-C₆ alkoxyalkyl, C₃-C₈ heterocyclylalkyl, C₁-C₆ alkoxycarbonylalkyl, C₁-C₆ hydroxyalkyl, or C₁-C₆ carboxylalkyl;
In some embodiments of the compound described herein, in the general formula I: R₁ is selected from hydrogen or C₁-C₃ alkyl;
in the general formula I: R₂ is selected from substituted or unsubstituted linear or branched alkyl, substituted or unsubstituted cycloalkyl, substituted or unsubstituted heterocyclyl, substituted or unsubstituted aryl, or substituted or unsubstituted heteroaryl; wherein the substituent is selected from halogen, trifluoromethyl, cyano, hydroxyl, carboxyl, linear or branched C₁-C₆ alkyl, C₃-C₁₀ cycloalkyl, C₁-C₆ alkoxyalkyl, C₁-C₆ alkylaminoalkyl, C₁-C₆ alkylsulfonylalkyl, C₃-C₈ heterocyclyl, C₆-C₁₂ aryl or C₄-C₁₂ heteroaryl, C₁-C₆ alkylsulfonylaminoalkyl, C₁-C₆ alkenylalkyl, or C₁-C₆ alkynylalkyl.

Further optionally, R₂ is selected from substituted or unsubstituted linear or branched C₁-C₄ alkyl, substituted or unsubstituted C₃-C₁₀ cycloalkyl, substituted or unsubstituted C₃-C₆ heterocyclyl, substituted or unsubstituted C₆-C₁₀ aryl, or substituted or unsubstituted C₄-C₁₀ heteroaryl; wherein the substituent is selected from halogen, trifluoromethyl, cyano, hydroxyl, carboxyl, linear or branched C₁-C₆ alkyl, C₃-C₁₀ cycloalkyl, C₁-C₆ alkoxyalkyl, C₁-C₆ alkylaminoalkyl, C₁-C₆ alkylsulfonylalkyl, C₃-C₈ heterocyclyl, C₆-C₁₀ aryl or C₄-C₁₀ heteroaryl, C₁-C₆ alkylsulfonylaminoalkyl, C₁-C₆ alkenylalkyl, or C₁-C₆ alkynylalkyl.

Further optionally, R₂ may be selected from the following groups: 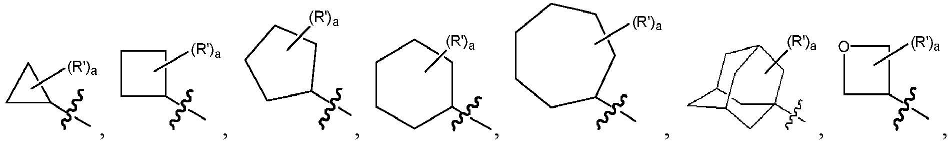 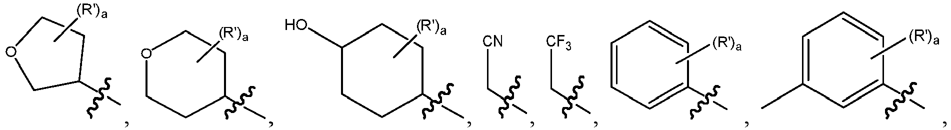 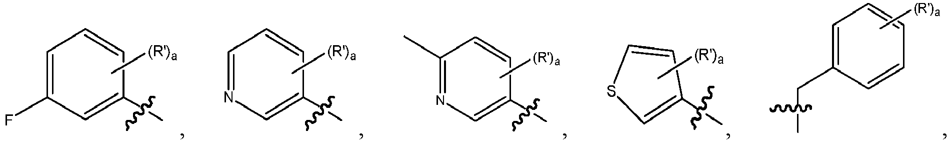 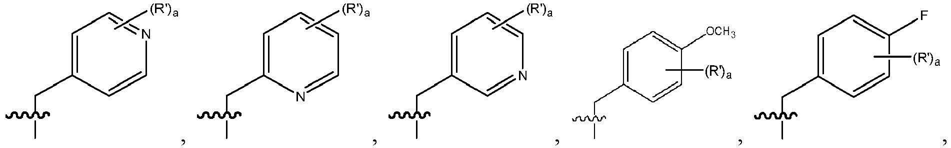 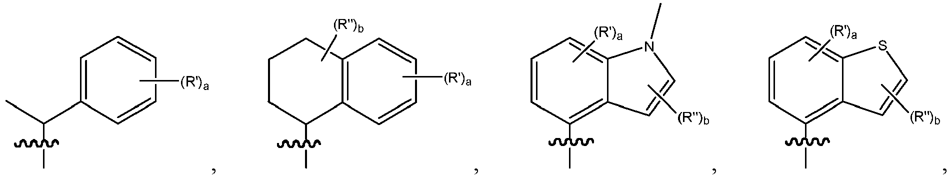 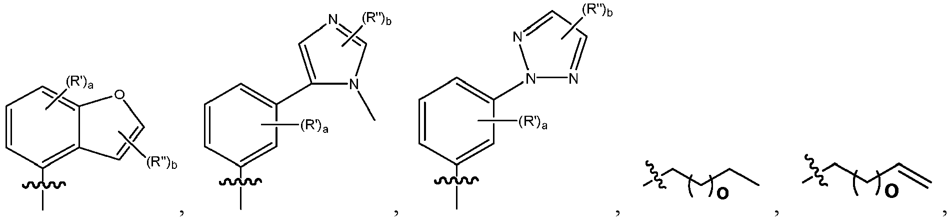 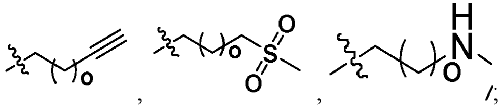
R', and R" are each independently selected from hydrogen, halogen, trifluoromethyl, cyano, hydroxyl, carboxyl, linear or branched C₁-C₆ alkyl, C₃-C₁₀ cycloalkyl, C₁-C₆ alkoxy, C₁-C₆ alkylamino, C₁-C₆ alkylsulfonyl, C₃-C₈ heterocyclyl, C₆-C₁₀ aryl or C₄-C₁₀ heteroaryl;
a is an integer from 1 to 5, b is an integer from 1 to 5, and o is an integer from 1 to 5;
wherein the specific values of a and b can be selected according to substitutable sites.

Further optionally, when the substituent is a hydrogen atom, R₂ may be selected from the following groups:

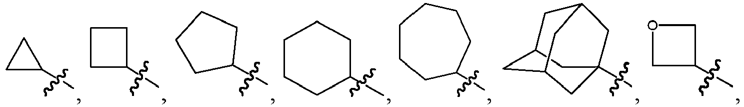

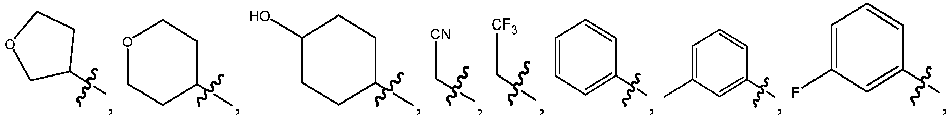

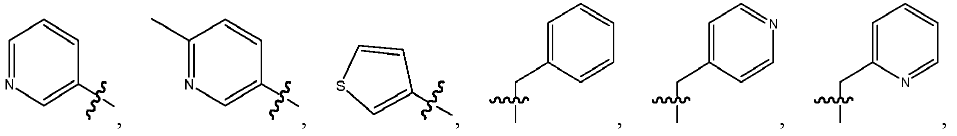

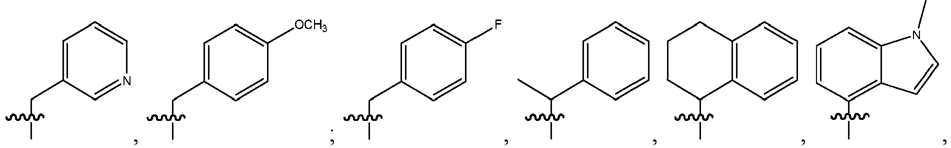

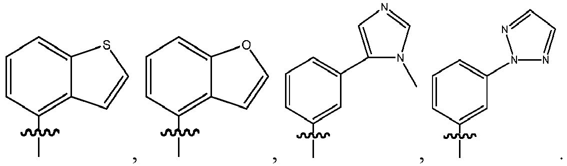

At least one substituent may be connected to the above substituents.

In the general formula I, m is 0 or 1; depending on the value of m, the substituent group 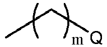 can be -Q or -CH₂-Q.

Further optionally, in some embodiments of the compound described herein, R₁ is hydrogen.

In some embodiments of the compound disclosed herein, when R₁ is hydrogen, the compound in examples disclosed herein is selected from the group consisting of compounds represented by the following structural formulae:

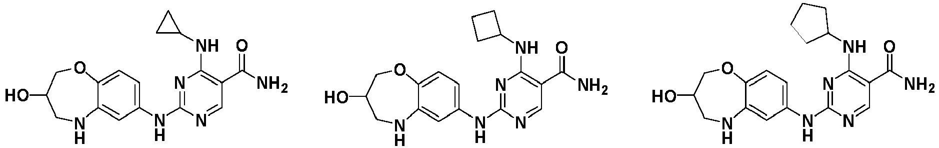

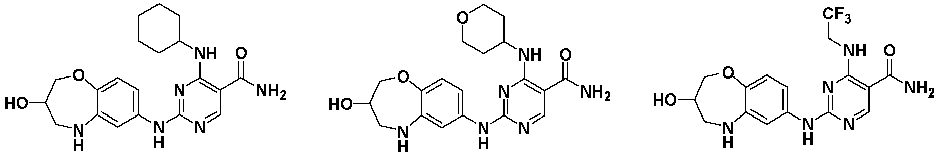

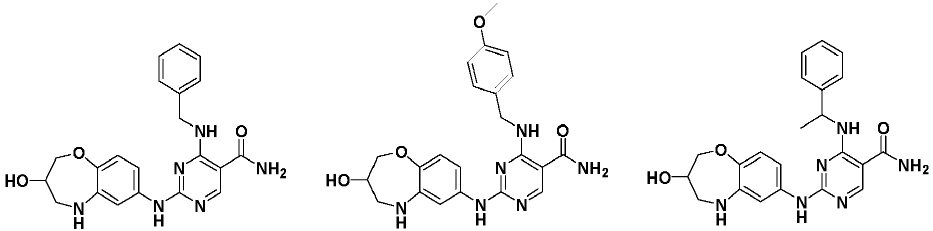

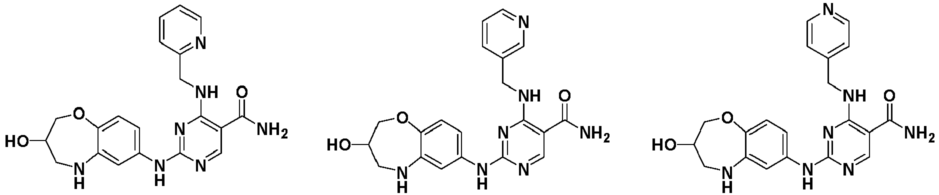

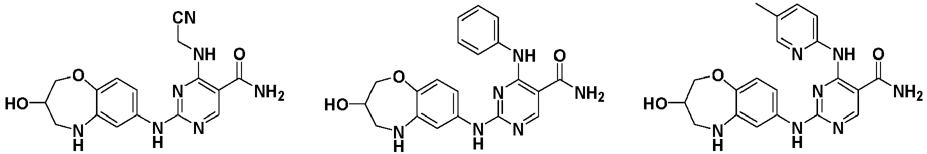

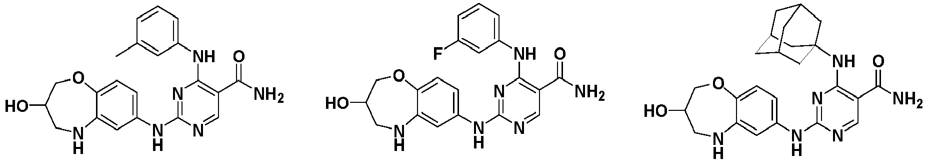

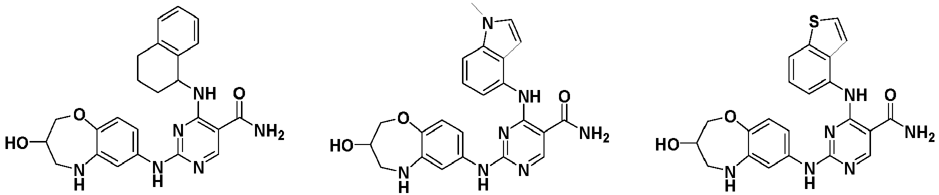

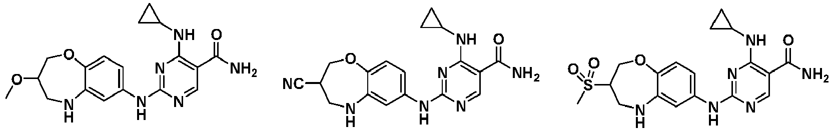

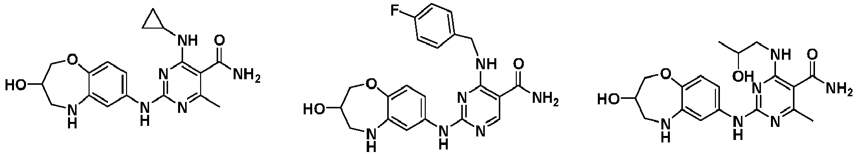

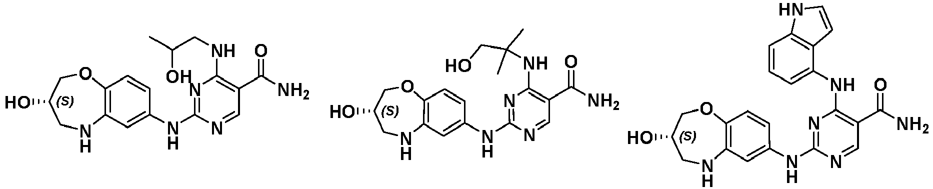

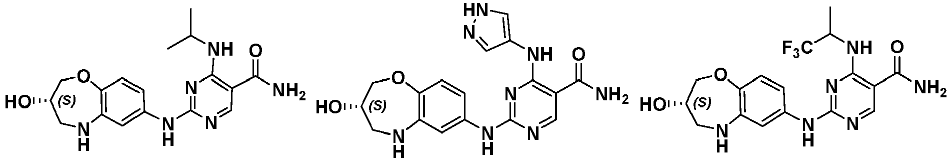

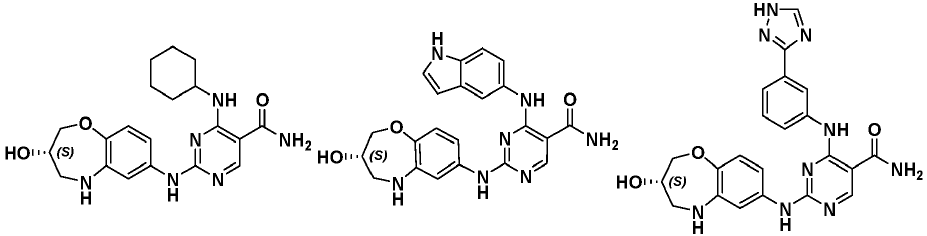

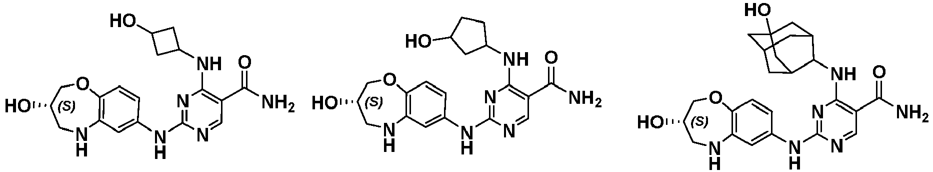

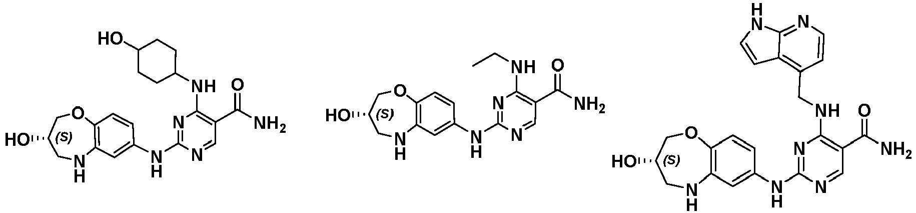

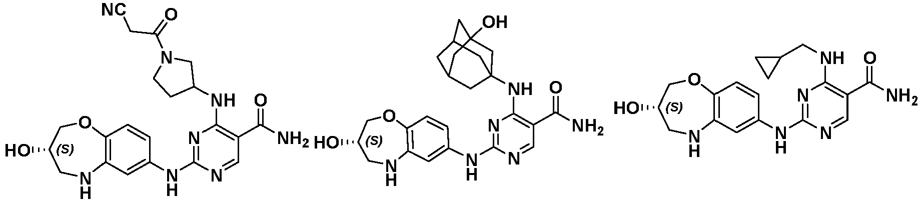

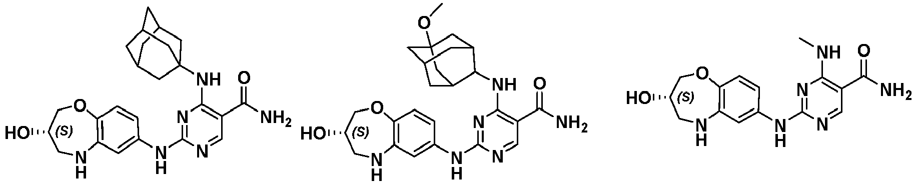

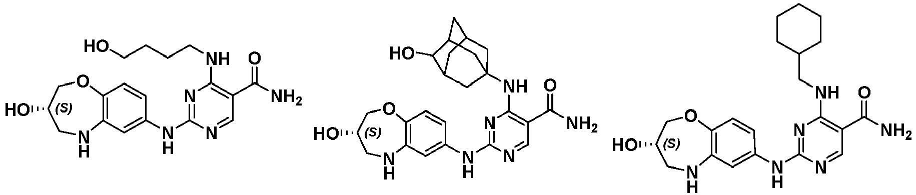

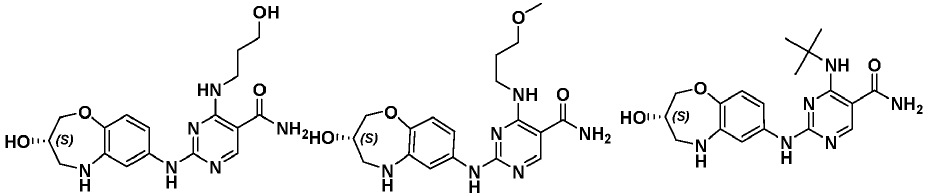

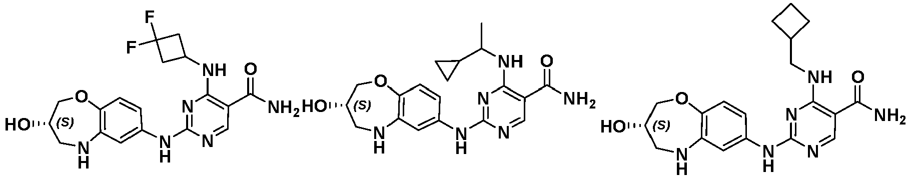

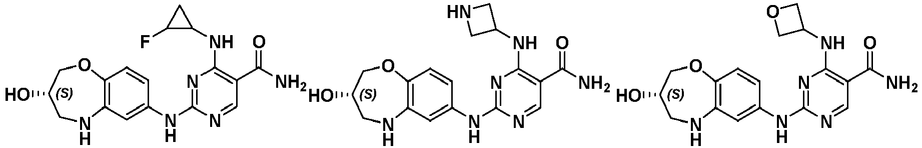

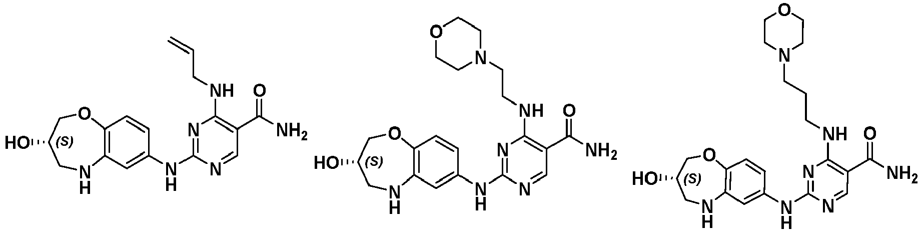

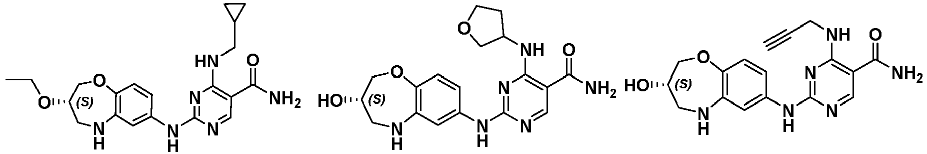

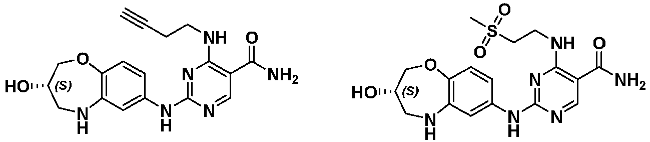

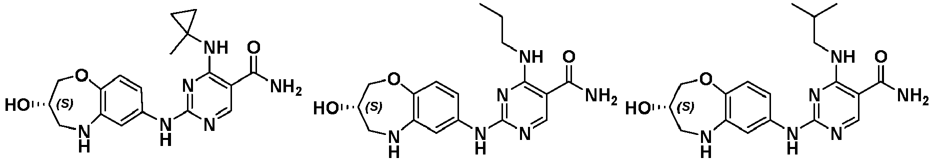

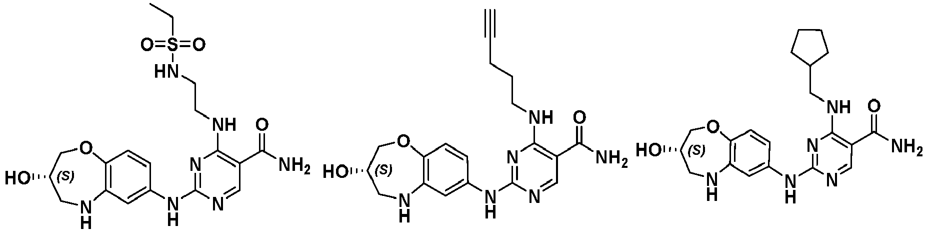

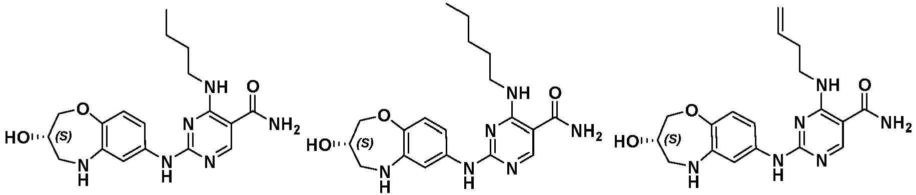

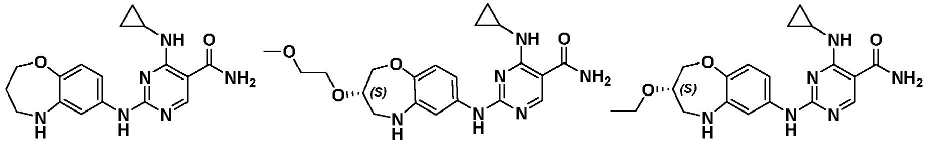

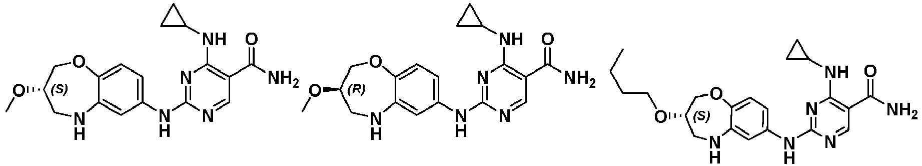

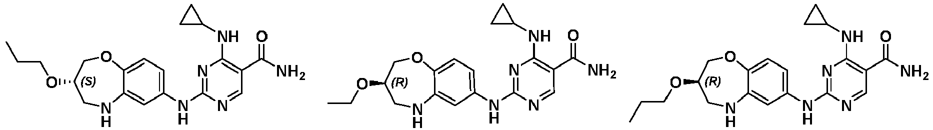

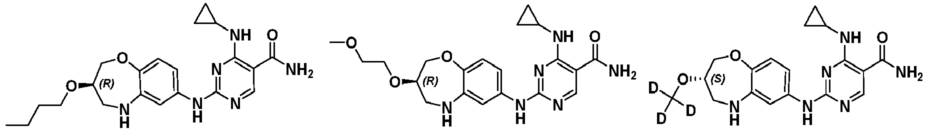

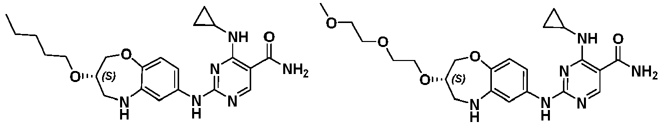

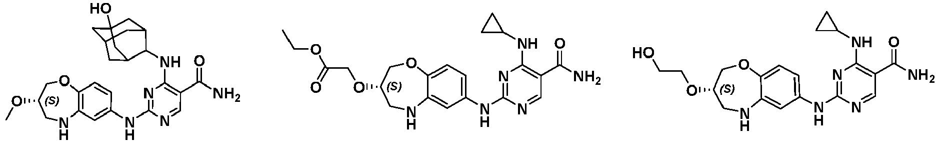

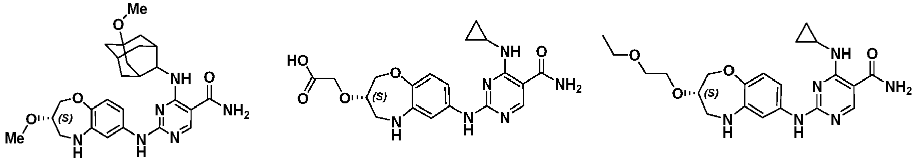

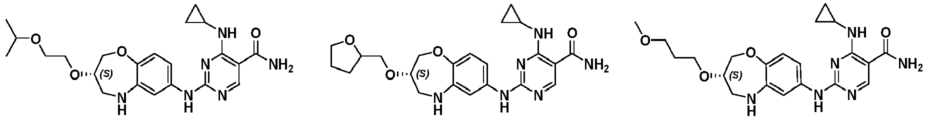

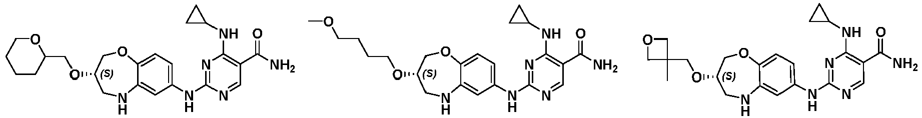

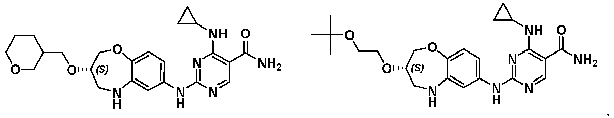

The compound in examples disclosed herein may exist in the form of an isomer, and the "compound disclosed herein" generally includes an isomer of the compound. The compound in examples disclosed herein has an isomer of S configuration or R configuration due to the presence of a chiral carbon atom. Table 1 exemplifies an isomer of S configuration or R configuration of several compounds. If a single isomer is required, it can be isolated according to a conventional method or prepared by a stereoselective synthesis.

By experiments, the inventors found that compounds with a (S) configuration have higher activity.

**Table 1**

| Structural Formula | Isomer of *S* Configuration | Isomer of *R* Configuration |
|---|---|---|
| 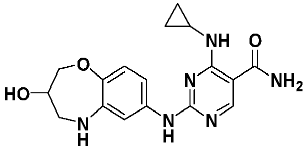 | 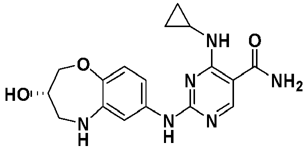 | 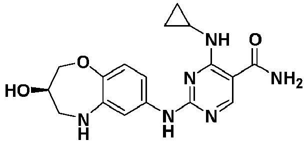 |
| 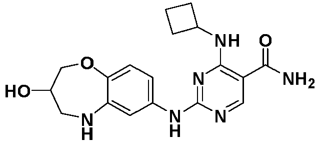 | 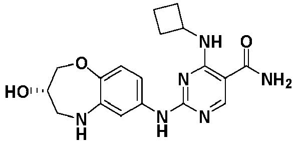 | 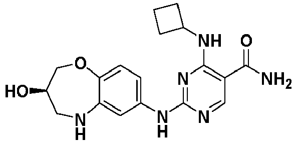 |
| 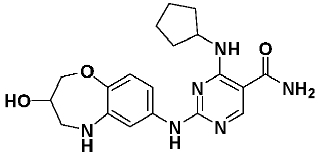 | 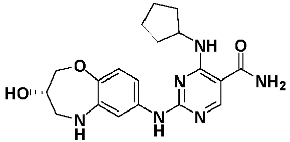 | 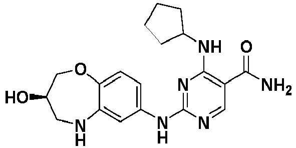 |
| 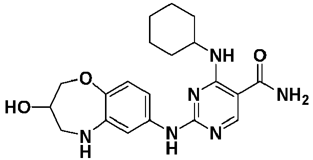 | 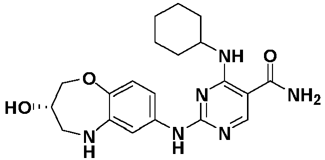 | 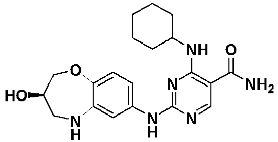 |
| 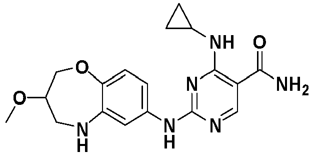 | 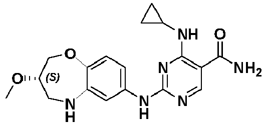 | 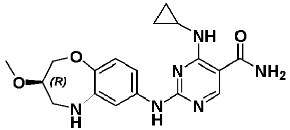 |
| 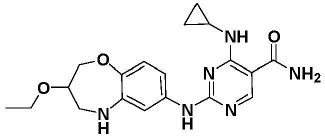 | 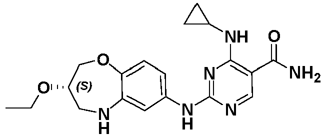 | 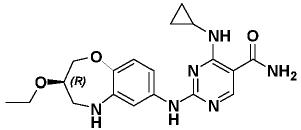 |
| 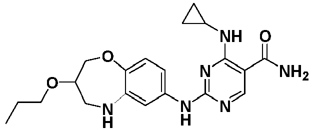 | 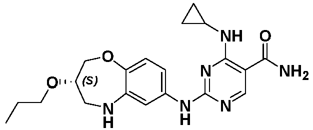 | 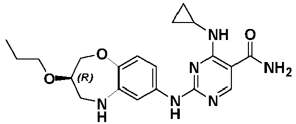 |
| 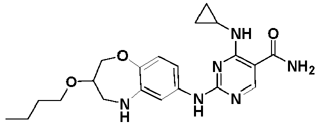 | 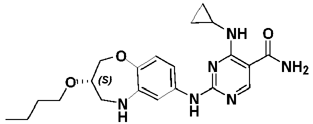 | 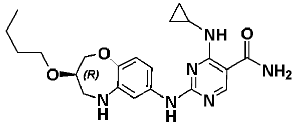 |
| 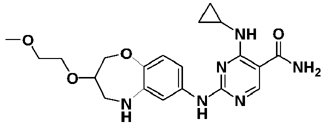 | 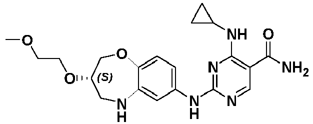 | 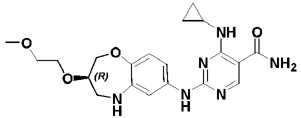 |

The following specifically describes the pharmaceutical composition of the second aspect in embodiments disclosed herein.

The pharmaceutical composition in embodiments disclosed herein comprises the compound of the first aspect in examples disclosed herein and pharmaceutically acceptable excipient(s).

Specifically, the excipient may be selected from auxiliary, adjuvant or pharmaceutical carrier.

The pharmaceutical composition of the example disclosed herein may further comprise at least one of anti-inflammatory drugs selected from non-steroidal anti-inflammatory drugs, non-selective or selective cyclooxygenase-2 inhibitors, corticosteroids, tumor necrosis factor receptor antagonists, salicylic esters or salts, immunosuppressants or methotrexate.

The pharmaceutical composition in embodiments disclosed herein can be formulated as a solid, semi-solid, liquid or gaseous preparation. A liquid dosage form can be a solution (including a true solution and a colloidal solution), an emulsion (including o/w type, w/o type and multiple emulsion), a suspension, an injection (including aqueous injection, powder injection and infusion), an eye drop, a nasal drop, a lotion and an elixir, etc.; a solid dosage form can be a tablet (including an plain tablet, an enteric tablet, a buccal tablet, a dispersible tablet, a chewable tablet, an effervescent tablet, an orally disintegrating tablet), a capsule (including a hard capsule, a soft capsule, an enteric capsule), a granule, a powder, a pellet, a dripping pill, a suppository, a film, a patch, an aerosol (a powder aerosol), a spray, etc.; and a semi-solid dosage form can be an ointment, a gel, a paste, etc. The compound disclosed herein can also be made into a sustained-release preparation, a controlled-release preparation, a targeted preparation and various microparticle delivery systems.

In order to make the compound in examples disclosed herein into a tablet, various excipients known in the art can be widely used, including a diluent, a binder, a wetting agent, a disintegrant, a lubricant, and a glidant. A diluent can be starch, dextrin, sucrose, glucose, lactose, mannitol, sorbitol, xylitol, microcrystalline cellulose, calcium sulfate, calcium hydrogen phosphate, calcium carbonate, etc.; a wetting agent can be water, ethanol, isopropanol, etc.; a binder can be starch slurry, dextrin, syrup, honey, glucose solution, microcrystalline cellulose, gum arabic slurry, gelatin slurry, sodium carboxylmethyl cellulose, methyl cellulose, hydroxypropyl methylcellulose, ethyl cellulose, acrylic resin, carbomer, polyvinylpyrrolidone, polyethylene glycol, etc.; a disintegrant can be dry starch, microcrystalline cellulose, low-substituted hydroxypropyl cellulose, cross-linked polyvinylpyrrolidone, croscarmellose sodium, carboxylmethyl starch sodium, sodium bicarbonate and citric acid, polyoxyethylene sorbitol fatty acid ester, sodium dodecyl sulfonate, etc.; a lubricant and a glidant can be talc powder, silica, stearate, tartaric acid, liquid paraffin, polyethylene glycol, etc. A tablet can also be further made into a coated tablet, such as a sugar-coated tablet, a film-coated tablet, a enteric-coated tablet, or a double-layered and multi-layered tablet. In order to make an administration unit into a capsule, an active ingredient (i.e., a compound disclosed herein) can be mixed with a diluent and a glidant, and the mixture is directly placed in a hard capsule or a soft capsule. An active ingredient (i.e., a compound disclosed herein) can also be made into a granule or a pellet with a diluent, a binder, and a disintegrant, and then placed in hard or soft capsules. Various kinds of diluents, binders, wetting agents, disintegrants, and glidants used for preparing the tablet of the compound disclosed herein can also be used to prepare a capsule of the compound disclosed herein. In order to make the compound disclosed herein into an injection, water, ethanol, isopropanol, propylene glycol, or a mixture thereof can be used as a solvent and an appropriate amount of a solubilizer, an auxiliary solvent, a pH adjuster, and an osmotic pressure adjuster commonly used in the art can be added. A solubilizer or an auxiliary solvent can be poloxamer, lecithin, hydroxypropyl-β-cyclodextrin, etc.; a pH adjuster can be phosphate, acetate, hydrochloric acid, sodium hydroxide, etc.; an osmotic pressure adjuster can be sodium chloride, mannitol, glucose, phosphate, acetate, etc. Mannitol and glucose, etc. can also be added as supplant when preparing a lyophilized powder injection. If desired, a colorant, a preservative, a flavor, a flavoring agent, or other additives can also be added to a pharmaceutical formulation.

The pharmaceutical composition in embodiments disclosed herein can be prepared by a method known in the pharmaceutical field. For example, a pharmaceutical composition intended for injection administration can be prepared by combining the compound of the first aspect in examples disclosed herein, or a pharmaceutically acceptable salt or a prodrug thereof, with sterilized distilled water to form a solution. A surfactant can be added to promote the formation of a homogeneous solution or suspension. The content of the compound in examples disclosed herein in its pharmaceutical composition is typically 0.1 to 95 weight %.

The compound in examples disclosed herein or the pharmaceutical composition containing the same can be administered in unit dosage form. The administration route can be intestinal or parenteral, such as oral, intravenous injection, intramuscular injection, subcutaneous injection, nasal cavity, oral mucosa, eye, lung and respiratory tract, skin, vagina, rectum, etc. In order to achieve the purpose of medication and enhance the therapeutic effect, the drug or pharmaceutical composition of the example disclosed herein can be administered by any known method of administration. The specific administration method and dosage form depend on the physical and chemical properties of the compound itself and the severity of the disease to be treated, etc. Those skilled in the art can determine the specific route of administration based on the above factors and combined with their own knowledge.

The following specifically describes the use and application of the third aspect and the fourth aspect in embodiments disclosed herein.

The embodiment disclosed herein relates to the use of the above compound of the first aspect of the example disclosed herein as an inhibitor of TYK2, JAK1, JAK2, JAK3, or SYK kinase.

The embodiment disclosed herein also relates to the use of the above compound of the first aspect in examples disclosed herein in the preparation of a medicament for preventing or treating a disease associated with TYK2, JAK1, JAK2, JAK3, or SYK kinase.

Among them, the disease associated with TYK2, JAK1, JAK2, JAK3, or SYK kinase is a disease caused by abnormal activation of TYK2, JAK1, JAK2, JAK3, or SYK kinase, that is, the TYK2, JAK1, JAK2, JAK3, or SYK kinase inhibitory activity possessed by the compound disclosed herein inhibits the abnormal activation of TYK2, JAK1, JAK2, JAK3, or SYK kinase.

Specifically, the diseases include autoimmune diseases, metabolic diseases, inflammatory diseases and cancers;
autoimmune diseases include, but are not limited to, asthma, psoriasis, lupus, multiple sclerosis, allergic rhinitis, atopic dermatitis, contact dermatitis, and delayed allergic reaction;
inflammatory diseases include, but are not limited to, inflammatory bowel disease and rheumatoid arthritis; wherein the inflammatory bowel disease includes Crohn's disease and ulcerative colitis;
cancers include, but are not limited to, leukemia.

The embodiment disclosed herein also relates to a method for treating a disease by administering to a patient the compound of the first aspect in embodiment disclosed herein or the pharmaceutical composition of the second aspect in embodiment disclosed herein to inhibit the activity of TYK2, JAK1, JAK2, JAK3, or SYK

### Preparation of Example Compounds Disclosed Herein

The example compounds in the present disclosure can be prepared according to the following schemes. However, the following reaction schemes only illustrate the preparation methods of compounds disclosed herein by way of example.

Those skilled in the art will understand that in the following description, the combination of substituents is allowed only when the combination of such substituents can obtain a stable compound.

Those skilled in the art will also understand that in the methods described below, functional groups of intermediate compounds may need to be protected by appropriate protecting groups. Such functional groups include hydroxyl, amino, mercapto, and carboxylic acids. Suitable protecting groups for hydroxy include trialkylsilyl or diarylalkylsilyl (e.g., tert-butyldimethylsilyl, tert-butyldiphenylsilyl or trimethylsilyl), tetrahydropyranyl, benzyl, etc. Suitable protecting groups for amino, amidino and guanidino include tert-butoxycarbonyl, benzyloxycarbonyl, etc. Suitable protecting groups for mercapto include - C(O)-R" (wherein R" is alkyl, aryl or aralkyl), p-methoxybenzyl, trityl, etc. Suitable protecting groups for carboxyl include alkyl, aryl, or aralkyl esters. The protecting groups can be introduced and removed according to standard techniques known to those skilled in the art and as described herein. The protecting groups may also be a polymer resin.

The example compounds disclosed herein can be synthesized by the following Route A, which is described using the synthesis of an intermediate in which X is O and Y is NRₐ as an example. 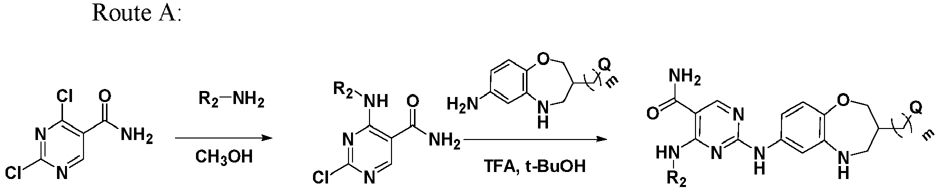 wherein, R₂ and Q have the same definitions as those in the compounds of the first aspect in examples disclosed herein. 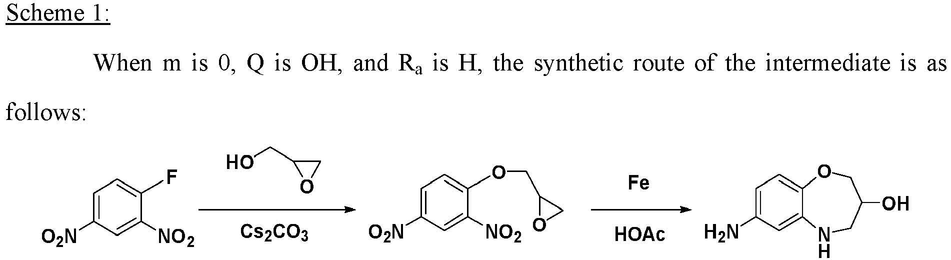 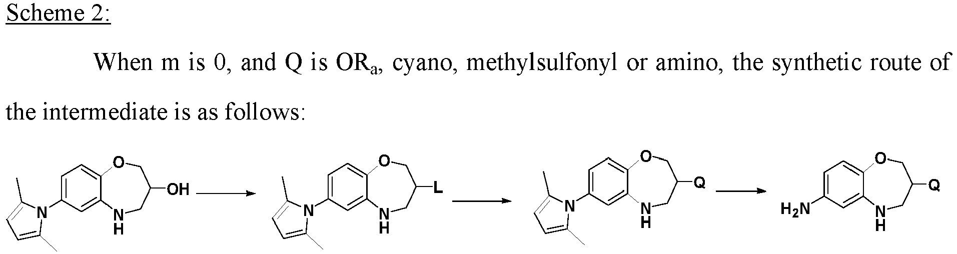 wherein, Rₐ is linear or branched C₁-C₄ alkyl, or C₃-C₈ cycloalkyl, and L is a leaving group such as Br, Cl, OTf, or OMs.

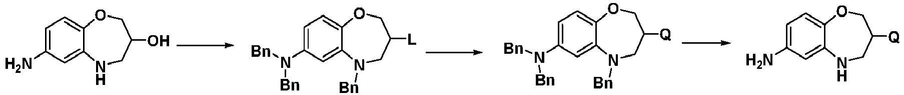

The present disclosure is further described below with reference to examples and experimental examples, but these examples and experimental examples do not limit the scope of the present disclosure.

### Example 1: 4-cyclobutylamino-2-(3-hydroxy-2,3,4,5-tetrahydro-benzo[1,4]oxazin-7-amino)pyrimidin-5-carboxamide (1)

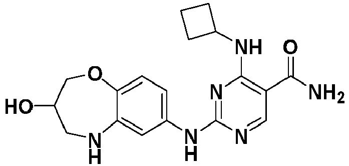

The compound of Example 1 was prepared according to Route A. The specific steps are as follows:

### (1) Preparation of 2-((2,4-dinitrophenoxy)methyl)oxirane (1-1):

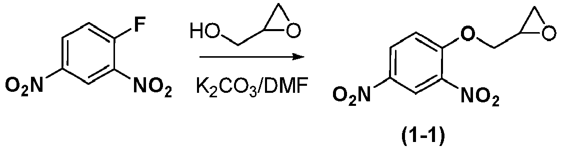

Hydroxymethyloxirane (163 mg, 2.2 mmol) was dissolved in dimethylformamide (DMF) (2 mL), and potassium carbonate was added. At room temperature, to the mixture was added slowly a solution of 1-fluoro-2,4-dinitrobenzene (372 mg, 2 mmol) in DMF (3 mL) dropwise, stirred for 5 hours, quenched with water (10 mL), and extracted with ethyl acetate (50 mL). The organic phase was washed with saturated ammonium chloride solution and then water, dried over anhydrous sodium sulfate, and concentrated under reduced pressure. The concentrate was purified by silica gel column chromatography (eluent: n-hexane:ethyl acetate = 1:1) to give 2-((2,4-dinitrophenoxy)methyl)oxirane **(1-1)** (384 mg) as a pale yellow solid with a yield of 80%.
ESI-MS: [M+H]^{+ =} 241.

### (2) Preparation of 7-amino-2,3,4,5-tetrahydro-benzo[1,4]oxazin-3-ol (1-2):

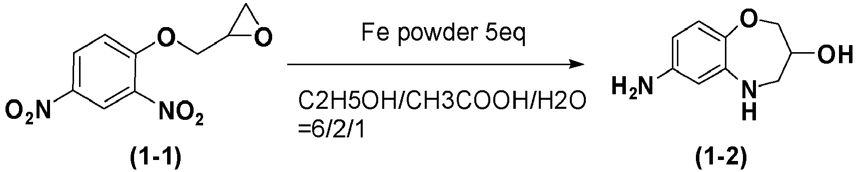

**1-1** (384 mg, 1.6 mmol) was dissolved in ethanol/acetic acid/water (6:2:1) (5 mL). At room temperature, to the mixture was added slowly iron powder (448 mg, 8 mmol), refluxed for 2 hours, cooled, and filtered. The filtrate was concentrated under reduced pressure. The concentrate was purified by silica gel column chromatography (eluent: dichloromethane:methanol:aqueous ammonia = 100:5:1) to give 7-amino-2,3,4,5-tetrahydro-benzo[1,4]oxazin-3-ol **(1-2)** (90 mg) as an off-white solid with a yield of 31%. ESI-MS: [M + H]⁺ = 181.

### (3) Preparation of 2-chloro-4-(cyclobutylamine)pyrimidin-5-carboxamide (1-3):

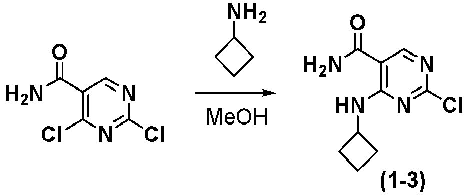

2,4-Dichloropyrimidin-5-carboxamide (384 mg, 2 mmol) was dissolved in methanol (5 mL). At room temperature, to the mixture was added slowly cyclobutylamine (284 mg, 4 mmol), stirred for 1 hour, filtered, washed with water (2 mL), and dried to give 2-chloro-4-(cyclobutylamine)pyrimidin-5-carboxamide **(1-3)** (366 mg) as a white solid with a yield of 81%. ESI-MS: [M + H]⁺ = 227.

### (4) Preparation of 4-cyclobutylamino-2-(3-hydroxy-2,3,4,5-tetrahydro-benzo[1,4]oxazin-7-amino)pyrimidin-5-carboxamide (1):

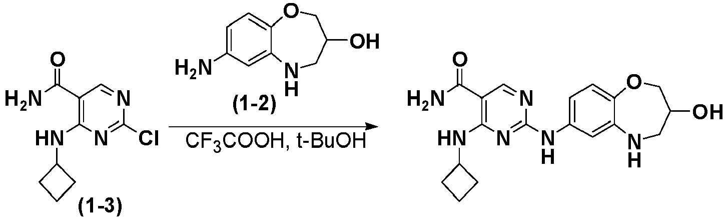

**(1-2)** (36 mg, 0.2 mmol), and **(1-3)** (45.3 mg, 0.2 mmol) were dissolved in tert-butanol (3 mL), and trifluoroacetic acid (0.1 mL) was added. The mixture was refluxed for 5 hours, cooled to room temperature, concentrated under reduced pressure, and purified by pre-HPLC (eluent: acetonitrile:water = 15:1-40:1) to give 4-cyclobutyl-2-(3-hydroxy-2,3,4,5-tetrahydro-benzo[1,4]oxazin-7-amino)pyrimidin-5-carboxamide **(1)** (20 mg) as a white solid with a yield of 27%. The relevant characterization data was as follows:
ESI-MS: [M+H]⁺ = 371.1.
¹H-NMR (300 MHz, MeOD-*d*₄) δ: ppm 8.24 (s, 1H), 6.90 (d, 1H, J = 8.4 Hz) 6.81-6.77 (m, 2H), 4.62-4.35 (m, 1H), 4.29-4.25 (m, 1H), 4.05-3.99 (m, 2H), 3.47-3.41 (m, 1H), 3.26-3.22 (m, 1H), 2.43-2.38 (m, 2H), 2.10-2.03 (m, 2H), 1.88-1.79 (m, 2H).

### Example 2: 4-cyclopropylamino-2-(3-hydroxy-2,3,4,5-tetrahydro-benzo[1,4]oxazin-7-amino)pyrimidin-5-carboxamide (2)

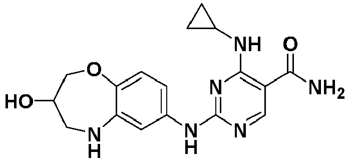

The compound of this example can be prepared according to the similar steps of the aforementioned method for preparing example 1, except that in the reaction of step 3, cyclopropylamine was used as a raw material in place of cyclobutylamine. The relevant characterization data was as follows:
ESI-MS: [M+H]^{+ =} 357.1.
¹H-NMR (300 MHz, DMSO-*d*₆) δ: ppm 9.27 (s, 1H), 9.09 (d, J = 3.3 Hz, 1H), 8.47 (s, 1H), 7.76 (s, 1H), 7.23 (d, J = 2.1 Hz, 1H), 7.11 (d, J = 7.8 Hz, 2H), 6.65 (d, J = 8.4 Hz, 1H), 5.15 (s, 1H), 4.96 (d, J = 5.4 Hz, 1H), 4.11-4.17 (m, 1H), 3.72-3.88 (m, 2H), 3.17 (d, J=4.8 Hz, 1H), 2.85-3.03 (m, 2H), 0.76-0.83 (m, 2H), 0.46-0.51 (m, 2H).

### Example 3: 4-cyclopentylamino-2-(3-hydroxy-2,3,4,5-tetrahydro-benzo[1,4]oxazin-7-amino)pyrimidin-5-carboxamide (3)

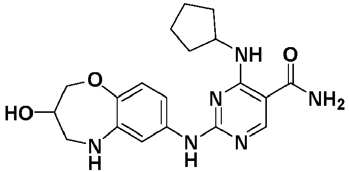

The compound of this example can be prepared according to the similar steps of the aforementioned method for preparing example 1, except that in the reaction of step 3, cyclopentylamine was used as a raw material in place of cyclobutylamine. The relevant characterization data was as follows:
ESI-MS: [M+H]⁺ = 385.2.
¹H-NMR (300 MHz, DMSO-*d*₆) δ: ppm 10.25 (s, 1H), 9.90 (s, 1H), 8.41 (s, 1H), 8.12 (s, 1H), 7.61 (s, 1H), 6.87 (s, 2H), 6.77 (d, 1H, J = 9 Hz), 4.32 (m, 1H), 4.18 (dd, 1H, J₁= 12 Hz, J₂= 3.9 Hz), 3.92-3.79 (m, 2H), 3.32 (dd, 1H, J₁= 12.9 Hz, J₂= 4.8 Hz), 3.08-3.01 (m, 1H), 1.99-1.96 (m, 2H), 1.68-1.48 (m, 6H).

### Example 4: 4-cyclohexylamino-2-(3-hydroxy-2,3,4,5-tetrahydro-benzo[1,4]oxazin-7-amino)pyrimidin-5-carboxamide (4)

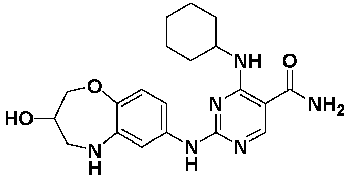

The compound of this example can be prepared according to the similar steps of the aforementioned method for preparing example 1, except that in the reaction of step 3, cyclohexylamine was used as a raw material in place of cyclobutylamine. The relevant characterization data was as follows:
ESI-MS: [M+H]⁺ = 399.2.
¹H-NMR (300 MHz, DMSO-*d*₆) δ: ppm 10.59 (s, 1H), 10.00 (d, J = 7.3 Hz, 1H), 8.48 (s, 1H), 8.18 (s, 1H), 7.71 (s, 1H), 6.83 (t, J = 22.7 Hz, 3H), 4.19 (dd, J = 11.8, 3.2 Hz, 1H), 4.06 - 3.75 (m, 3H), 3.33 (dd, J = 12.9, 4.7 Hz, 1H), 3.07 (dd, J = 12.9, 7.1 Hz, 1H), 1.91 (s, 2H), 1.80 - 1.47 (m, 3H), 1.46 - 1.10 (m, 5H).

### Example 5: 4-(1-adamantanyl-amino)-2-(3-hydroxy-2,3,4,5-tetrahydro-benzo[1,4]oxazin-7-amino)pyrimidin-5-carboxamide (5)

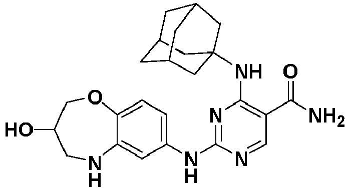

The compound of this example can be prepared according to the similar steps of the aforementioned method for preparing example 1, except that in the reaction of step 3, 1-adamantanamine was used as a raw material in place of cyclobutylamine. The relevant characterization data was as follows:
ESI-MS: [M+H]⁺ = 451.3.
¹H-NMR (300 MHz, DMSO-*d*₆) δ: ppm 11.77 (s, 1H), 10.26 (s, 1H), 8.72 (d, J = 10.26Hz, 1H), 8.42 (d, J = 8.66Hz, 1H), 7.23 (s, 1H), 6.85(m, 1H), 4.17 (d, J = 11.5 Hz, 2H), 3.60 (s, 1H), 3,56 (m, 3H), 3.34 (d, J = 4.2 Hz, 2H), 3.08 (m,2H), 2.22(m, 10H), 1.95 (m, 5H).

### Example 6: 4-(tetrahydropyran-4-yl-amino)-2-(3-hydroxy-2,3,4,5-tetrahydrobenzo[1,4]oxazin-7-amino)pyrimidin-5-carboxamide (6)

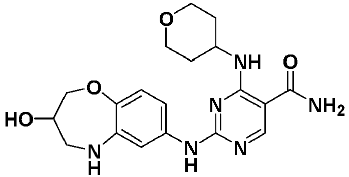

The compound of this example can be prepared according to the similar steps of the aforementioned method for preparing example 1, except that in the reaction of step 3, 4-aminotetrahydropyran was used as a raw material in place of cyclobutylamine. The relevant characterization data was as follows :
ESI-MS: [M+H]⁺ = 401.2.
¹H-NMR (300 MHz, DMSO-*d*₆) δ: ppm 10.59 (s, 1H), 10.00 (d, J = 7.3 Hz, 1H), 8.48 (s, 1H), 8.28 (s, 1H), 7.71 (s, 1H), 6.83 (t, J = 22.7 Hz, 3H), 4.19 (dd, J = 11.8, 3.2 Hz, 1H), 4.06 - 3.75 (m, 3H), 3.80 - 3.47 (m, 4H), 3.33 (dd, J = 12.9, 4.7 Hz, 1H), 3.07 (dd, J = 12.9, 7.1 Hz, 1H), 1.91 (s, 2H), 2.46 - 2.10 (m, 5H).

### Example 7: 4-trifluoroethylamino-2-(3-hydroxy-2,3,4,5-tetrahydro-benzo[1,4]oxazin-7-amino)pyrimidin-5-carboxamide (7)

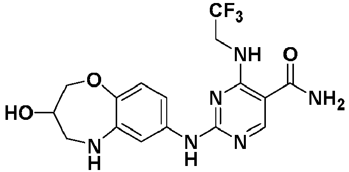

The compound of this example can be prepared according to the similar steps of the aforementioned method for preparing example 1, except that in the reaction of step 3, trifluoroethylamine was used as a raw material in place of cyclobutylamine. The relevant characterization data was as follows:
ESI-MS: [M+H]⁺ = 430.3.
¹H-NMR (300 MHz, DMSO-*d*₆) δ: ppm 8.03 (d, 2H, J = 8.7 Hz), 7.84 (s, 1H), 7.50 (d, 2H, J = 8.7 Hz), 7.22 (m, 3H), 6.96 (q, 2H, J = 7.8 Hz), 6.73 (d, 1H, J = 7.8 Hz), 2.60 (s, 3H), 1.87 (m, 1H), 0.81 (m, 2H), 0.62 (m, 2H).

### Example 8: 4-(2-cyanoethylamino)-2-(3-hydroxy-2,3,4,5-tetrahydro-benzo[1,4]oxazin-7-amino)pyrimidin-5-carboxamide (8)

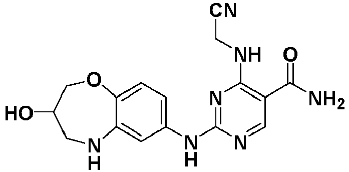

The compound of this example can be prepared according to the similar steps of the aforementioned method for preparing example 1, except that in the reaction of step 3, 2-cyanoethylamine was used as a raw material in place of cyclobutylamine. The relevant characterization data was as follows:
ESI-MS: [M+H]⁺ = 356.1.
¹H-NMR (300 MHz, DMSO-*d*₆) δ: ppm 9.39 (t, J = 5.8 Hz, 1H), 8.67 (s, 1H), 8.26 (s, 1H), 7.79 (s, 1H), 6.72(dd, J=11.8Hz, 8.5Hz), 6.58 (d, J=8.5Hz, 2.8Hz), 5.63 (d, J=11.8Hz, 2.8Hz), 4.63 - 4.26 (m, 2H), 4.25(m, 1H), 4.03(m, 3H), 3.78 (m, 1H), 3.46(m, 2H), 3.21(m, 1H).

### Example 9: 4-benzylamino-2-(3-hydroxy-2,3,4,5-tetrahydro-benzo[1,4]oxazin-7-amino)pyrimidin-5-carboxamide (9)

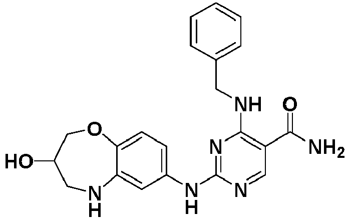

The compound of this example can be prepared according to the similar steps of the aforementioned method for preparing example 1, except that in the reaction of step 3, benzylamine was used as a raw material in place of cyclobutylamine. The relevant characterization data was as follows:
ESI-MS: [M+H]⁺ = 407.1.
¹H-NMR (300 MHz, DMSO-*d*₆) δ: ppm 9.52 (s, 1H), 8.58 (s, 2H), 7.99 (s, 1H), 7.51 - 7.11 (m, 8H), 4.64 (d, J = 5.9 Hz, 2H), 4.03 (s, 1H), 3.81 (s, 3H), 3.04 (q, J = 7.2 Hz, 2H), 1.24 - 1.01 (m, 2H).

### Example 10: 4-(4-fluorobenzylamino)-2-(3-hydroxy-2,3,4,5-tetrahydro-benzo[1,4]oxazin-7-amino)pyrimidin-5-carboxamide (10)

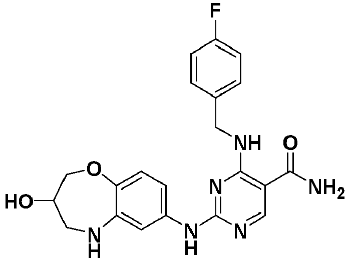

The compound of this example can be prepared according to the similar steps of the aforementioned method for preparing example 1, except that in the reaction of step 3, 4-fluorobenzylamine was used as a raw material in place of cyclobutylamine. The relevant characterization data was as follows:
ESI-MS: [M+H]⁺ = 425.2.
¹H-NMR (300 MHz, DMSO-*d*₆) δ: ppm 10.24 (s, 2H), 8.79 (d, J = 6.5 Hz, 2H), 8.57 (s, 1H), 8.10 (s, 1H), 7.83 (d, J = 6.1 Hz, 2H), 7.63 (s, 1H), 6.77 (s, 1H), 6.67 - 6.49 (m, 3H), 4.14 (dd, J = 12.0, 3.9 Hz, 1H), 3.91 (s, 1H), 3.81 (dd, J = 12.2, 4.6 Hz, 1H), 3.30 (dd, J = 12.9, 4.6 Hz, 1H), 3.04 (dd, J = 13.1, 7.2 Hz, 1H).

### Example 11: 4-(4-methoxybenzylamino)-2-(3-hydroxy-2,3,4,5-tetrahydro-benzo[1,4]oxazin-7-amino)pyrimidin-5-carboxamide (11)

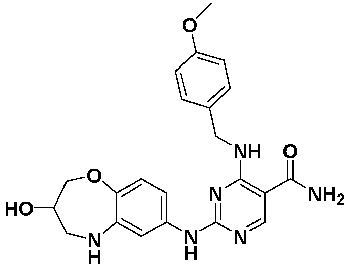

The compound of this example can be prepared according to the similar steps of the aforementioned method for preparing example 1, except that in the reaction of step 3, 4-methoxybenzylamine was used as a raw material in place of cyclobutylamine. The relevant characterization data was as follows:
ESI-MS: [M+H]⁺ = 437.2.
¹H-NMR (300 MHz, DMSO-*d*₆) δ: ppm 10.03 (br, 2H), 8.41 (s, 1H), 8.08 (br, 1H), 7.55 (br, 1H), 7.22 (d, 2H, J = 8.4 Hz), 6.88 (m, 3H), 6.75 (m, 2H), 4.58(d, 2H, J = 5.4 Hz), 4.18 (dd, 1H, J₁= 12 Hz, J₂= 3.6 Hz), 3.91-3.72 (m, 2H), 3.72 (s, 3H), 3.04 (dd, 1H, J₁= 12.9 Hz, J₂= 6.9 Hz), 3.07-3.00 (m, 1H).

### Example 12: 4-(1-phenylethylamino)-2-(3-hydroxy-2,3,4,5-tetrahydro-benzo[1,4] oxazin-7-amino)pyrimidin-5-carboxamide (12)

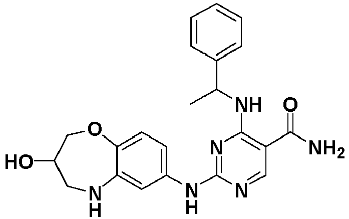

The compound of this example can be prepared according to the similar steps of the aforementioned method for preparing example 1, except that in the reaction of step 3, 1-phenylethylamine was used as a raw material in place of cyclobutylamine. The relevant characterization data was as follows:
ESI-MS: [M+H]⁺=421.2.
¹H-NMR (300 MHz, DMSO-*d*₆) δ: ppm 9.54 (s, 1H), 8.57 (s, 2H), 8.00 (s, 1H), 7.52 - 7.13 (m, 8H), 4.65 (d, J = 5.9 Hz, 2H), 4.03 (s, 1H), 3.81 (s, 3H), 3.03 (q, J = 7.2 Hz, 1H), 1.25 (d, J = 6.8 Hz, 3H).

### Example 13: 4-(pyridin-2-methylamino)-2-(3-hydroxy-2,3,4,5-tetrahydro-benzo[1,4]oxazin-7-amino)pyrimidin-5-carboxamide (13)

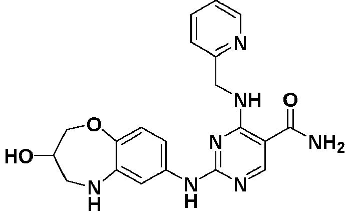

The compound of this example can be prepared according to the similar steps of the aforementioned method for preparing example 1, except that in the reaction of step 3, pyridin-2-ylmethanamine was used as a raw material in place of cyclobutylamine. The relevant characterization data was as follows:
ESI-MS: [M+H]⁺ = 408.2.
¹H-NMR (300 MHz, DMSO-*d*₆) δ: ppm 10.36 (br, 1H), 10.20 (br, 1H), 8.62 (d, 1H, J = 3.6 Hz), 8.48 (s, 1H), 8.11 (br, 1H), 8.00-7.93 (m, 1H), 7.59 (br, 1H), 7.48-7.43 (m, 2H), 6.91 (s, 1H), 6.66 (m, 2H), 4.85 (d, 2H, J = 5.4 Hz), 4.18 (dd, 1H, J₁= 12.3 Hz, J₂= 3.9 Hz), 3.90-3.78 (m, 2H), 3.33 (dd, 1H, J₁= 13.6 Hz, J₂= 4.8 Hz), 3.09-3.02 (m, 1H).

### Example 14: 4-(pyridin-3-methylamino)-2-(3-hydroxy-2,3,4,5-tetrahydro-benzo[1,4]oxazin-7-amino)pyrimidin-5-carboxamide (14)

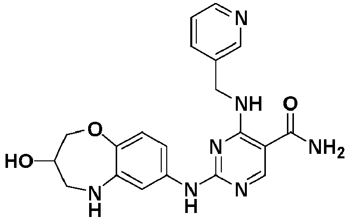

The compound of this example can be prepared according to the similar steps of the aforementioned method for preparing example 1, except that in the reaction of step 3, pyridin-3-ylmethanamine was used as a raw material in place of cyclobutylamine. The relevant characterization data was as follows:
ESI-MS: [M+H]⁺ = 408.2.
¹H-NMR (300 MHz, MeOD-*d*₄) δ: ppm 8.70 (s, 1H), 8.68 (s, 1H), 8.38 (br, 2H), 7.90 (m, 1H), 6.87 (d, 1H, J = 8.4 Hz), 6.81 (d, 1H, J = 2.1 Hz), 6.68 (dd, 1H, J₁= 8.7 Hz, J₂= 2.4 Hz), 4.88 (s, 1H), 4.25 (m, 1H), 4.07-4.02 (m, 2H), 3.46-3.40 (m, 1H), 3.29-3.23 (m, 1H).

### Example 15: 4-(pyridin-4-methylamino)-2-(3-hydroxy-2,3,4,5-tetrahydro-benzo[1,4]oxazin-7-amino)pyrimidin-5-carboxamide (15)

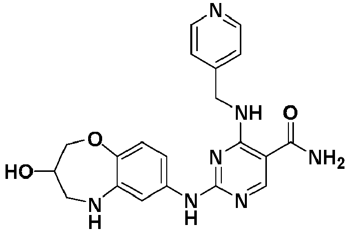

The compound of this example can be prepared according to the similar steps of the aforementioned method for preparing example 1, except that in the reaction of step 3, pyridin-4-ylmethanamine was used as a raw material in place of cyclobutylamine. The relevant characterization data was as follows:
ESI-MS: [M+H]⁺ = 408.2.
¹H-NMR (300 MHz, MeOD-*d*₄) δ: ppm 10.31 (s, 1H), 10.19 (s, 1H), 8.55 - 8.32 (m, 1H), 8.12 (s, 1H), 7.63 (s, 1H), 7.43 - 7.27 (m, 2H), 7.14 (t, J = 8.8 Hz, 2H), 6.86 (s, 1H), 6.75 (s, 2H), 4.65 (d, J = 5.7 Hz, 2H), 4.18 (dd, J = 11.8, 3.5 Hz, 1H), 4.00 - 3.73 (m, 2H), 3.32 (dd, J = 13.0, 4.8 Hz, 1H), 3.06 (dd, J = 12.9, 7.0 Hz, 1H).

### Example 16: 4-phenylamino-2-(3-hydroxy-2,3,4,5-tetrahydro-benzo[1,4]oxazin-7-amino)pyrimidin-5-carboxamide (16)

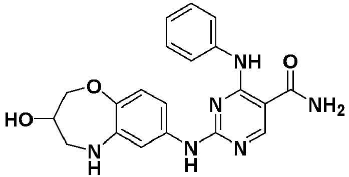

The compound of this example can be prepared according to the similar steps of the aforementioned method for preparing example 1, except that in the reaction of step 3, aniline was used as a raw material in place of cyclobutylamine. The relevant characterization data was as follows:
ESI-MS: [M+H]⁺=393.1.
¹H-NMR (300 MHz, MeOD-*d*₄) δ: ppm 12.10 (s, 1H), 10.55 (s, 1H), 8.73 (s, 1H), 8.46 (s, 1H), 7.88 (s, 1H), 7.43 (dd, J = 17.8, 10.7 Hz, 2H), 7.14- 6.82 (m, 6H), 4.17 (dd, J = 11.8, 3.0 Hz, 1H), 3.97 (ddd, J = 16.6, 13.1, 5.8 Hz, 2H), 3.35 (dd, J = 13.0, 4.2 Hz, 1H), 3.14 (dd, J = 13.0, 6.7 Hz, 1H), 2.28 (s, 3H).

### Example 17: 4-(3-methylphenylamino)-2-(3-hydroxy-2,3,4,5-tetrahydro-benzo[1,4]oxazin-7-amino)pyrimidin-5-carboxamide (17)

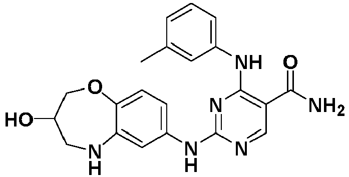

The compound of this example can be prepared according to the similar steps of the aforementioned method for preparing example 1, except that in the reaction of step 3, 3-methylaniline was used as a raw material in place of cyclobutylamine. The relevant characterization data was as follows:
ESI-MS: [M+H]⁺ = 407.2.
¹H-NMR (300 MHz, MeOD-*d*₄) δ: ppm 12.10 (s, 1H), 10.55 (s, 1H), 8.73 (s, 1H), 8.46 (s, 1H), 7.88 (s, 1H), 7.63 - 6.82 (m, 7H), 4.17 (dd, J = 11.8, 3.0 Hz, 1H), 3.97 (ddd, J = 16.6, 13.1, 5.8 Hz, 2H), 3.35 (dd, J = 13.0, 4.2 Hz, 1H), 3.14 (dd, J = 13.0, 6.7 Hz, 1H), 2.28 (s, 3H).

### Example 18: 4-(3-fluorophenylamino)-2-(3-hydroxy-2,3,4,5-tetrahydro-benzo[1,4]oxazin-7-amino)pyrimidin-5-carboxamide (18)

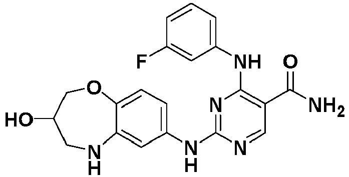

The compound of this example can be prepared according to the similar steps of the aforementioned method for preparing example 1, except that in the reaction of step 3, 3-fluoroaniline was used as a raw material in place of cyclobutylamine. The relevant characterization data was as follows:
ESI-MS: [M+H]⁺ = 411.1.
¹H-NMR (300 MHz, MeOD-*d*₄) δ: ppm 12.07 (s, 1H), 11.66 (d, J = 34.6 Hz, 1H), 10.22 (s, 1H), 8.74 (s, 1H), 8.35 (s, 1H), 8.12 (s, 1H), 7.74 (s, 2H), 7.34 (dd, J = 17.8, 10.7 Hz, 2H), 7.15 - 6.68 (m, 3H), 4.33 - 3.66 (m, 3H), 3.35 (d, J = 10.9 Hz, 1H), 3.15 (d, J = 5.9 Hz, 1H).

### Example 19: 4-(6-methylpyridin-3-amino)-2-(3-hydroxy-2,3,4,5-tetrahydrobenzo[1,4]oxazin-7-amino)pyrimidin-5-carboxamide (19)

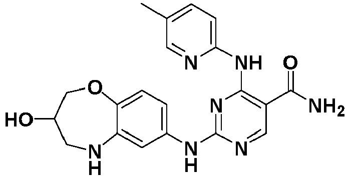

The compound of this example can be prepared according to the similar procedure of the aforementioned preparation example 1, except that in the reaction of step 3,5-methyl-2-aminopyridine was used as a raw material in place of cyclobutylamine. The relevant characterization data was as follows:
ESI-MS: [M+H]⁺ = 408.2.
¹H-NMR (300 MHz, MeOD-*d*₄) δ: ppm 9.45 (s, 2H), 7.96 (s, 1H), 8.68 (d, J = 9.7 Hz, 1H), 8.40(s, J = 9.7 Hz 1H), 7.56 (d, J = 8.67 Hz, 1H), 6.87(dd, J = 8.67Hz, J = 5.3Hz, 1H), 6.52 (s, J = 8.67 Hz, 2H), 5.55(s,2H), 4.25 (m, 2H), 4.18 (s, 2H), 3.80 (m, 1H), 3.03 (m, 2H), 2.45 (s, 3H).

### Example 20: 4-(1-methylindol-4-amino)-2-(3-hydroxy-2,3,4,5-tetrahydro-benzo[1,4]oxazin-7-amino)pyrimidin-5-carboxamide (20)

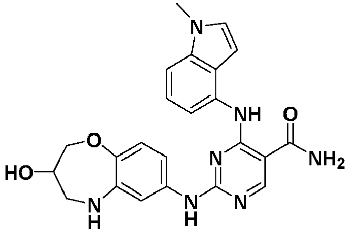

The compound of this example can be prepared according to the similar steps of the aforementioned method for preparing example 1, except that in the reaction of step 3, N-methyl-4-aminoindole was used as a raw material in place of cyclobutylamine. The characterization data was as follows:
ESI-MS: [M+H]⁺ = 446.2.
¹H-NMR (300 MHz, MeOD-*d*₄) δ: ppm 12.64 (s, 1H), 10.69 (s, 1H), 8.85 (d, J = 14.7 Hz, 1H), 8.59 (s, 1H), 7.96 (s, 2H), 7.41 (d, J = 3.0 Hz, 1H), 7.31 (d, J = 8.2 Hz, 1H), 7.23 - 7.01 (m, 3H), 6.90 (d, J = 8.5 Hz, 1H), 6.48 (d, J = 2.8 Hz, 1H), 4.19 (dd, J = 11.8, 2.7 Hz, 1H), 4.09 - 3.87 (m, 2H), 3.82 (s, 3H), 3.38 (dd, J = 13.0, 3.9 Hz, 1H), 3.16 (dd, J = 12.9, 6.8 Hz, 1H).

### Example 21: 4-(benzothiophen-4-amino)-2-(3-hydroxy-2,3,4,5-tetrahydro-benzo[1,4]oxazin-7-amino)pyrimidin-5-carboxamide (21)

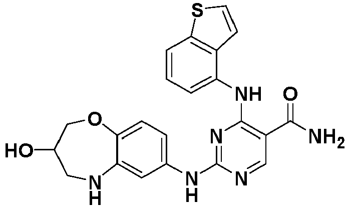

The compound of this example can be prepared according to the similar steps of the aforementioned method for preparing example 1, except that in the reaction of step 3, 4-aminobenzothiophene was used as a raw material in place of cyclobutylamine. The relevant characterization data was as follows:
ESI-MS: [M+H]⁺ = 449.2.
¹H-NMR (300 MHz, MeOD-*d*₄) δ: ppm 12.50 (s, 1H), 11.65 (d, J = 24.7 Hz, 1H), 10.08 (s, 1H), 8.73 (s, 1H), 8.24 (d, J = 73.5 Hz, 2H), 7.84 (dd, J = 16.6, 6.6 Hz, 2H), 7.63 - 7.22 (m, 2H), 6.83 (d, J = 31.7 Hz, 2H), 4.16 (d, J = 10.7 Hz, 1H), 4.08 - 3.65 (m, 2H), 3.34 (d, J = 8.9 Hz, 1H), 3.11 (s, 1H).

### Example 22: 4-(1,2,3,4-tetrahydronaphthalen-1-amino)-2-(3-hydroxy-2,3,4,5-tetrahydrobenzo[1,4]oxazin-7-amino)pyrimidin-5-carboxamide (22)

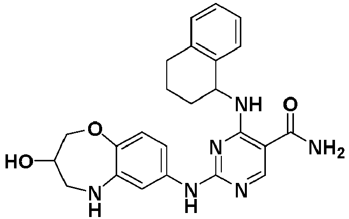

The compound of this example can be prepared according to the similar steps of the aforementioned method for preparing example 1, except that in the reaction of step 3, 1-amino-1,2,3,4-tetrahydronaphthalene was used as a raw material in place of cyclobutylamine. The relevant characterization data was as follows:
ESI-MS: [M+H]⁺ = 447.2.
¹H-NMR (300 MHz, MeOD-*d*₄) δ: ppm 9.73 (m, 2H), 8.52 (s, 1H), 7.16 (dd, J = 29.5, 17.7 Hz, 6H), 6.63 (d, J = 8.6 Hz, 1H), 5.29 (d, J = 47.5 Hz, 2H), 4.03 (d, J = 7.1 Hz, 3H), 3.49 - 3.16 (m, 5H), 3.03 - 2.55 (m, 2H), 2.25 - 1.68 (m, 2H), 1.19 (dd, J = 16.1, 9.0 Hz, 2H).

### Example 23: 4-(2-hydroxypropylamino)-2-(3-hydroxy-2,3,4,5-tetrahydro-benzo[1,4]oxazin-7-amino)pyrimidin-5-carboxamide (23)

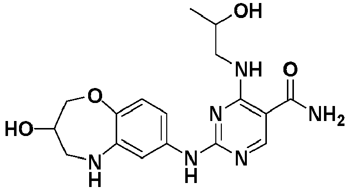

The compound of this example can be prepared according to the similar steps of the aforementioned method for preparing example 1, except that in the reaction of step 3, 2-hydroxypropylamine was used as a raw material in place of cyclobutylamine. The relevant characterization data was as follows:
ESI-MS: [M+H]⁺=375.1.
¹H-NMR (300 MHz, DMSO-d6) δ: 10.33 (s, 1H), 10.02 (s, 1H), 8.42 (s, 1H), 8.13-8.11 (m, 1H), 7.76 (s, 1H), 6.90-6.76 (m, 3H), 4.21-4.16 (m, 1H), 3.92-3.88 (m, 1H), 3.87-3.85 (m, 2H), 3.83-3.79 (m, 1H), 3.54-3.53 (m, 2H), 3.35-3.26 (m, 1H), 1.10-1.08 (J =6.3Hz,d, 3H).

### Example 24: 4-cyclopropylamino-2-(3-methoxy-2,3,4,5-tetrahydro-benzo[1,4]oxazin-7-amino)pyrimidin-5-carboxamide (24)

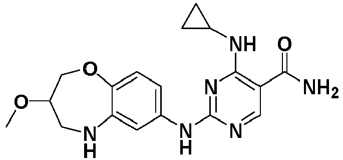

The compound of this example can be prepared according to the similar steps of the aforementioned method for preparing example 1, expect that in the last step, 3-methoxy-7-amino-2,3,4,5-tetrahydro-benzo[1,4]oxazine **(23-2)** was used in place of 7-amino-2,3,4,5-tetrahydro-benzo[1,4]oxazin-3-ol **(1-2)** as the aminobenzoxazine intermediate.

The synthetic route of the 3-methoxy-7-amino-2,3,4,5-tetrahydro-benzo[1,4]oxazine **(23-2)** intermediate was as follows:

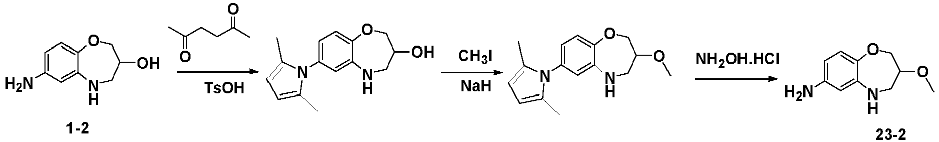

The specific synthetic method is:

### (1) Synthesis of 7-(2,5-dimethyl-1H-pyrrol-1-yl)-3-hydroxy-2,3,4,5-tetrahydrobenzoxazine

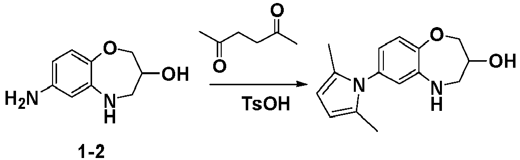

To a 50 mL round-bottom flask were added substance **1-2** (180 mg, 1 mmol), p-toluenesulfonic acid (20 mg, 0.1 mmol), and 2,5-hexanedione (171.25 mg, 1.5 mmol), followed by toluene. The reaction was refluxed for 3 hours while separating water with a water separator. After the reaction was completed, the reaction solvent was rotary evaporated to dryness. To the residue was added silica gel. The mixture was purified by silica gel column chromatography to give 200 mg of the product with a yield of 76%.

### (2) Synthesis of 3-methoxy-7-(2,5-dimethyl-1H-pyrrol-1-yl)-2,3,4,5-tetrahydrobenzoxazine

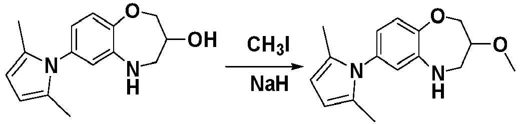

NaH (60%, 10 mg, 0.223 mmol) was suspended in anhydrous THF (3 mL). To the mixture was added a 2 mL solution of a substance (6-(2,5-dimethyl-1H-pyrrol-1-yl)-3-hydroxymethyl-benzoxazine) in THF (50 mg, 0.194 mmol) dropwise in an ice bath. After the dropwise addition was completed, the system was stirred under ice-bath for 1 hour; and then methyl iodide (33 mg, 0.223 mmol) was added dropwise. The mixture was reacted for 10 hours at a temperature from an ice bath to room temperature. After the reaction was completed, the reaction solution was poured into water (5 mL), and extracted with ethyl acetate (5 mL × 3). The organic phases were combined, washed with saturated brine, dried over anhydrous sodium sulfate, and rotary evaporated to dryness. The residue was purified by silica gel column chromatography to give 35 mg of the product with a yield of 50%.

### (3) Synthesis of 3-methoxy-7-amino-2,3,4,5-tetrahydro-benzo[1,4]oxazine (23-2)

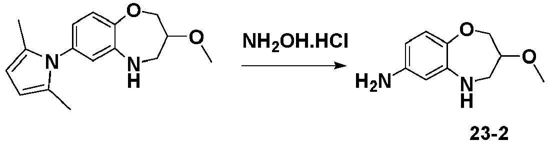

3-methoxy-7-(2,5-dimethyl-1H-pyrrol-1-yl)-2,3,4,5-tetrahydrobenzoxazine (50 mg, 0.25 mmol) was dissolved in 15 mL of anhydrous ethanol. To the mixture was added hydroxylamine hydrochloride (80 mg, 2.5 mmol), and reacted for 48 hours under reflux of ethanol. After the reaction was completed, the ethanol was concentrated and removed. The residue was purified by silica gel column chromatography to give 15 mg of the product **(23-2)** with a yield of 28.5%. The relevant characterization data was as follows:
ESI-MS: [M+H]⁺ = 371.1.
¹H-NMR (300 MHz, MeOD-*d*₄) δ: ppm 8.35 (s, 2H), 7.24 - 6.91 (m, 2H), 6.73 (d, J = 8.5 Hz, 2H), 4.37 - 3.92 (m, 2H), 3.64 (s, 3H), 3.41 (s, 2H), 2.90 (s, 2H), 1.31 (m, 3H), 0.84 (d, J = 5.7 Hz, 2H), 0.55 (s, 2H).

### Example 25: (R)-4-cyclopropylamino-2-(3-hydroxy-2,3,4,5-tetrahydro-benzo[1,4]oxazin-7-amino)pyrimidin-5-carboxamide (25)

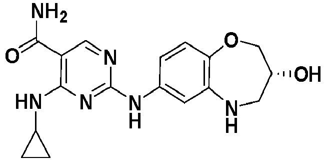

The compound of this example can be prepared according to the similar steps of the aforementioned method for preparing example 2, except that (R)-hydroxymethyloxirane was used as a raw material to prepare the intermediate (R)-7-amino-2,3,4,5-tetrahydrobenzo[1,4]oxazin-3-ol.
ESI-MS: [M+H]⁺ = 357.1.
¹H-NMR (300 MHz, DMSO-*d*₆) δ: ppm 9.27 (s, 1H), 9.09 (d, J= 3.3 Hz, 1H), 8.47 (s, 1H), 7.76 (s, 1H), 7.23 (d, J= 2.1 Hz, 1H), 7.11 (d, J= 7.8 Hz, 2H), 6.65 (d, J= 8.4 Hz, 1H), 5.15 (s, 1H), 4.96 (d, J= 5.4 Hz, 1H), 4.11-4.17 (m, 1H), 3.72-3.88 (m, 2H), 3.17 (d, J=4.8 Hz, 1H), 2.85-3.03 (m, 2H), 0.76-0.83 (m, 2H), 0.46-0.51 (m, 2H).

### Example 26: (S)-4-cyclopropylamino-2-(3-hydroxy-2,3,4,5-tetrahydro-benzo[1,4]oxazin-7-amino)pyrimidin-5-carboxamide (26)

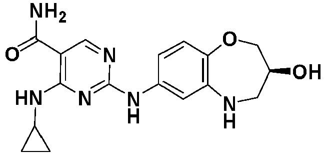

The compound of this example can be prepared according to the similar steps of the aforementioned method for preparing example 2, except that (S)-hydroxymethyloxirane was used as a raw material to prepare the intermediate (S)-7-amino-2,3,4,5-tetrahydrobenzo[1,4]oxazin-3-ol.
ESI-MS: [M+H]⁺ = 357.1.
¹H-NMR (300 MHz, DMSO-*d*₆) δ: ppm 9.27 (s, 1H), 9.09 (d, J= 3.3 Hz, 1H), 8.47 (s, 1H), 7.76 (s, 1H), 7.23 (d, J= 2.1 Hz, 1H), 7.11 (d, J= 7.8 Hz, 2H),6.65 (d, J= 8.4 Hz, 1H), 5.15 (s, 1H), 4.96 (d, J= 5.4 Hz, 1H), 4.11-4.17 (m, 1H), 3.72-3.88 (m, 2H), 3.17 (d, J=4.8 Hz, 1H), 2.85-3.03 (m, 2H), 0.76-0.83 (m, 2H), 0.46-0.51 (m, 2H).

### Example 27: (S)-4-cyclopropylamino-2-(3-methoxy-2,3,4,5-tetrahydro-benzo[1,4]oxazin-7-amino)pyrimidin-5-carboxamide (27)

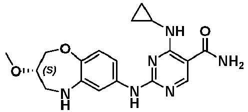

The compound of this example can be prepared according to the similar steps of the aforementioned method for preparing example 1, except that in the last step of the reaction, (S)-3-methoxy-7-amino-2,3,4,5-tetrahydro-benzo[1,4]oxazine **(26-2)** was used in place of 7-amino-2,3,4,5-tetrahydro-benzo[1,4]oxazin-3-ol **(1-2)** as the aminobenzoxazine intermediate.
ESI-MS: [M+H]⁺ = 371.1.
¹H-NMR (300 MHz, DMSO-*d*₆) δ: ppm 10.75 (s, 1H), 9.97 (d, J= 3.9 Hz, 1H), 8.59 (s, 1H), 8.28 (br, 1H), 7.72 (br, 1H), 7.12 (m, 2H), 6.85 (d, J= 8.1 Hz, 1H), 4.22-4.10 (m, 2H), 3.68-3.66 (m, 1H), 3.44-3.38 (m, 1H), 3.32 (s, 1H), 3.30-3.03 (m, 1H), 2.51-2.49 (m, 1H), 0.88-0.86 (m, 2H), 0.66-0.65 (m, 2H).

The synthetic route of (S)-3-methoxy-7-amino-2,3,4,5-tetrahydro-benzo[1,4]oxazine **(26-2)** intermediate was as follows:

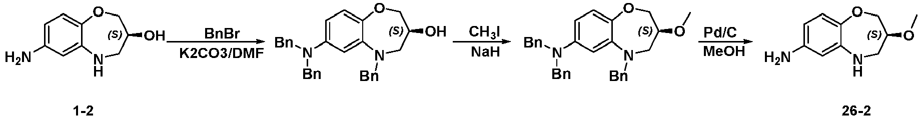

The specific synthetic method is:

### (1) Synthesis of (S)-5-benzyl-7-(dibenzylamino)-2,3,4,5-tetrahydro-benzo[b][1,4]oxazin-3-ol

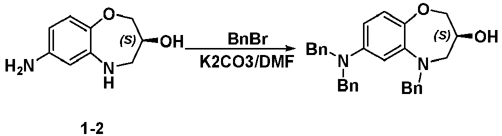

To a 100 mL round-bottom flask were added DMF (10 mL), substance 1-2 (180 mg, 1 mmol), and potassium carbonate (498 mg, 3.6 mmol). To the mixture was added benzyl bromide (513 mg, 3.3 mmol), and stirred at room temperature for 12 hours. After the reaction was completed, to the reaction solution were added water (10 mL) and ethyl acetate (50 mL), and the mixture was separated. The organic phase was washed 3 times with saturated ammonium chloride and then washed twice with water. The mixture was dried over anhydrous magnesium sulfate and filtered, and the filtrate was concentrated under reduced pressure. The concentrate was purified by silica gel column chromatography to give 405 mg of the product with a yield of 90%.

### (2) Synthesis of (S)-N,N,5-tribenzyl-2,3,4,5-tetrahydro-3-methoxybenzo[b][1,4]oxazin-7-amine

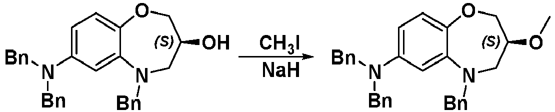

(S)-5-Benzyl-7-(dibenzylamino)-2,3,4,5-tetrahydro-benzo[b][1,4]oxazin-3-ol (450 mg, 1 mmol) was dissolved in anhydrous THF (30 mL) at room temperature. To the mixture was added NaH (60%, 40 mg, 1.2 mmol) in portions in an ice bath, and stirred for 10 minutes. Methyl iodide (170 mg, 1.2 mmol) was then added. The system was stirred for an additional hour in an ice bath. After the reaction was completed, the reaction was quenched with water (5 mL), and extracted with ethyl acetate (20 mL × 3). The organic phases were combined, washed with saturated brine, dried over anhydrous sodium sulfate, and rotary evaporated to dryness. The residue was purified by silica gel column chromatography to give 422 mg of the product with a yield of 91%.

### (3) Synthesis of (S)-3-methoxy-7-amino-2,3,4,5-tetrahydro-benzo[1,4]oxazine (23-2)

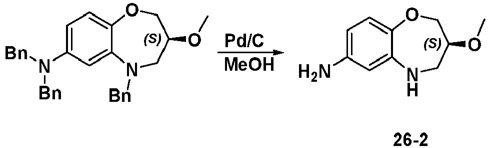

(S)-N,N,5-tribenzyl-2,3,4,5-tetrahydro-3-methoxybenzo[b][1,4]oxazin-7-amine (464 mg, 1 mmol) was dissolved in 20 mL of anhydrous methanol. To the mixture was added Pd/C (50 mg), and hydrogenated at room temperature and normal pressure for 2 hours. After the reaction was completed, the reaction solution was filtered and concentrated to give 194 mg of the product (26-2) with a yield of 100%.

### Example 28: 4-(1-hydroxy-2-methylpropyl-2-amino)-2-((S)-2,3,4,5-tetrahydro-3-hydroxybenzo[1,4]oxazin-7-amino)pyrimidin-5-carboxamide (28)

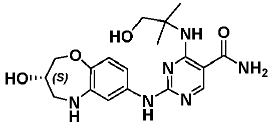

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 389.1.

### Example 29: 4-(1H-indol-4-amino)-2-((S)-2,3,4,5-tetrahydro-3-hydroxybenzo[1,4]oxazin-7-amino)pyrimidin-5-carboxamide (29)

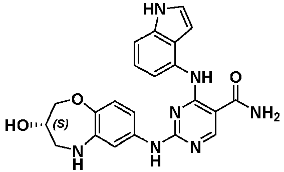

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 432.1.

### Example 30: 4-isopropylamino-2-((S)-2,3,4,5-tetrahydro-3-hydroxybenzo[1,4]oxazin-7-amino)pyrimidin-5-carboxamide (30)

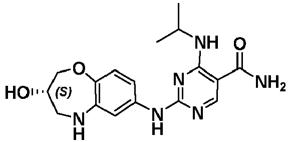

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 359.1.

### Example 31: 4-(1H-pyrazol-4-amino)-2-((S)-2,3,4,5-tetrahydro-3-hydroxybenzo[1,4]oxazin-7-amino)pyrimidin-5-carboxamide (31)

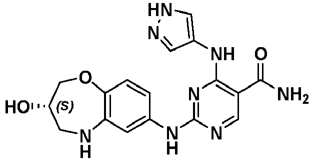

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 383.1.

### Example 32: 4-(2-trifluoromethylethylamino)-2-((S)-2,3,4,5-tetrahydro-3-hydroxybenzo[1,4]oxazin-7-amino)pyrimidin-5-carboxamide (32)

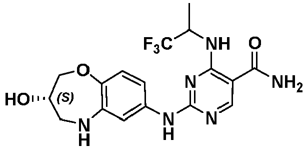

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 413.2.
¹H-NMR (300 MHz, MeOD-*d*₄) δ: ppm 8.41 (s, 1H), 6.88-6.94 (m, 2H), 6.79-6.82 (m, 1H), 4.21-4.30 (m, 2H), 4.02-4.10 (m, 2H), 3.34-3.50 (m, 2H),1.31 (s, 3H).

### Example 33: 4-cyclohexylamino-2-((S)-2,3,4,5-tetrahydro-3-hydroxybenzo[1,4]oxazin-7-amino)pyrimidin-5-carboxamide (33)

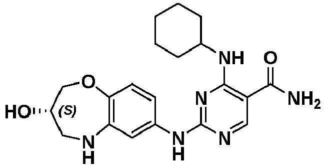

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 399.2.

### Example 34: 4-(1H-indol-5-amino)-2-((S)-2,3,4,5-tetrahydro-3-hydroxybenzo[1,4]oxazin-7-amino)pyrimidin-5-carboxamide (34)

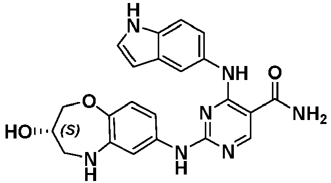

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 432.1.

### Example 35: 4-(3-(1,2,4-triazole)-3-phenylamino)-2-((S)-2,3,4,5-tetrahydro-3-hydroxybenzo[1,4]oxazin-7-amino)pyrimidin-5-carboxamide (35)

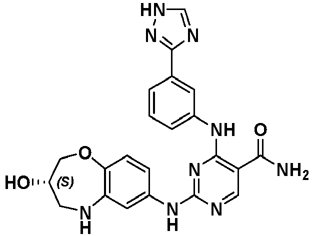

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 460.3.
¹H-NMR (300 MHz, DMSO-*d*₆) δ: ppm 11.85 (s, 1H), 9.76-9.77 (m, 1H), 8.65 (s, 1H), 8.46 (s, 1H), 8.02-8.19 (m, 2H), 7.92 (s, 1H), 7.75-7.78 (m, 1H), 7.61 (s, 1H), 7.42-7.48 (m, 1H), 6.92 (s, 1H), 6.59 (s, 1H), 3.25-3.31 (m, 2H), 3.00-3.06 (m, 2H), 2.27-2.28 (m, 2H).

### Example 36: 4-(3-hydroxycyclobutylamino)-2-((S)-2,3,4,5 -tetrahydro-3-hydroxybenzo[1,4]oxazin-7-amino)pyrimidin-5-carboxamide (36)

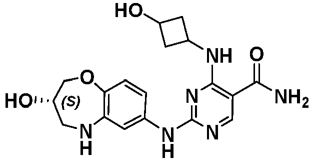

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 387.1.
¹H-NMR (300 MHz, DMSO-*d*₆) δ: ppm 10.02 (br, 1H), 9.87 (br, 1H), 8.39 (s, 1H), 8.09 (br, 1H), 7.56 (br, 1H), 6.93 (m, 2H), 6.76 (d, J = 8.1, 1H), 4.52-4.49 (m, 1H), 4.32-4.28 (m, 1H), 4.21-4.16 (m, 1H), 3.90-3.81 (m, 2H), 3.36-3.30 (m, 1H), 3.09-3.03 (m, 1H), 2.26-2.22 (m, 4H).

### Example 37: 4-(3-hydroxycyclopentylamino)-2-((S)-2,3,4,5-tetrahydro-3-hydroxybenzo[1,4]oxazin-7-amino)pyrimidin-5-carboxamide (37)

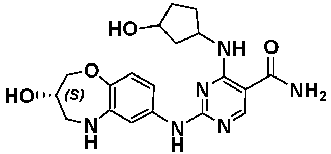

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 401.1.
¹H-NMR (300 MHz, DMSO-*d*₆) δ: ppm 10.11 (br, 1H), 10.03 (br, 1H), 8.36 (s, 1H), 8.07 (br, 1H), 7.53 (br, 1H), 6.85 (m, 2H), 6.77 (d, J = 8.7, 1H), 4.46-4.42 (m, 1H), 4.20-4.16 (m, 2H), 3.89-3.79 (m, 2H), 3.68 (br, 1H), 3.35-3.29 (m, 1H), 3.08-3.01 (m, 1H), 2.12-2.04 (m, 2H), 1.67-1.47 (m, 4H).

### Example 38: 4-(1-hydroxyadamantan-4-amino)-2-((S)-2,3,4,5-tetrahydro-3-hydroxybenzo[1,4]oxazin-7-amino)pyrimidin-5-carboxamide (38)

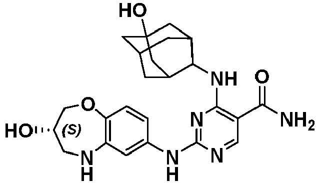

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 467.2.
¹H-NMR (300 MHz, DMSO-*d*₆)δ: ppm 10.29 (br, 1H), 10.03 (br, 1H), 8.44 (s, 1H), 8.12 (br, 1H), 7.57 (br, 1H), 6.99 (br, 1H), 6.75-6.72 (m, 2H), 4.21-4.16 (m, 1H), 4.10 (m, 1H), 3.89-3.88 (m, 1H), 3.84-3.79 (m, 1H), 3.35-3.29 (m, 1H), 3.08-3.02 (m, 1H), 2.24-2.19 (m, 3H), 1.69-1.65 (m, 8H), 1.49-1.45 (m, 2H).

### Example 39: 4-(4-hydroxycyclohexylamino)-2-((S)-2,3,4,5-tetrahydro-3-hydroxybenzo[1,4]oxazin-7-amino)pyrimidin-5-carboxamide (39)

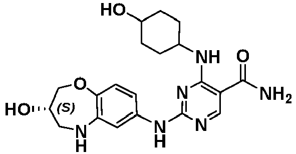

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 415.2.

### Example 40: 4-ethylamino-2-((S)-2,3,4,5-tetrahydro-3-hydroxybenzo[1,4]oxazin-7-amino)pyrimidin-5-carboxamide (40)

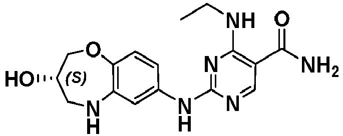

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 345.3.
¹H-NMR (300 MHz, DMSO-*d*₆) δ: ppm 10.29 (s, 1H), 9.79 (s, 1H), 8.40 (s, 1H), 8.10-8.14 (m, 1H), 7.61 (s, 1H), 6.75-6.89 (m, 3H), 4.16-4.21 (m, 2H), 3.85-3.94 (m, 2H), 3.78-3.84 (m, 2H), 3.47-3.54 (m, 1H), 1.15-1.20 (m, 3H).

### Example 41: 4-((1H-pyrrolo[2,3]pyridin-4-methylamino)-2-((S)-2,3,4,5-tetrahydro-3-hydroxybenzo[1,4]oxazin-7-amino)pyrimidin-5-carboxamide (41)

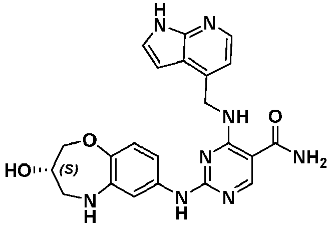

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 447.1.
¹H-NMR (300 MHz, DMSO-*d*₆) δ: ppm 12.05 (s, 1H), 11.65 (d, J = 27.1 Hz, 1H), 10.28 (d, J = 41.8 Hz, 2H), 8.55 (s, 1H), 8.16 (dd, J = 28.5, 5.7 Hz, 2H), 7.57 (t, J = 20.9 Hz, 2H), 7.00 (d, J = 4.7 Hz, 1H), 6.80 (s, 1H), 6.74 - 6.53 (m, 2H), 6.41 (s, 1H), 4.07 (dd, J = 25.2, 9.4 Hz, 2H), 3.95 - 3.69 (m, 3H), 3.24 (d, J = 4.2 Hz, 1H), 3.01 (s, 1H).

### Example 42: 4-((1-cyanoacetyl-3-pyrrolidinylamino)-2-((S)-2,3,4,5-tetrahydro-3-hydroxybenzo[1,4]oxazin-7-amino)pyrimidin-5-carboxamide (42)

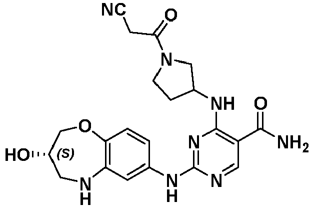

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 453.2.
¹H-NMR (300 MHz, MeOD-*d*₄) δ: ppm 8.36 (s, 1H), 6.93-6.96 (m, 2H), 6.85 (br, 1H), 4.63-4.73 (m, 1H), 4.25-4.30 (m, 1H), 4.02-4.07 (m, 2H), 3.80-3.86 (m, 3H), 3.52-3.70 (m, 4H), 2.14-2.18 (m, 2H).

### Example 43: 4-((1-hydroxyadamantan-3-amino)-2-((S)-2,3,4,5-tetrahydro-3-hydroxybenzo[1,4]oxazin-7-amino)pyrimidin-5-carboxamide (43)

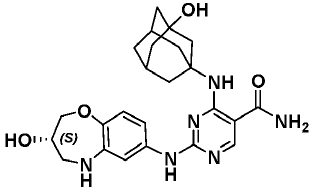

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 467.2.
¹H-NMR (300 MHz, DMSO-*d*₆) δ: ppm 8.30 (m, 1H), 6.91 (d, J = 8.4, 1H), 6.74 (s, 1H), 6.69 (dd, J₁ = 8.4 Hz, J₂ = 2.1 Hz, 1H), 4.35-4.29 (m, 1H), 4.07-4.02 (m, 1H), 3.49-3.43 (m, 1H), 3.33-3.25 (m, 1H), 2.23 (br, 2H), 2.01 (br, 6H), 1.66 (br, 4H), 1.55 (br, 4H).

### Example 44: 4-cyclopropylmethylamino-2-((S)-2,3,4,5-tetrahydro-3-hydroxybenzo[1,4]oxazin-7-amino)pyrimidin-5-carboxamide (44)

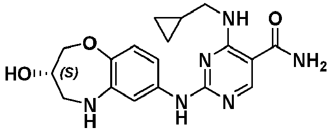

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 371.1.

### Example 45: 4-(1-adamantylamino)-2-((S)-2,3,4,5-tetrahydro-3-hydroxybenzo[1,4]oxazin-7-amino)pyrimidin-5-carboxamide (45)

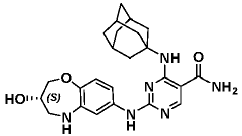

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 451.5.
¹H-NMR (300 MHz, DMSO-*d*₆) δ: ppm 10.48-10.56 (m, 2H), 8.51 (s, 1H), 8.19 (s, 1H), 7.69 (s, 1H), 6.94 (s, 1H), 6.74-6.94 (m, 2H), 4.16-4.21 (m, 2H), 3.79-3.93 (m, 2H), 3.29-3.35 (m, 1H), 3.01-3.08 (m, 1H), 1.99 (s, 1H), 1.63-1.85 (m, 13H).

### Example 46: 4-(1-methoxyadamantan-4-amino)-2-((S)-2,3,4,5-tetrahydro-3-hydroxybenzo[1,4]oxazin-7-amino)pyrimidin-5-carboxamide (46)

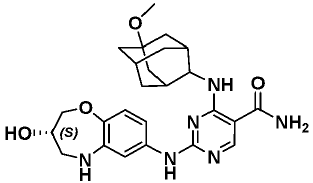

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 481.2.
¹H-NMR (300 MHz, DMSO-*d*₆) δ: ppm 8.18 (m, 1H), 6.90 (d, J = 8.7, 1H), 6.77 (m, 2H), 4.31-4.27 (m, 2H), 4.06-4.03 (m, 1H), 3.49-3.43 (m, 1H), 3.26-3.21 (m, 1H), 2.88 (m, 3H), 2.24-2.19 (m, 3H), 1.90-1.82 (m, 8H), 1.65-1.60 (m, 2H).

### Example 47: 4-methylamino-2-((S)-2,3,4,5-tetrahydro-3-hydroxybenzo[1,4]oxazin-7-amino)pyrimidin-5-carboxamide (47)

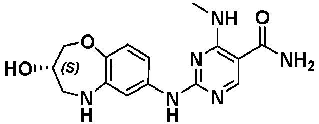

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 331.1.
¹H-NMR (300 MHz, DMSO-*d*₆) δ: ppm 10.48 (s, 1H), 9.80 (s, 1H), 8.49 (s, 1H), 8.18 (s, 1H), 7.65 (s, 1H), 7.01 (s, 1H), 6.82 (t, J = 12.5 Hz, 2H), 4.18 (dd, J = 11.8, 3.2 Hz, 1H), 4.00 - 3.72 (m, 2H), 3.33 (dd, J = 13.0, 4.6 Hz, 1H), 3.16 - 2.74 (m, 4H).

### Example 48: 4-(4-hydroxybutylamino)-2-((S)-2,3,4,5-tetrahydro-3-hydroxybenzo[1,4]oxazin-7-amino)pyrimidin-5-carboxamide (48)

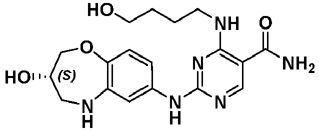

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 389.1.

### Example 49: 4-(4-hydroxyadamantylamino)-2-((S)-2,3,4,5-tetrahydro-3-hydroxybenzo[1,4]oxazin-7-amino)pyrimidin-5-carboxamide (49)

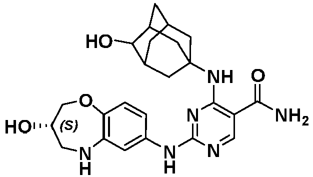

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 467.2.

### Example 50: 4-(cyclohexylmethylamino)-2-((S)-2,3,4,5-tetrahydro-3-hydroxybenzo[1,4]oxazin-7-amino)pyrimidin-5-carboxamide (50)

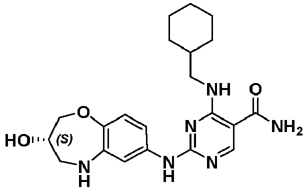

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 413.2.
¹H-NMR (300 MHz, DMSO-*d₆*) δ: ppm 9.16 (s 1H), 8.44 (s, 1H), 7.72 (br, 1H), 7.06-6.97 (m, 2H), 6.62 (d*, J* = 8.4 Hz, 1H), 5.13 (d, *J* = 16.8 Hz, 1H), 4.95 (d, *J* = 5.2 Hz, 1H), 4.16-4.10 (m, 1H), 3.83-3.81 (m, 1H), 3.75-3.69 (m, 1H), 3.29-3.25 (m, 1H), 3.01-2.94 (m, 1H), 1.70-1.63 (m, 6H), 1.12-1.10 (m, 5H).

### Example 51: 4-(3-hydroxypropylamino)-2-((S)-2,3,4,5-tetrahydro-3-hydroxybenzo[1,4]oxazin-7-amino)pyrimidin-5-carboxamide (51)

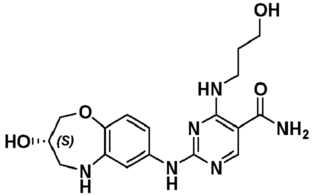

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 375.1.

### Example 52: 4-(3 -methoxypropylamino)-2-((S)-2,3,4,5 -tetrahydro-3 - hydroxybenzo[1,4]oxazin-7-amino)pyrimidin-5-carboxamide (52)

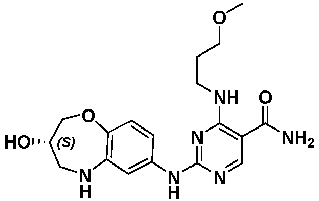

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 389.1.

### Example 53: 4-tert-butylamino-2-((S)-2,3,4,5-tetrahydro-3-hydroxybenzo[1,4]oxazin-7-amino)pyrimidin-5-carboxamide (53)

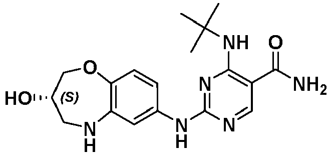

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 373.1.
¹H-NMR (300 MHz, DMSO-*d₆*) δ ppm: 9.25 (s 1H), 9.02 (s, 1H), 8.42 (s, 1H), 7.72 (br, 1H), 6.94-6.91 (m, 2H), 6.65 (d, *J* = 8.6 Hz, 1H), 5.13 (d, *J* = 16.8 Hz, 1H), 4.95 (d*, J* = 5.2 Hz, 1H), 4.17-4.41 (m, 1H), 3.85-3.83 (m, 1H), 3.76-3.70 (m, 1H), 3.34-3.26 (m, 1H), 3.02-2.95 (m, 1H), 1.40 (s, 9H).

### Example 54: 4-(3,3-difluorocyclobutylamino)-2-((S)-2,3,4,5-tetrahydro-3-hydroxybenzo[1,4]oxazin-7-amino)pyrimidin-5-carboxamide (54)

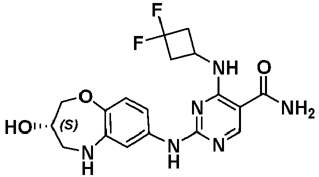

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 407.3.

### Example 55: 4-(1-cyclopropylethylamino)-2-((S)-2,3,4,5-tetrahydro-3 - hydroxybenzo[1,4]oxazin-7-amino)pyrimidin-5-carboxamide (55)

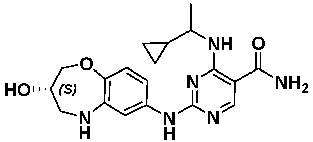

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 385.1.

### Example 56: 4-(cyclobutylmethylamino)-2-((S)-2,3,4,5-tetrahydro-3-hydroxybenzo[1,4]oxazin-7-amino)pyrimidin-5-carboxamide (56)

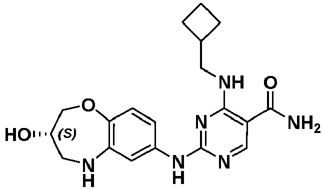

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 385.1.
¹H-NMR (300 MHz, DMSO-*d*₆) δ: ppm 10.37 (s, 1H), 9.96 (s, 1H), 8.49 (s, 1H), 8.21 (s, 1H), 7.65 (s, 1H), 7.02 - 6.70 (m, 3H), 4.18 (dd, J = 11.9, 3.6 Hz, 1H), 3.87 (ddd, J = 22.9, 11.3, 5.7 Hz, 2H), 3.52 (t, J = 6.4 Hz, 2H), 3.32 (dd, J = 13.0, 4.8 Hz, 1H), 3.06 (dd, J = 12.9, 7.1 Hz, 1H), 2.69 - 2.50 (m, 3H), 2.08 - 1.91 (m, 2H), 1.91 - 1.60 (m, 4H).

### Example 57: 4-(2-fluorocyclopropylamino)-2-((S)-2,3,4,5-tetrahydro-3-hydroxybenzo[1,4]oxazin-7-amino)pyrimidin-5-carboxamide (57)

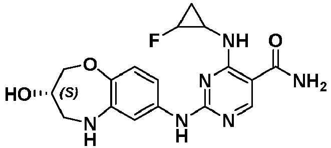

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 375.1.
¹H-NMR (300 MHz, DMSO-*d₆*) δ: ppm 10.76 (s, 1H), 10.14 (d, J = 4.2, 1H), 8.64 (s, 1H), 8.28 (br, 1H), 7.72 (br, 1H), 7.37 (br, 1H), 7.16 (br, 1H), 6.95 (d, J = 8.6, 1H), 5.08-5.04 (m, 1H), 4.87-4.82 (m, 1H), 4.18-4.13 (m, 1H), 4.01-3.97 (m, 1H), 3.99-3.88 (m, 1H), 3.38-3.33 (m, 1H), 3.23-3.18 (m, 1H), 1.33-1.22 (m, 1H), 1.16-1.10 (m, 1H), 1.07-1.01 (m, 1H).

### Example 58: 4-(azetidin-3-amino)-2-((S)-2,3,4,5-tetrahydro-3-hydroxybenzo[1,4]oxazin-7-amino)pyrimidin-5-carboxamide (58)

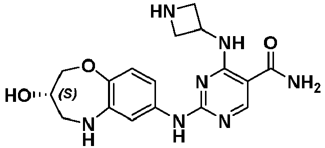

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 372.1.

### Example 59: 4-(azetidin-3-amino)-2-((S)-2,3,4,5-tetrahydro-3-hydroxybenzo[1,4]oxazin-7-amino)pyrimidin-5-carboxamide (59)

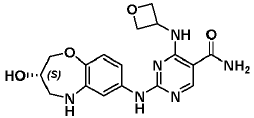

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 373.4.
¹H-NMR (300 MHz, DMSO-*d*₆) δ: ppm 8.58 (s, 1H), 6.92-6.96 (m, 2H), 6.79-6.89 (m, 1H), 4.54-4.60 (m, 2H), 4.26-4.47 (m, 2H), 4.07-4.11 (m, 2H), 4.02-4.04 (m, 1H), 3.83-3.88 (m, 1H), 3.29-3.48 (m, 1H), 3.24-3.29 (m, 1H).

### Example 60: 4-allylamino-2-((S)-2,3,4,5-tetrahydro-3-hydroxybenzo[1,4]oxazin-7-amino)pyrimidin-5-carboxamide (60)

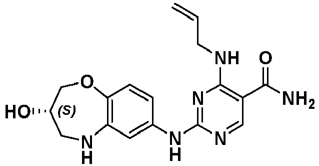

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 357.1

### Example 61: 4-(2-morpholinoethylamino)-2-((S)-2,3,4,5-tetrahydro-3-hydroxybenzo[1,4]oxazin-7-amino)pyrimidin-5-carboxamide (61)

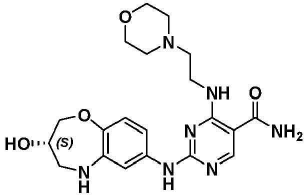

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 430.2.

### Example 62: 4-(3 -morpholinopropylamino)-2-((S)-2,3,4,5 -tetrahydro-3 - hydroxybenzo[1,4]oxazin-7-amino)pyrimidin-5-carboxamide (62)

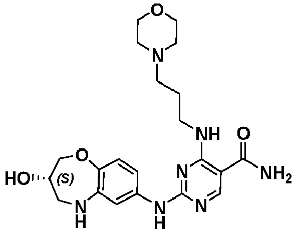

The compound of this example can be prepared according to the similar steps of the aforementioned method for preparing example 2, except that the intermediate (S)-7-amino-2,3,4,5-tetrahydro-benzo[1,4]oxazin-3-ol was used.
ESI-MS: [M+H]⁺ = 444.2.

### Example 63: 4-(cyclopropylmethylamino)-2-((S)-2,3,4,5-tetrahydro-3-hydroxybenzo[1,4]oxazin-7-amino)pyrimidin-5-carboxamide (63)

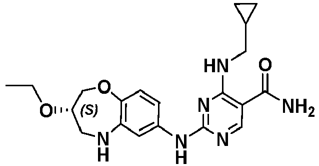

The compound of this example can be prepared according to the similar steps of the aforementioned method for preparing example 26, except that the intermediate (S)-3-ethoxy-2,3,4,5-tetrahydro-benzo][1,4]oxazin-7-amine was used.
ESI-MS: [M+H]⁺ = 399.2.
¹H-NMR (300 MHz, DMSO-*d*₆) δ: ppm 11.65 (s, 1H), 9.99 (s, 1H), 8.09 (s, 2H), 7.68 (s, 1H), 6.87 (d, J = 8.3 Hz, 1H), 6.61 (dd, J = 21.9, 5.3 Hz, 2H), 4.59 - 3.51 (m, 11H), 1.15 (t, J = 7.0 Hz, 3H), 0.50 (d, J = 7.2 Hz, 2H), 0.29 (s, 2H).

### Example 64: 4-(tetrahydrofuran-3-amino)-2-((S)-2,3,4,5-tetrahydro-3-hydroxybenzo[1,4]oxazin-7-amino)pyrimidin-5-carboxamide (64)

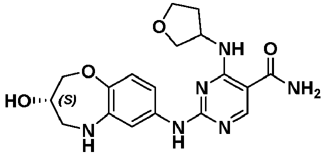

The compound of this example can be prepared according to the similar steps of the aforementioned method for preparing example 26, except that the intermediate (S)-7-amino-2,3,4,5-tetrahydro-benzo[1,4]oxazin-3-ol was used.
ESI-MS: [M+H]⁺ = 387.1.

### Example 65: 4-propynylamino-2-((S)-2,3,4,5-tetrahydro-3-hydroxybenzo[1,4]oxazin-7-amino)pyrimidin-5-carboxamide (65)

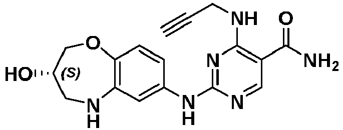

The compound of this example can be prepared according to the similar steps of the aforementioned method for preparing example 26, except that the intermediate (S)-7-amino-2,3,4,5-tetrahydro-benzo[1,4]oxazin-3-ol was used.
ESI-MS: [M+H]⁺ = 355.1.

### Example 66: 4-(butyn-3-amino)-2-((S)-2,3,4,5-tetrahydro-3-hydroxybenzo[1,4]oxazin-7-amino)pyrimidin-5-carboxamide (66)

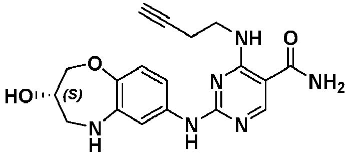

The compound of this example can be prepared according to the similar steps of the aforementioned method for preparing example 26, except that the intermediate (S)-7-amino-2,3,4,5-tetrahydro-benzo[1,4]oxazin-3-ol was used.
ESI-MS: [M+H]⁺ = 369.1

### Example 67: 4-(2-methanesulfonylethylamino)-2-((S)-2,3,4,5-tetrahydro-3-hydroxybenzo[1,4]oxazin-7-amino)pyrimidin-5-carboxamide (67)

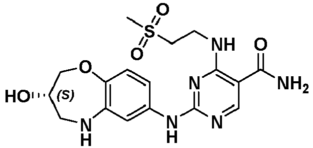

The compound of this example can be prepared according to the similar steps of the aforementioned method for preparing example 26, except that the intermediate (S)-7-amino-2,3,4,5-tetrahydro-benzo[1,4]oxazin-3-ol was used.
ESI-MS: [M+H]⁺ = 423.4.
¹H-NMR (300 MHz, MeOD-*d*₄) δ: ppm 8.35 (s, 1H), 7.11-7.12 (m, 1H), 6.95-6.98 (m, 1H), 6.89 (s, 1H), 4.23-4.29 (m, 2H), 4.05-4.10 (m, 4H), 3.44-3.52 (m, 3H), 3.02 (s, 3H).

### Example 68: 4-(1-methylcyclopropylamino)-2-((S)-2,3,4,5-tetrahydro-3-hydroxybenzo[1,4]oxazin-7-amino)pyrimidin-5-carboxamide (68).

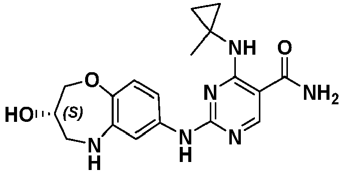

The compound of this example can be prepared according to the similar steps of the aforementioned method for preparing example 26, except that the intermediate (S)-7-amino-2,3,4,5-tetrahydro-benzo[1,4]oxazin-3-ol was used.
ESI-MS: [M+H]^{+ =} 371.1.
¹H-NMR (300 MHz, DMSO-*d*₆) δ: ppm 10.74 (s, 1H), 10.14 (s, 1H), 8.58 (s, 1H), 8.26 (s, 1H), 7.74 (s, 1H), 7.51 (s, 1H), 7.14 - 6.92 (m, 2H), 4.15 (dd, J = 12.0, 3.0 Hz, 1H), 3.94 (dt, J = 16.6, 5.9 Hz, 2H), 3.36 (dd, J = 13.0, 4.2 Hz, 1H), 3.14 (dd, J = 13.0, 6.7 Hz, 1H), 1.39 (s, 3H).

### Example 69: 4-propylamino-2-((S)-2,3,4,5-tetrahydro-3-hydroxybenzo[1,4]oxazin-7-amino)pyrimidin-5-carboxamide (69)

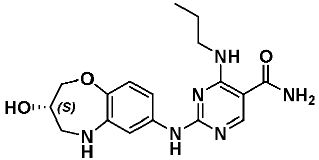

The compound of this example can be prepared according to the similar steps of the aforementioned method for preparing example 26, except that the intermediate (S)-7-amino-2,3,4,5-tetrahydro-benzo[1,4]oxazin-3-ol was used.
ESI-MS: [M+H]⁺ = 359.1.

### Example 70: 4-isobutylamino-2-((S)-2,3,4,5-tetrahydro-3-hydroxybenzo[1,4]oxazin-7-amino)pyrimidin-5-carboxamide (70)

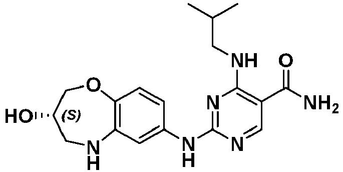

The compound of this example can be prepared according to the similar steps of the aforementioned method for preparing example 26, except that the intermediate (S)-7-amino-2,3,4,5-tetrahydro-benzo[1,4]oxazin-3-ol was used.
ESI-MS: [M+H]⁺ = 373.1.

### Example 71: 4-(2-ethylsulfonylaminoethylamino)-2-((S)-2,3,4,5-tetrahydro-3-hydroxybenzo[1,4]oxazin-7-amino)pyrimidin-5-carboxamide (71)

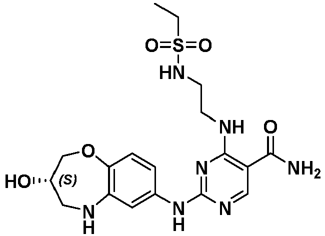

The compound of this example can be prepared according to the similar steps of the aforementioned method for preparing example 26, except that the intermediate (S)-7-amino-2,3,4,5-tetrahydro-benzo[1,4]oxazin-3-ol was used.
ESI-MS: [M+H]⁺ = 452.5.
¹H-NMR (300 MHz, MeOD-*d*₄) δ: ppm 8.30-8.32 (m, 1H), 6.92-6.95 (m, 2H), 6.77-6.80 (m, 1H), 4.25-4.30 (m, 1H), 4.01-4.06 (m, 2H), 3.73-3.79 (m, 4H), 3.63-3.67 (m, 1H), 3.48-3.49 (m, 1H), 3.03-3.12 (m, 2H), 1.29-1.34 (m, 3H).

### Example 72: 4-(pentyn-4-amino)-2-((S)-2,3,4,5-tetrahydro-3-hydroxybenzo[1,4]oxazin-7-amino)pyrimidin-5-carboxamide (72)

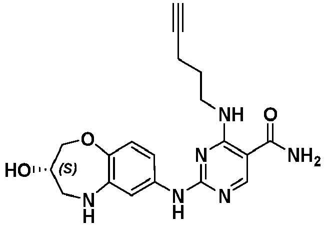

The compound of this example can be prepared according to the similar steps of the aforementioned method for preparing example 26, except that the intermediate (S)-7-amino-2,3,4,5-tetrahydro-benzo[1,4]oxazin-3-ol was used.
ESI-MS: [M+H]⁺ = 383.1.

### Example 73: 4-(cyclopentylmethylamino)-2-((S)-2,3,4,5-tetrahydro-3-hydroxybenzo[1,4]oxazin-7-amino)pyrimidin-5-carboxamide (73)

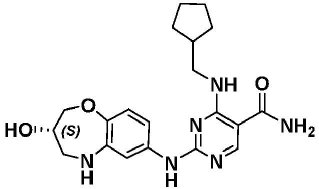

The compound of this example can be prepared according to the similar steps of the aforementioned method for preparing example 26, except that the intermediate (S)-7-amino-2,3,4,5-tetrahydro-benzo[1,4]oxazin-3-ol was used.
ESI-MS: [M+H]⁺ = 399.5.
¹H-NMR (300 MHz, MeOD-*d*₄) δ: ppm 8.30 (s, 1H), 6.93-6.96 (m, 3H), 4.24-4.29 (m, 1H), 4.04-4.10 (m, 2H), 3.43-3.51 (m, 3H), 2.20-2.30 (m, 1H), 1.57-1.86 (m, 7H), 1.13-1.35 (m, 2H).

### Example 74: 4-butylamino-2-((S)-2,3,4,5-tetrahydro-3-hydroxybenzo[1,4]oxazin-7-amino)pyrimidin-5-carboxamide (74)

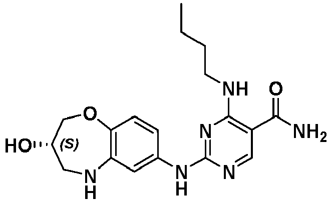

The compound of this example can be prepared according to the similar steps of the aforementioned method for preparing example 26, except that the intermediate (S)-7-amino-2,3,4,5-tetrahydro-benzo[1,4]oxazin-3-ol was used.
ESI-MS: [M+H]⁺ = 373.1.
¹H-NMR (300 MHz, DMSO-*d*₆) δ: ppm 10.62 (s, 1H), 10.01 (s, 1H), 8.45 (s, 1H), 8.14 (s, 1H), 7.68 (s, 1H), 6.84 (dd, J = 28.1, 9.3 Hz, 3H), 4.18 (dd, J = 11.7, 3.2 Hz, 1H), 4.00 - 3.77 (m, 2H), 3.40 (ddd, J = 17.6, 13.0, 5.7 Hz, 3H), 3.07 (dd, J = 13.0, 7.0 Hz, 1H), 1.54 (dd, J = 14.4, 7.2 Hz, 2H), 1.30 (dt, J = 22.4, 11.3 Hz, 2H), 0.89 (t, J = 7.3 Hz, 3H).

### Example 75: 4-pentylamino-2-((S)-2,3,4,5-tetrahydro-3-hydroxybenzo[1,4]oxazin-7-amino)pyrimidin-5-carboxamide (75)

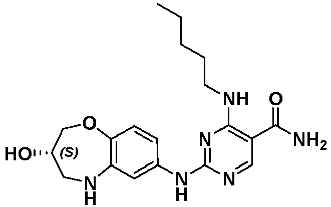

The compound of this example can be prepared according to the similar steps of the aforementioned method for preparing example 26, except that the intermediate (S)-7-amino-2,3,4,5-tetrahydro-benzo[1,4]oxazin-3-ol was used.
ESI-MS: [M+H]⁺ = 387.2.

### Example 76: 4-(but-3-en-1-ylamino)-2-((S)-2,3,4,5-tetrahydro-3-hydroxybenzo[1,4]oxazin-7-amino)pyrimidin-5-carboxamide (76)

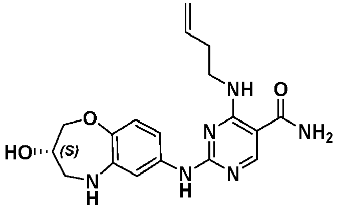

The compound of this example can be prepared according to the similar steps of the aforementioned method for preparing example 26, except that the intermediate (S)-7-amino-2,3,4,5-tetrahydro-benzo[1,4]oxazin-3-ol was used.
ESI-MS: [M+H]⁺ = 371.2.
¹H-NMR (300 MHz, DMSO-*d*₆) δ: ppm 10.80 (s, 1H), 10.06 (t, J = 5.5 Hz, 1H), 8.59 (s, 1H), 8.29 (s, 1H), 7.71 (s, 1H), 7.39 - 7.11 (m, 3H), 6.93 (t, J = 13.1 Hz, 2H), 5.80 (m, 1H), 5.18 - 5.01 (m, 2H), 4.15 (m, 1H), 3.95 - 3.81 (m, 1H), 3.57 (dd, J = 12.6, 6.7 Hz, 2H), 3.33 - 3.39 (m, 1H), 3.23 - 3.08 (m, 2H), 2.33 (q, J = 6.7 Hz, 2H).

### Example 77: 4-(but-3-en-1-ylamino)-2-((S)-2,3,4,5-tetrahydro-3-benzo[1,4]oxazin-7-amino)pyrimidin-5-carboxamide (77)

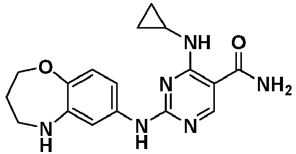

The compound of this example can be prepared according to the similar steps of the aforementioned method for preparing example 26, except that the intermediate (S)-7-amino-2,3,4,5-tetrahydro-benzo[1,4]oxazin-3-ol was used.
ESI-MS: [M+H]⁺ = 341.1.

### Example 78: 4-cyclopropylamino-2-((S)-2,3,4,5-tetrahydro-3-methoxyethoxybenzo[1,4]oxazin-7-amino)pyrimidin-5-carboxamide (78)

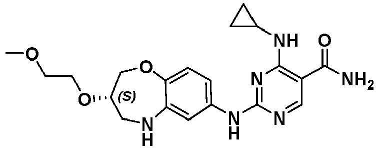

The compound of this example can be prepared according to the similar steps of the aforementioned method for preparing example 26, except that the intermediate (S)-7-amino-2,3,4,5-tetrahydro-3-methoxyethoxybenzo[1,4]oxazine was used.
ESI-MS: [M+H]⁺ = 415.5.
¹H-NMR (300 MHz, MeOD-*d*₄) δ: ppm 8.29 (s, 1H), 6.91-7.01 (m, 3H), 4.26 (s, 2H), 3.86-3.91 (m, 1H), 3.72-3.74 (m, 2H), 3.58-3.60 (m, 2H), 3.45-3.50 (m, 2H), 3.40 (s, 1H), 3.05-3.16 (m, 1H), 0.88-0.97 (m, 2H), 0.70-0.73 (m, 2H).

### Example 79: 4-cyclopropylamino-2-((S)-2,3,4,5-tetrahydro-3-ethoxybenzo[1,4]oxazin-7-amino)pyrimidin-5-carboxamide (79)

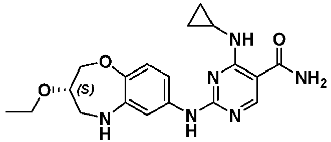

The compound of this example can be prepared according to the similar steps of the aforementioned method for preparing example 26, except that the intermediate (S)-7-amino-2,3,4,5-tetrahydro-3-ethoxybenzo[1,4]oxazine was used.
ESI-MS: [M+H]⁺ = 385.4.
¹H-NMR (300 MHz, MeOD-*d*₄) δ: ppm 8.27 (s, 1H), 6.89-6.91 (m, 3H), 4.17-4.33 (m, 2H), 3.81-3.85 (m, 1H), 3.59-3.66 (m, 2H), 3.42-3.48 (m, 1H), 3.38-3.40 (m, 1H), 3.02-3.12 (m, 1H), 1.21-1.26 (m, 3H), 0.91-0.95 (m, 2H), 0.70-0.73 (m, 2H).

### Example 80: 4-cyclopropylamino-2-((R)-2,3,4,5-tetrahydro-3-methoxybenzo[1,4]oxazin-7-amino)pyrimidin-5-carboxamide (80)

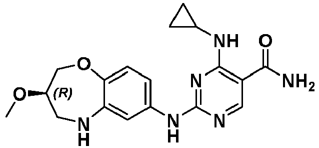

The compound of this example can be prepared according to the similar steps of the aforementioned method for preparing example 26, except that the intermediate (R)-7-amino-2,3,4,5-tetrahydro-3-methoxybenzo[1,4]oxazine was used.
ESI-MS: [M+H]⁺ = 371.1.
¹H-NMR (300 MHz, DMSO-*d*₆) δ ppm: 10.75 (s, 1H), 9.97 (d, J = 8.7, 1H), 8.59 (s, 1H), 8.28 (br, 1H), 7.72 (br, 1H), 7.12 (m, 2H), 6.85 (d, J = 8.7, 1H), 4.22-4.10 (m, 2H), 3.68-3.66 (m, 1H), 3.44-3.38 (m, 1H), 3.32 (s, 3H), 3.30-3.03 (m, 1H), 2.51-2.50 (m, 1H), 0.88-0.86 (m, 2H), 0.66-0.65 (m, 2H).

### Example 81: 4-cyclopropylamino-2-((S)-2,3,4,5-tetrahydro-3-butoxybenzo[1,4]oxazin-7-amino)pyrimidin-5-carboxamide (81)

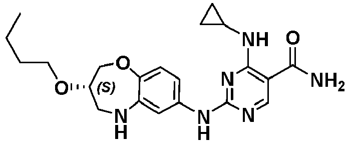

The compound of this example can be prepared according to the similar steps of the aforementioned method for preparing example 26, except that the intermediate (S)-7-amino-2,3,4,5-tetrahydro-3-butoxybenzo[1,4]oxazine was used.
ESI-MS: [M+H]⁺ = 413.5.
¹H-NMR (300 MHz, DMSO-*d*₆) δ ppm: 10.06 (s, 1H), 9.63 (s, 1H), 8.42 (s, 1H), 8.05 (s, 1H), 7.51 (s, 1H), 7.00 (s, 2H), 6.71-6.74 (m, 1H), 4.18-4.22 (m, 1H), 4.04-4.05 (m, 1H), 3.43-4.00 (m, 4H), 3.15-3.22 (m, 2H), 2.93-2.98 (m, 1H), 1.43-1.50 (m, 2H), 1.23-1.39 (m, 2H), 0.82-0.89 (m, 6H), 0.62 (s, 2H).

### Example 82: 4-cyclopropylamino-2-((S)-2,3,4,5-tetrahydro-3-propoxybenzo[1,4]oxazin-7-amino)pyrimidin-5-carboxamide (82)

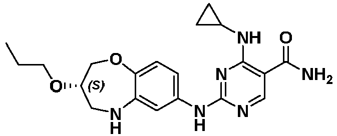

The compound of this example can be prepared according to the similar steps of the aforementioned method for preparing example 26, except that the intermediate (S)-7-amino-2,3,4,5-tetrahydro-3-propoxybenzo[1,4]oxazine was used.
ESI-MS: [M+H]⁺ = 399.5.
¹H-NMR (300 MHz, DMSO-*d*₆) δ: ppm 10.54 (s, 1H), 9.81-9.82 (m, 1H), 8.49 (s, 1H), 8.10 (s, 1H), 7.62 (s, 1H), 7.00 (s, 2H), 6.73-6.76 (m, 1H), 4.19-4.23 (m, 1H), 4.02-4.07 (m, 1H), 3.70-3.75 (m, 1H), 3.36-3.43 (m, 3H), 3.17-3.24 (m, 1H), 2.96-3.02 (m, 1H), 1.45-1.54 (m, 2H), 0.83-0.90 (m, 5H), 0.65-0.75 (m, 2H).

### Example 83: 4-cyclopropylamino-2-((R)-2,3,4,5-tetrahydro-3-ethoxybenzo[1,4]oxazin-7-amino)pyrimidin-5-carboxamide (83)

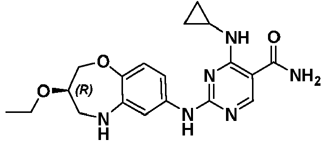

The compound of this example can be prepared according to the similar steps of the aforementioned method for preparing example 26, except that the intermediate (R)-7-amino-2,3,4,5-tetrahydro-3-ethoxybenzo[1,4]oxazine was used.
ESI-MS: [M+H]⁺ = 385.1.
¹H-NMR (300 MHz, CDCl₃) δ: ppm 8.91 (s, 1H), 8.24 (s, 1H), 7.73 (s, 1H), 7.22-7.00 (m, 1H), 6.84 (d, J = 8.6 Hz, 1H), 4.24 (ddd, J = 17.2, 12.5, 5.1 Hz, 1H), 3.87-3.35 (m, 4H), 3.05-2.81 (m, 2H), 1.26 (d, J = 4.2 Hz, 3H), 0.84 (d, J = 7.0 Hz, 2H), 0.64 (d, J = 4.4 Hz, 2H).

### Example 84: 4-cyclopropylamino-2-((R)-2,3,4,5-tetrahydro-3-propoxybenzo[1,4]oxazin-7-amino)pyrimidin-5-carboxamide (84)

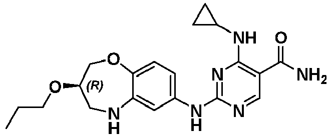

The compound of this example can be prepared according to the similar steps of the aforementioned method for preparing example 26, except that the intermediate (R)-7-amino-2,3,4,5-tetrahydro-3-propoxybenzo[1,4]oxazine was used.
ESI-MS: [M+H]⁺ = 399.2.
¹H-NMR (300 MHz, DMSO-*d*₆) δ: ppm δ 9.26 (s, 0H), 9.09 (s, 0H), 8.43 (d, J = 24.6 Hz, 0H), 7.74 (s, 0H), 7.09 (d, J = 7.3 Hz, 1H), 6.63 (d, J = 8.6 Hz, 0H), 5.22 (s, 0H), 4.07 (ddd, J = 58.4, 12.4, 3.5 Hz, 1H), 3.68 (s, 0H), 3.38 (dd, J = 13.9, 7.3 Hz, 2H), 3.17 (d, J = 7.3 Hz, 0H), 2.50 (s, 1H), 1.50 (dd, J = 13.9, 6.9 Hz, 1H), 0.94 - 0.73 (m, 2H), 0.49 (s, 2H).

### Example 85: 4-cyclopropylamino-2-((R)-2,3,4,5-tetrahydro-3-butoxybenzo[1,4]oxazin-7-amino)pyrimidin-5-carboxamide (85)

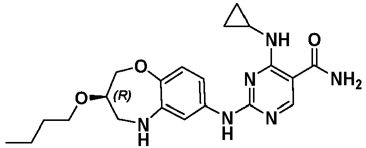

The compound of this example can be prepared according to the similar steps of the aforementioned method for preparing example 26, except that the intermediate (R)-7-amino-2,3,4,5-tetrahydro-3-butoxybenzo[1,4]oxazine was used.
ESI-MS: [M+H]⁺ = 413.5.
¹H-NMR (300 MHz, DMSO-*d*₆) δ ppm: 10.06 (s, 1H), 9.63 (s, 1H), 8.42 (s, 1H), 8.05 (s, 1H), 7.51 (s, 1H), 7.00 (s, 2H), 6.71-6.74 (m, 1H), 4.18-4.22 (m, 1H), 4.04-4.05 (m, 1H), 3.43-4.00 (m, 4H), 3.15-3.22 (m, 2H), 2.93-2.98 (m, 1H), 1.43-1.50 (m, 2H), 1.23-1.39 (m, 2H), 0.82-0.89 (m, 6H), 0.62 (s, 2H).

### Example 86: 4-cyclopropylamino-2-((R)-2,3,4,5-tetrahydro-3-methoxyethoxybenzo[1,4]oxazin-7-amino)pyrimidin-5-carboxamide (86)

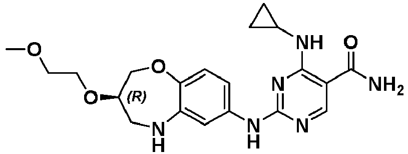

The compound of this example can be prepared according to the similar steps of the aforementioned method for preparing example 26, except that the intermediate (R)-7-amino-2,3,4,5-tetrahydro-3-methoxyethoxybenzo[1,4]oxazine was used.
ESI-MS: [M+H]⁺ = 415.5.
¹H-NMR (300 MHz, MeOD-*d*₄) δ: ppm 8.29 (s, 1H), 6.91-7.01 (m, 3H), 4.26 (s, 2H), 3.86-3.91 (m, 1H), 3.72-3.74 (m, 2H), 3.58-3.60 (m, 2H), 3.45-3.50 (m, 2H), 3.40 (s, 1H), 3.05-3.16 (m, 1H), 0.88-0.97 (m, 2H), 0.70-0.73 (m, 2H).

### Example 87: 4-cyclopropylamino-2-((S)-2,3,4,5-tetrahydro-3-deuteromethoxybenzo[1,4]oxazin-7-amino)pyrimidin-5-carboxamide (87)

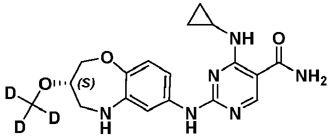

The compound of this example can be prepared according to the similar steps of the aforementioned method for preparing example 26, except that the intermediate (S)-7-amino-2,3,4,5-tetrahydro-3-deuteromethoxybenzo[1,4]oxazine was used.
ESI-MS: [M+H]⁺ = 374.4.
¹H-NMR (300 MHz, DMSO-*d*₆) δ: ppm 10.69 (s, 1H), 9.94-9.95 (m, 1H), 8.58 (s, 1H), 8.27 (s, 1H), 7.72 (s, 1H), 7.05 (s, 2H), 6.79-6.82 (m, 1H), 4.04-4.22 (m, 2H), 3.63-3.65 (m, 1H), 3.37-3.43 (m, 1H), 3.23-3.27 (m, 1H), 2.98-3.06 (m, 1H), 0.67-0.85 (m, 2H), 0.65-0.66 (m, 2H).

### Example 88: 4-cyclopropylamino-2-((S)-2,3,4,5-tetrahydro-3-pentoxybenzo[1,4]oxazin-7-amino)pyrimidin-5-carboxamide (88)

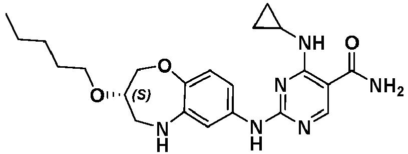

The compound of this example can be prepared according to the similar steps of the aforementioned method for preparing example 26, except that the intermediate (S)-7-amino-2,3,4,5-tetrahydro-3-pentoxybenzo[1,4]oxazine was used.
ESI-MS: [M+H]⁺ = 427.2.
¹H-NMR (300 MHz, CD₃OD-*d*₄) δ: ppm 8.28 (s, 1H), 6.90 (m, 3H), 3.81(m, 1H), 3.60(m, 2H), 3.48(m, 2H), 3.35 (m, 2H), 1.44 (m, 2H), 1.39(m, 2H), 0.97 (m, 4H), 0.70 (m, 2H).

### Example 89: 4-cyclopropylamino-2-((S)-2,3,4,5-tetrahydro-3-(2-(2-methoxyethoxy)ethoxy)benzo[1,4]oxazin-7-amino)pyrimidin-5-carboxamide (89)

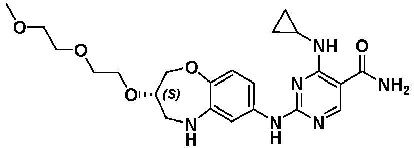

The compound of this example can be prepared according to the similar steps of the aforementioned method for preparing example 26, except that the intermediate (S)-7-amino-2,3,4,5-tetrahydro-3-(2-(2-methoxyethoxy)ethoxy)benzo[1,4]oxazine was used.
ESI-MS: [M+H]⁺ = 427.2.

### Example 90: 4-(1-hydroxyadamantan-4-amino)-2-((S)-2,3,4,5-tetrahydro-3-methoxybenzo[1,4]oxazin-7-amino)pyrimidin-5-carboxamide (90)

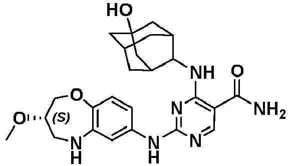

The compound of this example can be prepared according to the similar steps of the aforementioned method for preparing example 26, except that the intermediate (S)-7-amino-2,3,4,5-tetrahydro-3-methoxybenzo[1,4]oxazine was used.
ESI-MS: [M+H]⁺ = 481.2.
¹H-NMR (300 MHz, DMSO-*d*₆) δ: ppm 10.50 (s, 1H), 8.57 (s, 1H), 8.29 (br, 1H), 7.71 (br, 1H), 6.93 (br, 1H), 6.73 (d, J = 8.6, 1H), 6.72 (s, 1H), 4.24-4.19 (m, 1H), 4.11-4.06 (m, 2H), 3.65-3.62 (m, 1H), 3.45-3.37 (m, 1H), 3.30 (s, 3H), 3.26-3.19 (m, 1H), 2.12-2.07 (m, 3H), 1.74-1.65 (m, 8H), 1.50-1.46 (m, 2H).

### Example 91: 4-cyclopropylamino-2-((S)-2,3,4,5-tetrahydro-3-ethoxycarbonylmethoxybenzo[1,4]oxazin-7-amino)pyrimidin-5-carboxamide (91)

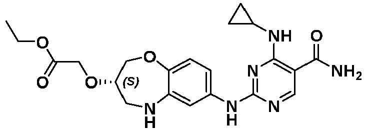

The compound of this example can be prepared according to the similar steps of the aforementioned method for preparing example 26, except that the intermediate (S)-7-amino-2,3,4,5-tetrahydro-3-ethoxycarbonylmethoxybenzo[1,4]oxazine was used.
ESI-MS: [M+H]⁺ = 443.5.
¹H-NMR (300 MHz, CD₃OD-*d*₄) δ: ppm 8.27 (s, 1H), 6.89-6.92 (m, 3H), 4.20-4.37 (m, 4H), 3.88-3.95 (m, 1H), 3.52-3.58 (m, 1H), 3.40-3.47 (m, 1H), 3.04-3.11 (m, 1H), 1.27-1.33 (m, 3H), 0.95-0.97 (m, 2H), 0.92-0.94 (m, 2H).

### Example 92: 4-cyclopropylamino-2-((S)-2,3,4,5-tetrahydro-3-(2-hydroxyethyl)benzo[1,4]oxazin-7-amino)pyrimidin-5-carboxamide (92)

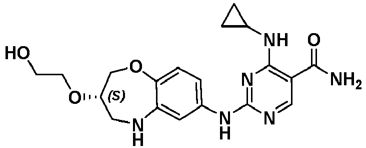

The compound of this example can be prepared according to the similar steps of the aforementioned method for preparing example 26, except that the intermediate (S)-7-amino-2,3,4,5-tetrahydro-3-(2-hydroxyethyl)benzo[1,4]oxazine was used.
ESI-MS: [M+H]⁺ = 401.2.
¹H-NMR (300 MHz, CD₃OD) δ: ppm 8.30 (s, 1H), 6.93 (m, 3H), 4.28 (m, 2H), 3.74 (m, 1H), 3.63 (m, 4H), 3.48 (m, 2H), 3.07 (m, 1H), 0.97 (m, 2H), 0.70 (m, 2H).

### Example 93: 4-(1-methoxyadamantan-4-amino)-2-((S)-2,3,4,5-tetrahydro-3-methoxybenzo[1,4]oxazin-7-amino)pyrimidin-5-carboxamide (93)

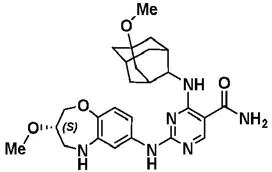

The compound of this example can be prepared according to the similar steps of the aforementioned method for preparing example 26, except that the intermediate (S)-7-amino-2,3,4,5-tetrahydro-3-methoxybenzo[1,4]oxazine was used.
ESI-MS: [M+H]⁺ = 495.6.
¹H-NMR (300 MHz, DMSO-*d*₆) δ: ppm 8.32 (s, 1H), 6.85-6.88 (m, 1H), 6.78 (s, 2H), 4.19-4.33 (m, 3H), 3.70-3.74 (m, 1H), 3.52-3.53 (m, 1H), 3.38-3.48 (m, 5H), 3.28 (s, 3H), 2.21-2.28 (m, 3H), 1.86-1.89 (m, 8H), 1.60-1.65 (m, 2H).

### Example 94: 4-cyclopropylamino-2-((S)-2,3,4,5-tetrahydro-3-carboxymethoxybenzo[1,4]oxazin-7-amino)pyrimidin-5-carboxamide (94)

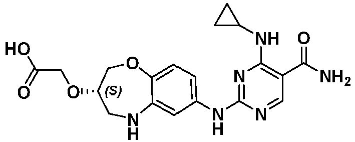

The compound of this example can be prepared according to the similar steps of the aforementioned method for preparing example 26, except that the intermediate (S)-7-amino-2,3,4,5-tetrahydro-3-carboxymethoxybenzo[1,4]oxazine was used.
ESI-MS: [M+H]⁺ = 415.4.
¹H-NMR (300 MHz, CD₃OD) δ: ppm 8.29 (s, 1H), 6.89-6.97 (m, 3H), 4.29-4.31 (m, 2H), 4.24 (s, 2H), 3.90-3.97 (m, 2H), 3.05-3.12 (m, 1H), 0.91-0.97 (m, 2H), 0.68-0.73 (m, 2H).

### Example 95: 4-cyclopropylamino-2-((S)-2,3,4,5-tetrahydro-3-(2-ethoxyethoxy)benzo[1,4]oxazin-7-amino)pyrimidin-5-carboxamide (95)

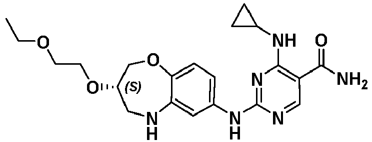

The compound of this example can be prepared according to the similar steps of the aforementioned method for preparing example 26, except that the intermediate (S)-7-amino-2,3,4,5-tetrahydro-3-(2-ethoxyethoxy)benzo[1,4]oxazine was used.
ESI-MS: [M+H]⁺ = 429.2.
¹H-NMR (300 MHz, DMSO-*d*₆) δ: ppm 10.29 (s, 1H), 9.73 (d, J = 3.2 Hz, 1H), 8.46 (s, 1H), 8.05 (s, 1H), 7.55 (s, 1H), 7.01 (s, 2H), 6.74 (d, J = 8.7 Hz, 1H), 4.13 (ddd, J = 49.6, 12.6, 3.9 Hz, 2H), 3.78 (dd, J = 6.6, 4.9 Hz, 1H), 3.49 (m, 7H), 3.21 (dd, J = 13.2, 7.3 Hz, 1H), 2.99 (td, J = 7.2, 3.7 Hz, 1H), 1.09 (m, 3H), 0.84 (m, 2H), 0.63 (m, 2H).

### Example 96: 4-cyclopropylamino-2-((S)-2,3,4,5-tetrahydro-3-(2-isopropoxyethoxy)benzo[1,4]oxazin-7-amino)pyrimidin-5-carboxamide (96)

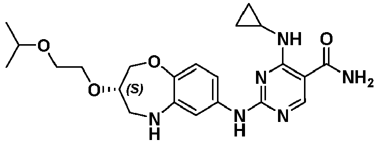

The compound of this example can be prepared according to the similar steps of the aforementioned method for preparing example 26, except that the intermediate (S)-7-amino-2,3,4,5-tetrahydro-3-(2-isopropoxyethoxy)benzo[1,4]oxazine was used.
ESI-MS: [M+H]⁺ = 443.2.
¹H-NMR (300 MHz, CD₃OD) δ: ppm 8.28 (s, 1H), 6.87 (m, 3H), 3.88 (m, 1H), 3.69 (m, 2H), 3.63 (m, 2H), 3.40 (m, 1H), 3.32 (m, 1H), 3.09 (m, 1H), 1.16 (m, 6H), 0.96 (m, 2H), 0.71 (m, 2H).

### Example 97: 4-cyclopropylamino-2-((S)-2,3,4,5-tetrahydro-3-(tetrahydrofuran-2-methoxy)benzo[1,4]oxazin-7-amino)pyrimidin-5-carboxamide (97)

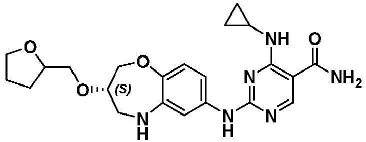

The compound of this example can be prepared according to the similar steps of the aforementioned method for preparing example 26, except that the intermediate (S)-7-amino-2,3,4,5-tetrahydro-3-(tetrahydrofuran-2-methoxy)benzo[1,4]oxazine was used.
ESI-MS: [M+H]⁺ = 441.5.
¹H-NMR (300 MHz, DMSO-*d*₆) δ: ppm 9.88 (s, 1H), 9.43 (s, 1H), 8.46 (s, 1H), 7.02-7.05 (m, 2H), 6.68-6.70 (m, 1H), 4.21-4.22 (m, 1H), 3.93-4.04 (m, 4H), 3.15-3.19 (m, 2H), 2.91-2.97 (m, 2H), 1.79-1.89 (m, 4H), 1.51-1.59 (m, 2H), 0.79-0.86 (m, 2H), 0.53-0.58 (m, 2H).

### Example 98: 4-cyclopropylamino-2-((S)-2,3,4,5-tetrahydro-3-(3-methoxypropoxy)benzo[1,4]oxazin-7-amino)pyrimidin-5-carboxamide (98)

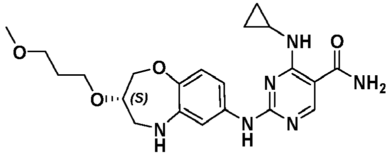

The compound of this example can be prepared according to the similar steps of the aforementioned method for preparing example 26, except that the intermediate (S)-7-amino-2,3,4,5-tetrahydro-3-(3-methoxypropoxy)benzo[1,4]oxazine was used.
ESI-MS: [M+H]⁺ = 429.2.
¹H-NMR (300 MHz, CD₃OD) δ: ppm 8.27 (s, 1H), 6.91 (m, 3H), 3.81(m, 1H), 3.60 (m, 2H), 3.48 (m, 2H), 3.35 (s, 3H), 3.32 (m, 4H), 3.08 (m, 1H), 1.89 (m, 2H), 0.97 (m, 2H), 0.70 (m, 2H).

### Example 99: 4-cyclopropylamino-2-((S)-2,3,4,5-tetrahydro-3-(tetrahydro-2H-2-pyranylmethoxy)benzo[1,4]oxazin-7-amino)pyrimidin-5-carboxamide (99)

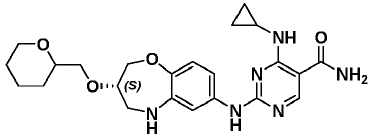

The compound of this example can be prepared according to the similar steps of the aforementioned method for preparing example 26, except that the intermediate (S)-7-amino-2,3,4,5-tetrahydro-3-(tetrahydrogen-2H-2-pyranylmethoxy)benzo[1,4]oxazine was used.
ESI-MS: [M+H]⁺ = 455.5.
¹H-NMR (300 MHz, CD₃OD) δ: ppm 8.37 (s, 1H), 7.17-7.18 (m, 1H), 7.07-7.11 (m, 1H), 6.74-6.77 (m, 1H), 4.24-4.30 (m, 2H), 4.01-4.11 (m, 1H), 3.95-4.00 (m, 2H), 3.74-3.82 (m, 1H), 3.40-3.60 (m, 5H), 2.90-2.96 (m, 1H), 1.49-1.65 (m, 4H), 0.85-0.94 (m, 4H), 0.58-0.60 (m, 2H).

### Example 100: 4-cyclopropylamino-2-((S)-2,3,4,5-tetrahydro-3-(4-methoxybutoxy)benzo[1,4]oxazin-7-amino)pyrimidin-5-carboxamide (100)

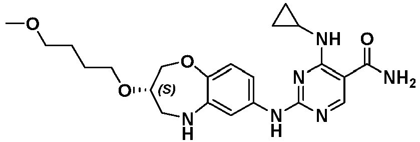

The compound of this example can be prepared according to the similar steps of the aforementioned method for preparing example 26, except that the intermediate (S)-7-amino-2,3,4,5-tetrahydro-3-(4-methoxybutoxy)benzo[1,4]oxazine was used.
ESI-MS: [M+H]⁺ = 443.2.

### Example 101: 4-cyclopropylamino-2-((S)-2,3,4,5-tetrahydro-3-(3-methyloxetan-3-methoxy)benzo[1,4]oxazin-7-amino)pyrimidin-5-carboxamide (101)

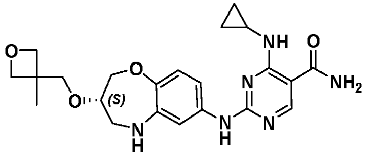

The compound of this example can be prepared according to the similar steps of the aforementioned method for preparing example 26, except that the intermediate (S)-7-amino-2,3,4,5-tetrahydro-3-(3-methyloxetan-3-methoxy)benzo[1,4]oxazine was used.
ESI-MS: [M+H]⁺ = 441.5.
¹H-NMR (300 MHz, DMSO-*d*₆) δ: ppm 10.25 (s, 1H), 9.72 (s, 1H), 8.45-8.57 (m, 1H), 8.13 (s, 1H), 7.56 (s, 1H), 6.92-7.05 (m, 2H), 6.73-6.76 (m, 1H), 4.22-4.39 (m, 2H), 4.05-4.10 (m, 4H), 3.71-3.81 (m, 2H), 3.55-3.60 (m, 1H),3.25-3.28 (m, 2H), 2.95-3.01 (m, 1H), 1.25 (s, 3H), 0.83-0.88 (m, 2H), 0.65-0.70 (m, 2H).

### Example 102: 4-cyclopropylamino-2-((S)-2,3,4,5-tetrahydro-3-(tetrahydropyran-3-methoxy)benzo[1,4]oxazin-7-amino)pyrimidin-5-carboxamide (102)

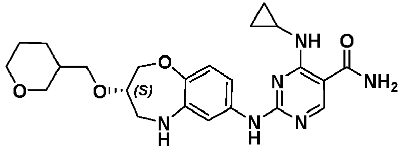

The compound of this example can be prepared according to the similar steps of the aforementioned method for preparing example 26, except that the intermediate (S)-7-amino-2,3,4,5-tetrahydro-3-(3-tetrahydropyran-3-methoxy)benzo[1,4]oxazine was used.
ESI-MS: [M+H]⁺ = 455.2.
¹H-NMR (300 MHz, MeOD-*d*₄) δ: ppm 8.28 (s, 1H), 6.87-6.99 (m, 3H), 4.14-4.32 (m, 2H), 3.94-3.98 (m, 1H), 3.74-3.87 (m, 2H), 3.37-3.52 (m, 5H), 3.29-3.30 (m, 1H), 3.04-3.12 (m, 1H), 1.81-1.96 (m, 2H), 1.59-1.68 (m, 2H), 1.13-1.43 (m, 1H), 0.91-0.97 (m, 2H), 0.68-0.73 (m, 2H).

### Example 103: 4-cyclopropylamino-2-((S)-2,3,4,5-tetrahydro-3-(2-tert-butoxyethoxy)benzo[1,4]oxazin-7-amino)pyrimidin-5-carboxamide (103)

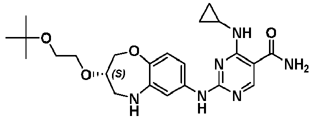

The compound of this example can be prepared according to the similar steps of the aforementioned method for preparing example 26, except that the intermediate (S)-7-amino-2,3,4,5-tetrahydro-3-(2-tert-butoxyethoxy)benzo[1,4]oxazine was used.
ESI-MS: [M+H]⁺ = 457.5.
¹H-NMR (300 MHz, DMSO-*d*₆) δ: ppm 10.32 (br, 1H), 9.75 (s, 1H), 8.45 (s, 1H), 8.08 (br, 1H), 7.57 (s, 1H), 7.00 (s, 2H), 6.73-6.76 (m, 1H), 4.18-4.24 (m, 1H), 4.02-4.07 (m, 1H), 3.76-3.82 (m, 1H), 3.53-3.56 (m, 2H), 3.37-3.44 (m, 3H), 3.17-3.24 (m, 1H), 2.96-3.02 (m, 1H), 1.15 (s, 9H), 0.82-0.88 (m, 2H), 0.60-0.65 (m, 2H).

### Assay Examples: Determination of inhibition of TYK2 kinase activity by the examples compounds disclosed herein

Inhibition assay of TYK2 kinase activity: the method of ADP-Glo^{TM} Kinase Assay (Promega, V9102) was used to determine the inhibitory activity of the test compounds against TYK2 kinase.

TYK2 kinase (purchased from Life Technologies, article number: PR8440C) was diluted to the 2-fold final concentration (0.8 ng/mL) using reaction buffer (40mM Tris-HCl, pH 7.5; 20 mM MgCl₂; 0.1 mg/mL BSA; 1 mM DTT), and added to a 384-well plate at 5 µL/well. The test drugs were subjected to a 10-fold gradient dilution, from 10 µM, to obtain 6 concentrations, and added to experimental wells of the 384-well plate at 2.5 µL/well. 5 µL of water and 2.5 µL of buffer were added to negative control wells. 5 µL of TYK2 and 2.5 µL of water were added to positive control wells. After incubating for 10 minutes at room temperature, the substrate ATP and polyE4Y1 (Sigma, P0275) were added at 2.5 µL/well, mixed well, and then reacted at room temperature for 60 minutes. After the reaction was completed, the kinase activity was detected according to the instruction of the kit. First, the ADP-Glo reaction reagent was added at 10 µL/well, mixed well, and reacted at room temperature for 40 minutes. The kinase detection reagent was then added at 20 µL/well, mixed well, and then reacted at room temperature for 30 minutes. The detection was then performed and the IC₅₀s of test drugs were calculated.

The results of assay were shown in Table 2.

**Table 2**

| Example Compounds | Activity IC₅₀ (nM) | Example Compounds | Activity IC₅₀ (nM) |
|---|---|---|---|
| 1 | A | 55 | A |
| 2 | A | 56 | A |
| 3 | A | 57 | A |
| 4 | A | 58 | B |
| 5 | B | 59 | - |
| 6 | A | 60 | A |
| 7 | A | 61 | A |
| 8 | A | 62 | B |
| 9 | A | 63 | - |
| 10 | B | 64 | A |
| 11 | B | 65 | A |
| 12 | B | 66 | A |
| 13 | A | 67 | A |
| 14 | A | 68 | A |
| 15 | B | 69 | A |
| 16 | B | 70 | A |
| 17 | A | 71 | A |
| 18 | B | 72 | A |
| 19 | B | 73 | A |
| 20 | A | 74 | A |
| 21 | A | 75 | A |
| 22 | B | 76 | B |
| 23 | A | 77 | A |
| 24 | A | 78 | A |
| 25 | A | 79 | A |
| 26 | A | 80 | A |
| 27 | A | 81 | A |
| 28 | A | 82 | A |
| 29 | A | 83 | A |
| 30 | A | 84 | B |
| 31 | - | 85 | A |
| 32 | B | 86 | A |
| 33 | A | 87 | A |
| 34 | A | 88 | B |
| 35 | A | 89 | A |
| 36 | A | 90 | A |
| 37 | A | 91 | A |
| 38 | A | 92 | A |
| 39 | A | 93 | A |
| 40 | A | 94 | A |
| 41 | A | 95 | A |
| 42 | A | 96 | A |
| 43 | A | 97 | A |
| 44 | A | 98 | A |
| 45 | B | 99 | A |
| 46 | A | 100 | A |
| 47 | A | 101 | A |
| 48 | A | 102 | A |
| 49 | A | 103 | A |
| 50 | A | | |
| 51 | A | | |
| 52 | A | | |
| 53 | A | | |
| 54 | A | | |

| | | | |
|---|---|---|---|
| wherein, A means IC₅₀ value < 10 nM; B means 10 nM <IC₅₀ value <100 nM. | | | |

From the results of the above assay example, it can be seen that the various novel pyrimidinylamide compounds in examples disclosed herein have significant TYK2 kinase inhibitory activity.

Inhibition assay of JAK1 kinase activity: the method in ADP-Glo^{TM} Kinase Assay (Promega, V9102) was used to determine the inhibitory activity of the test compounds against JAK1 kinase.

The results of assay were shown in Table 3.

**Table 3**

| Example Compounds | Activity IC₅₀ (nM) | Example Compounds | Activity IC₅₀ (nM) |
|---|---|---|---|
| 1 | A | 2 | A |
| 3 | A | 4 | B |
| 5 | B | 6 | B |
| 7 | B | 8 | B |
| 9 | A | 14 | B |
| 17 | B | 20 | B |
| 24 | B | 25 | B |
| 26 | A | 27 | A |
| 29 | A | 30 | B |
| 32 | B | 33 | B |
| 34 | A | 35 | B |
| 36 | B | 37 | A |
| 38 | A | 39 | B |
| 40 | B | 41 | A |
| 42 | B | 43 | A |
| 44 | A | 45 | B |
| 46 | A | 47 | B |
| 48 | A | 49 | A |
| 50 | B | 51 | B |
| 52 | B | 53 | B |
| 54 | B | 55 | A |
| 56 | A | 57 | A |
| 58 | B | 60 | A |
| 61 | B | 62 | B |
| 64 | B | 65 | A |
| 66 | A | 67 | B |
| 68 | A | 69 | A |
| 70 | A | 72 | A |
| 73 | A | 74 | A |
| 75 | A | 76 | A |
| 78 | A | 79 | A |
| 80 | B | 81 | B |
| 82 | A | 84 | B |
| 86 | B | 87 | A |
| 89 | A | 91 | A |
| 92 | A | 93 | A |
| 94 | A | 95 | A |
| 96 | A | 97 | A |
| 98 | A | 99 | A |
| 100 | A | 101 | A |
| 102 | A | 103 | A |

| | | | |
|---|---|---|---|
| wherein, A means IC₅₀ value <10 nM; B means 10 nM <IC₅₀ value <100 nM. | | | |

From the results of the above assay example, it can be seen that the various novel pyrimidinylamide compounds in examples disclosed herein have significant JAK1 kinase inhibitory activity.

Inhibition assay of JAK2 kinase activity: the method in ADP-Glo^{TM} Kinase Assay (Promega, V9102) was used to determine the inhibitory activity of the test compounds against JAK2 kinase.

The results of assay were shown in Table 4.

**Table 4**

| Example Compounds | Activity IC₅₀ (nM) | Example Compounds | Activity IC₅₀ (nM) |
|---|---|---|---|
| 2 | B | 7 | B |
| 9 | B | 24 | B |
| 25 | A | 26 | A |
| 27 | B | 34 | A |
| 38 | A | 44 | A |
| 46 | A | 48 | A |
| 49 | A | 50 | A |
| 51 | B | 53 | B |
| 54 | B | 55 | A |
| 56 | A | 57 | B |
| 60 | B | 61 | B |
| 64 | B | 65 | B |
| 66 | B | 67 | B |
| 68 | A | 69 | B |
| 70 | A | 72 | A |
| 73 | A | 74 | A |
| 75 | A | 76 | A |
| 78 | B | 79 | B |
| 80 | A | 82 | B |
| 84 | B | 86 | A |
| 87 | A | 89 | B |
| 90 | A | 91 | B |
| 92 | A | 93 | A |
| 94 | A | 95 | B |
| 96 | B | 97 | B |
| 98 | B | 101 | B |
| 102 | B | 103 | B |

| | | | |
|---|---|---|---|
| wherein, A means IC₅₀ value < 10nM; B means 10 nM < IC₅₀ value < 100 nM. | | | |

From the results of the above assay example, it can be seen that the various novel pyrimidinylamide compounds in examples disclosed herein have significant JAK2 kinase inhibitory activity.

Inhibition assay of JAK3 kinase activity: the method in ADP-Glo^{TM} Kinase Assay (Promega, V9102) was used to determine the inhibitory activity of the test compounds against JAK3 kinase.

The results of assay were shown in Table 5.

**Table 5**

| Example Compounds | Activity IC₅₀ (nM) | Example Compounds | Activity IC₅₀ (nM) |
|---|---|---|---|
| 2 | A | 24 | B |
| 25 | A | 34 | A |
| 38 | A | 44 | A |
| 46 | A | 50 | A |
| 51 | B | 53 | B |
| 54 | B | 55 | A |
| 56 | A | 57 | B |
| 60 | B | 61 | B |
| 64 | B | 65 | B |
| 66 | B | 67 | B |
| 68 | B | 69 | B |
| 70 | A | 72 | A |
| 73 | A | 74 | A |
| 75 | A | 76 | A |
| 77 | B | 78 | B |
| 79 | B | 80 | A |
| 81 | B | 82 | B |
| 86 | B | 87 | B |
| 89 | B | 90 | A |
| 91 | B | 92 | A |
| 93 | A | 94 | B |
| 95 | A | 96 | B |
| 97 | B | 98 | B |
| 101 | B | 102 | B |
| 103 | B | | |

| | | | |
|---|---|---|---|
| wherein, A means IC₅₀ value < 10 nM; B means 10 nM < IC₅₀ value < 100 nM. | | | |

From the results of the above assay example, it can be seen that the various novel pyrimidinylamide compounds in examples disclosed herein have significant JAK3 kinase inhibitory activity.

Inhibition assay of SYK kinase activity: the method in ADP-Glo^{TM} Kinase Assay (Promega, V9102) was used to determine the inhibitory activity of the test compounds against SYK kinase.

The results of assay were shown in Table 6.

**Table 6**

| Example Compounds | Activity IC₅₀ (nM) | Example Compounds | Activity IC₅₀ (nM) |
|---|---|---|---|
| 1 | B | 24 | B |
| 25 | B | 44 | B |
| 57 | B | 60 | B |
| 66 | B | 68 | B |
| 69 | B | 72 | B |
| 76 | B | 78 | B |
| 79 | B | 80 | B |
| 86 | B | 87 | B |
| 89 | B | 90 | B |
| 91 | B | 92 | B |
| 94 | B | 95 | B |
| 96 | B | 97 | B |
| 98 | B | 99 | B |
| 100 | B | 101 | B |

| | | | |
|---|---|---|---|
| wherein, A means IC₅₀ value < 10 nM; B means 10 nM < IC₅₀ value < 100 nM. | | | |

From the results of the above assay example, it can be seen that the various novel pyrimidinylamide compounds in examples disclosed herein have significant SYK kinase inhibitory activity.

## Claims

1. A compound selected from the group consisting of a compound represented by the following general formula I, a stereoisomer thereof, a tautomer thereof, a pharmaceutically acceptable salt thereof, a solvate thereof, and a prodrug thereof, 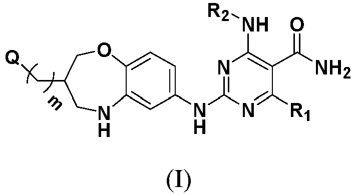 wherein,
Q is selected from carboxyl, cyano, ORₐ, NHR_{b} or SO₂R_{c};
R₁ is selected from hydrogen or C₁-C₃ alkyl;
R₂ is selected from substituted or unsubstituted linear or branched alkyl, substituted or unsubstituted cycloalkyl, substituted or unsubstituted heterocyclyl, substituted or unsubstituted aryl, or substituted or unsubstituted heteroaryl;
wherein the substituent is selected from halogen, trifluoromethyl, cyano, hydroxyl, carboxyl, linear or branched C₁-C₆ alkyl, C₃-C₁₀ cycloalkyl, C₁-C₆ alkoxyalkyl, C₁-C₆ alkylaminoalkyl, C₁-C₆ alkylsulfonylalkyl, C₃-C₈ heterocyclyl, C₆-C₁₂ aryl or C₄-C₁₂ heteroaryl;
Rₐ, R_{b}, and R_{c} are each independently selected from hydrogen, linear or branched C₁-C₄ alkyl, or C₃-C₈ cycloalkyl;
m is 0 or 1.

2. The compound selected from the group consisting of a compound, a stereoisomer thereof, a tautomer thereof, a pharmaceutically acceptable salt thereof, a solvate thereof, and a prodrug thereof according to claim 1, wherein
Q is selected from ORₐ, or NHR_{b};
R₁ is selected from hydrogen or C₁-C₃ alkyl;
R₂ is selected from substituted or unsubstituted linear or branched C₁-C₄ alkyl, substituted or
unsubstituted C₃-C₁₀ cycloalkyl, substituted or unsubstituted C₃-C₆ heterocyclyl, substituted or unsubstituted C₆-C₁₀ aryl, or substituted or unsubstituted C₄-C₁₀ heteroaryl;
wherein the substituent is selected from halogen, trifluoromethyl, cyano, hydroxyl, carboxyl, linear or branched C₁-C₆ alkyl, C₃-C₁₀ cycloalkyl, C₁-C₆ alkoxyalkyl, C₁-C₆ alkylaminoalkyl, C₁-C₆ alkylsulfonylalkyl, C₃-C₈ heterocyclyl, C₆-C₁₀ aryl, or C₄-C₁₀ heteroaryl;
Rₐ, and R_{b} are each independently selected from hydrogen, linear or branched C₁-C₄ alkyl, or C₃-C₆ cycloalkyl;
m is 0 or 1.

3. The compound according to claim 1 or 2, wherein
Q is selected from ORₐ, or NHR_{b};
Rₐ, and R_{b} are each independently selected from hydrogen, linear or branched C₁-C₄ alkyl, or C₃-C₆ cycloalkyl;
R₁ is selected from hydrogen or methyl;
m is 0.

4. The compound according to any one of claims 1 to 3, wherein R₂ is selected from the following groups: 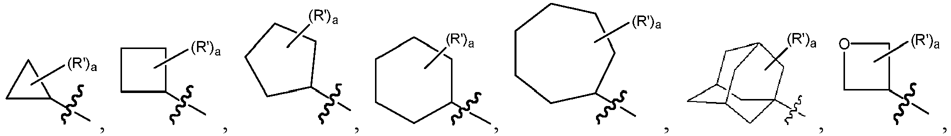 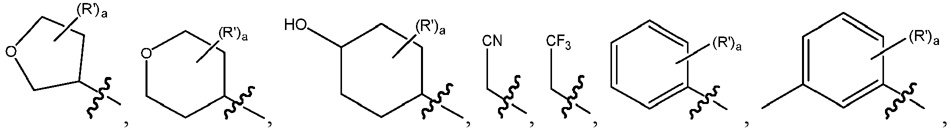 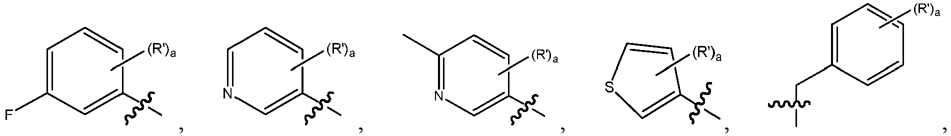 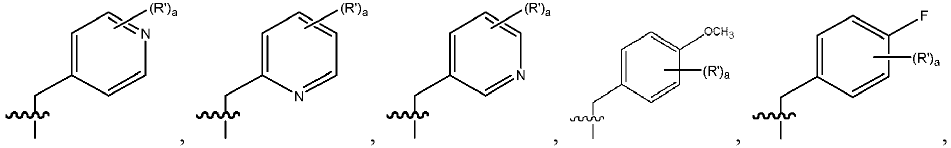 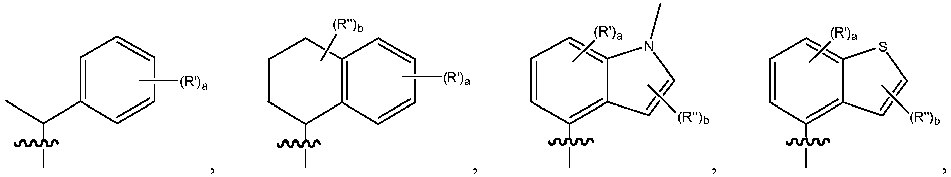 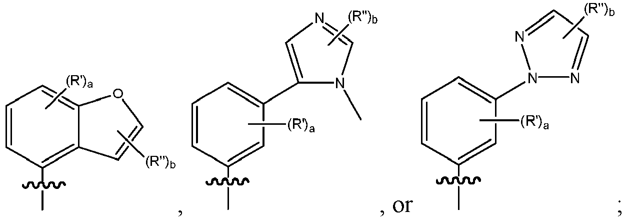
R', and R" are each independently selected from hydrogen, halogen, trifluoromethyl, cyano, hydroxyl, carboxyl, linear or branched C₁-C₆ alkyl, C₃-C₁₀ cycloalkyl, C₁-C₆ alkoxy, C₁-C₆ alkylamino, C₁-C₆ alkylsulfonyl, C₃-C₈ heterocyclyl, C₆-C₁₀ aryl, or C₄-C₁₀ heteroaryl;
a is an integer from 1 to 5, and b is an integer from 1 to 5.

5. The compound according to claim 1, wherein the compound is selected from the group consisting of compounds represented by the following structural formulae:

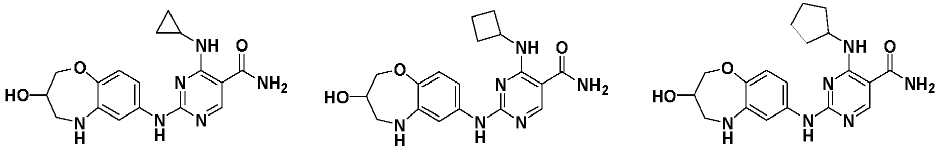

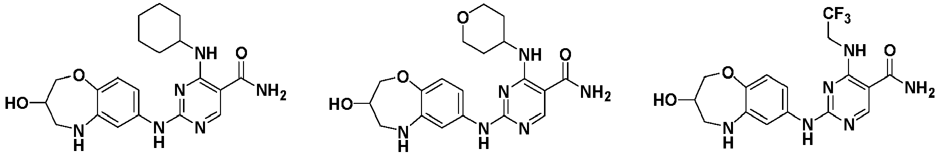

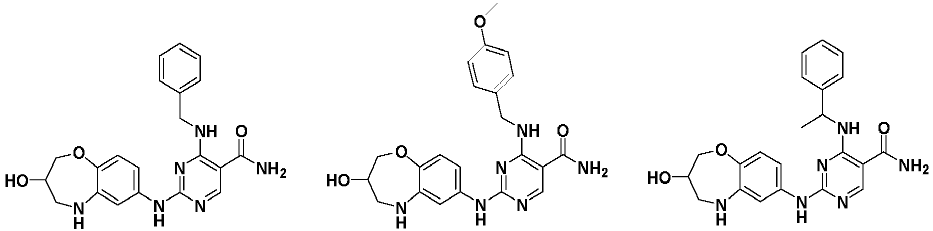

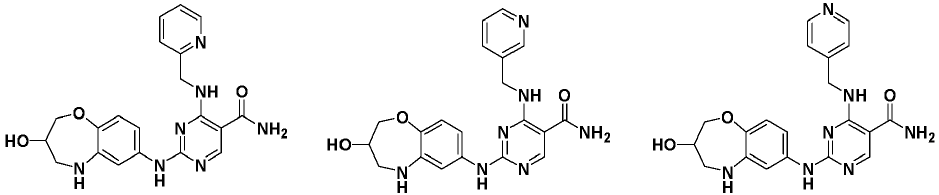

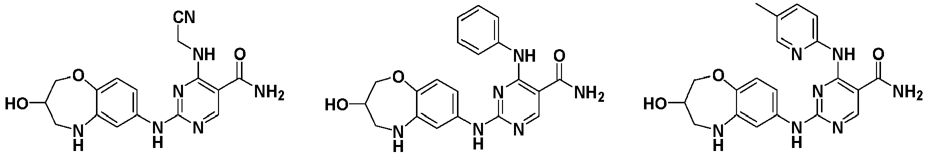

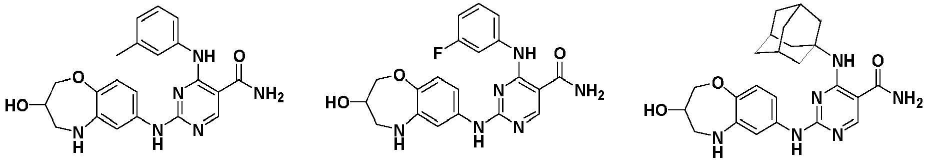

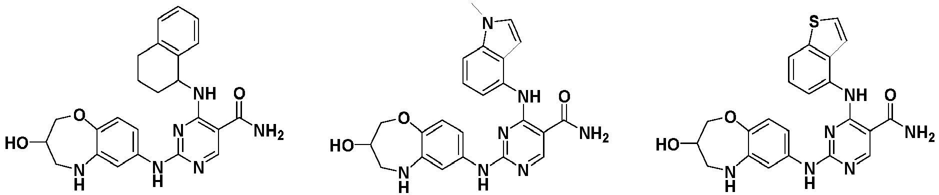

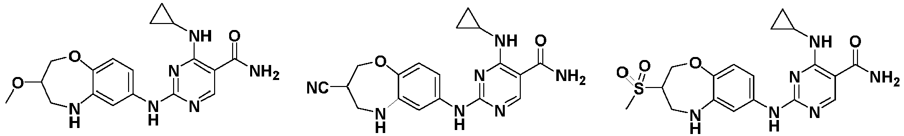

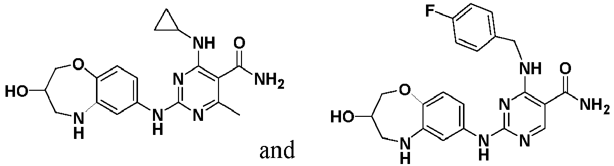

6. A compound selected from the group consisting of a compound represented by the following general formula I, a stereoisomer thereof, a tautomer thereof, a pharmaceutically acceptable salt thereof, a solvate thereof, and a prodrug thereof, 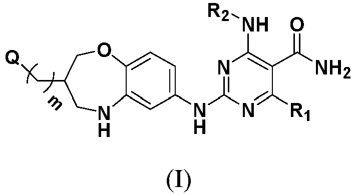 wherein,
Q is selected from hydrogen, carboxyl, cyano, ORₐ, NHR_{b} or SO₂R_{c};
R₁ is selected from hydrogen or C₁-C₃ alkyl;
R₂ is selected from substituted or unsubstituted linear or branched alkyl, substituted or unsubstituted cycloalkyl, substituted or unsubstituted heterocyclyl, substituted or unsubstituted aryl, or substituted or unsubstituted heteroaryl;
wherein the substituent is selected from halogen, trifluoromethyl, cyano, hydroxyl, carboxyl, linear or branched C₁-C₆ alkyl, C₃-C₁₀ cycloalkyl, C₁-C₆ alkoxyalkyl, C₁-C₆ alkylaminoalkyl, C₁-C₆ alkylsulfonylalkyl, C₃-C₈ heterocyclyl, C₆-C₁₂ aryl or C₄-C₁₂ heteroaryl, C₁-C₆ alkylsulfonylaminoalkyl, C₁-C₆ alkenylalkyl, or C₁-C₆ alkynylalkyl;
Rₐ, R_{b}, and R_{c} are each independently selected from hydrogen, C₃-C₈ cycloalkyl, trifluoromethyl, trideuterated methyl, linear or branched C₁-C₆ alkyl, C₁-C₆ alkylaminoalkyl, C₁-C₆ alkoxyalkyl, C₃-C₈ heterocyclylalkyl, C₁-C₆ alkoxycarbonylalkyl, C₁-C₆ hydroxyalkyl, or C₁-C₆ carboxylalkyl;
m is 0 or 1.

7. The compound selected from the group consisting of a compound, a stereoisomer thereof, a tautomer thereof, a pharmaceutically acceptable salt thereof, a solvate thereof, and a prodrug thereof according to claim 6, wherein
Q is selected from hydrogen, ORₐ, or NHR_{b};
R₁ is selected from hydrogen or C₁-C₃ alkyl;
R₂ is selected from substituted or unsubstituted linear or branched C₁-C₄ alkyl, substituted or unsubstituted C₃-C₁₀ cycloalkyl, substituted or unsubstituted C₃-C₆ heterocyclyl, substituted or unsubstituted C₆-C₁₀ aryl, or substituted or unsubstituted C₄-C₁₀ heteroaryl;
wherein the substituent is selected from halogen, trifluoromethyl, cyano, hydroxyl, carboxyl, linear or branched C₁-C₆ alkyl, C₃-C₁₀ cycloalkyl, C₁-C₆ alkoxyalkyl, C₁-C₆ alkylaminoalkyl, C₁-C₆ alkylsulfonylalkyl, C₃-C₈ heterocyclyl, C₆-C₁₀ aryl or C₄-C₁₀ heteroaryl, C₁-C₆ alkylsulfonylaminoalkyl, C₁-C₆ alkenylalkyl, or C₁-C₆ alkynylalkyl;
Rₐ, and R_{b} are each independently selected from hydrogen, C₃-C₆ cycloalkyl, trifluoromethyl, trideuterated methyl, linear or branched C₁-C₆ alkyl, C₁-C₆ alkylaminoalkyl, C₁-C₆ alkoxyalkyl, C₃-C₈ heterocyclylalkyl, C₁-C₆ alkoxycarbonylalkyl, C₁-C₆ alkylaminocarbonylalkyl, C₁-C₆ hydroxyalkyl, or C₁-C₆ carboxylalkyl;
m is 0 or 1.

8. The compound according to claim 6 or 7, wherein
Q is selected from hydrogen, ORₐ, or NHR_{b};
Rₐ and R_{b} are each independently selected from hydrogen, C₃-C₆ cycloalkyl, trifluoromethyl, trideuterated methyl, linear or branched C₁-C₆ alkyl, C₁-C₆ alkylaminoalkyl, C₁-C₆ alkoxyalkyl, C₃-C₈ heterocyclylalkyl, C₁-C₆ alkoxycarbonylalkyl, C₁-C₆ alkylaminocarbonylalkyl, C₁-C₆ hydroxyalkyl, or C₁-C₆ carboxylalkyl;
R₁ is selected from hydrogen or methyl;
m is 0.

9. The compound according to any one of claims 6 to 8, **characterized in that** R₂ is selected from the following groups: 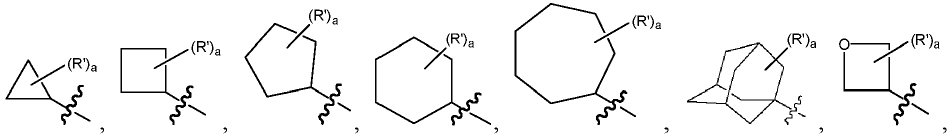 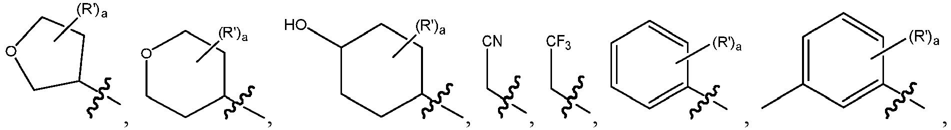 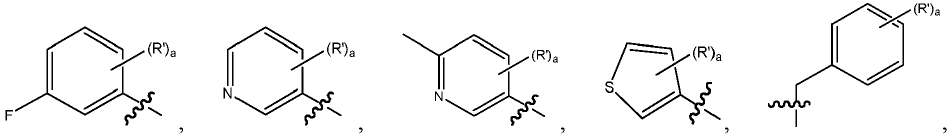 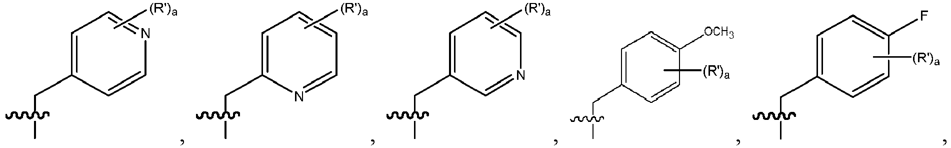 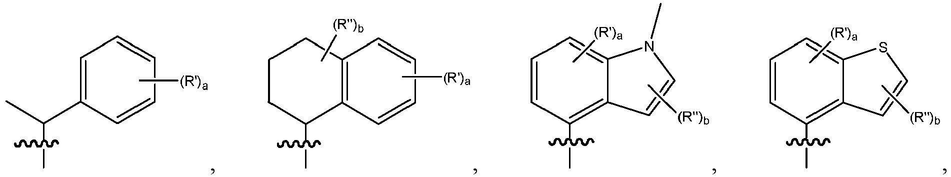 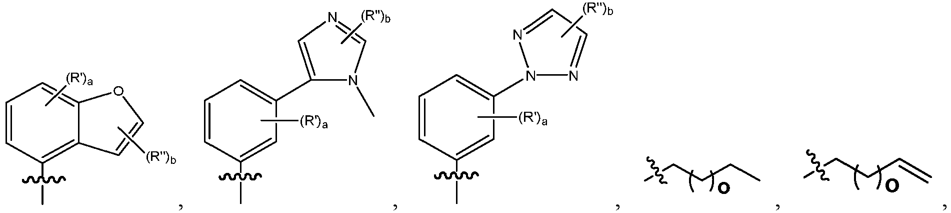 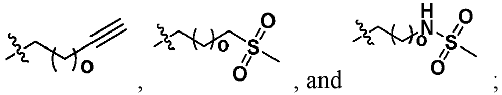
R', and R" are each independently selected from hydrogen, halogen, trifluoromethyl, cyano, hydroxyl, carboxyl, linear or branched C₁-C₆ alkyl, C₃-C₁₀ cycloalkyl, C₁-C₆ alkoxy, C₁-C₆ alkylamino, C₁-C₆ alkylsulfonyl, C₃-C₈ heterocyclyl, C₆-C₁₀ aryl or C₄-C₁₀ heteroaryl;
a is an integer from 1 to 5, b is an integer from 1 to 5, and o is an integer from 1 to 5.

10. The compound according to any one of claims 6 to 9, wherein the compound is selected from the group consisting of compounds represented by the following structural formulae:

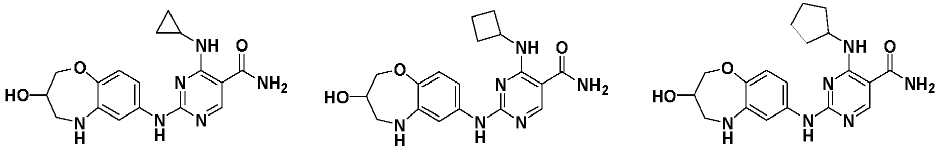

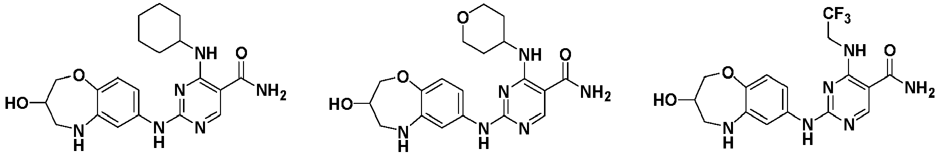

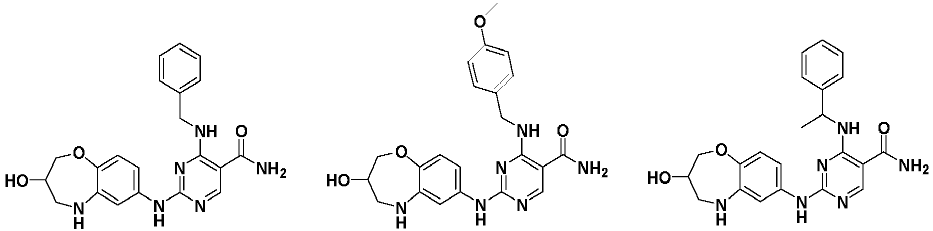

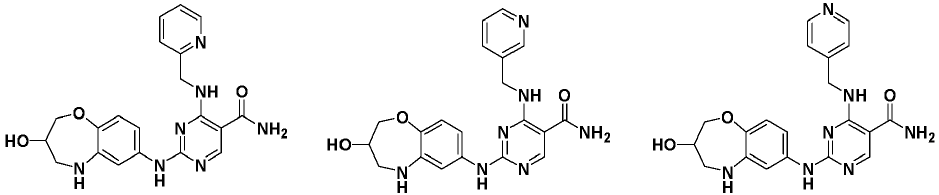

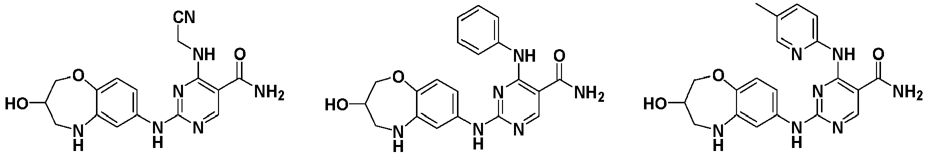

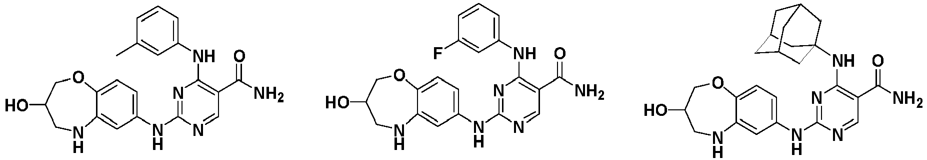

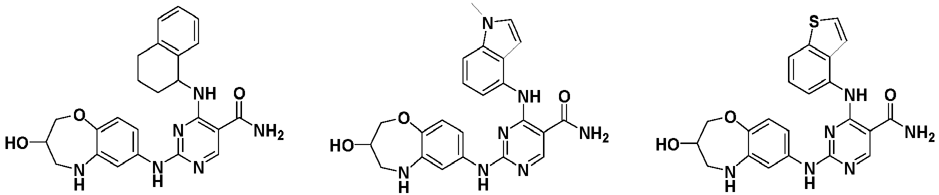

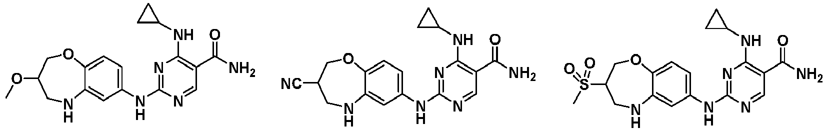

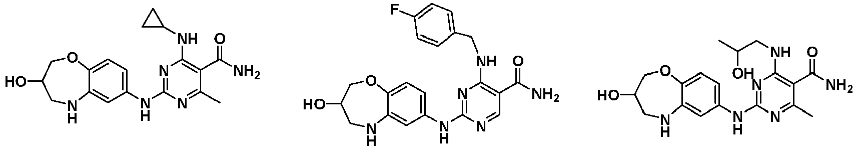

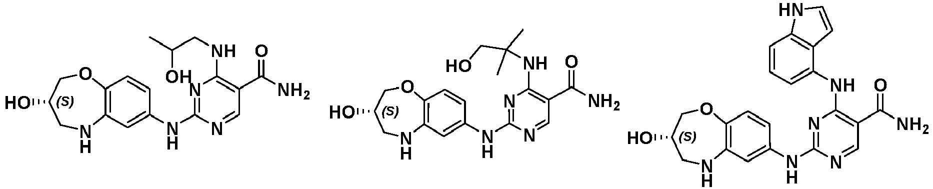

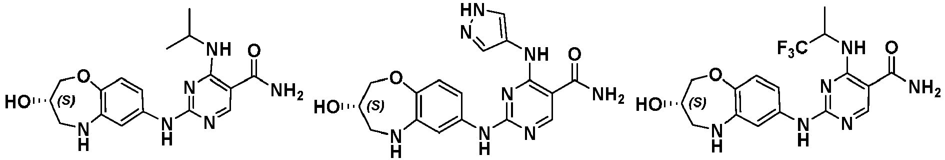

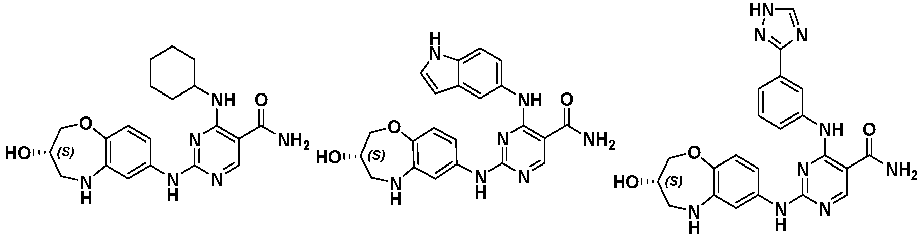

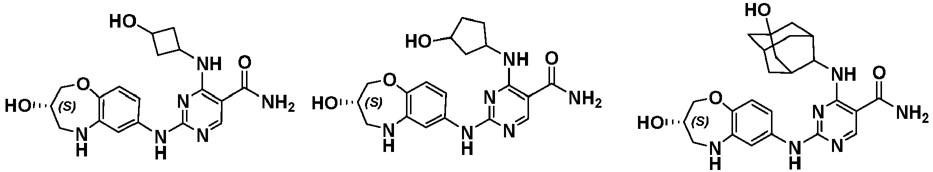

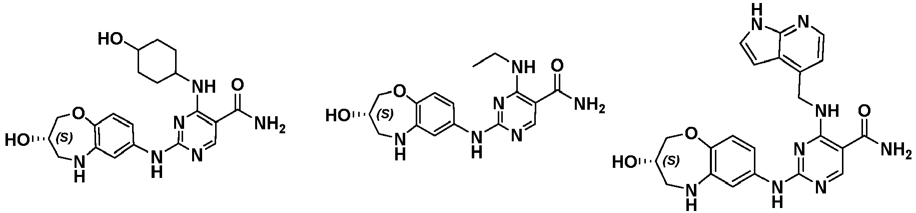

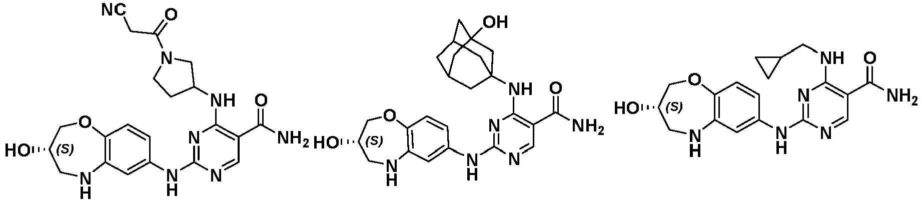

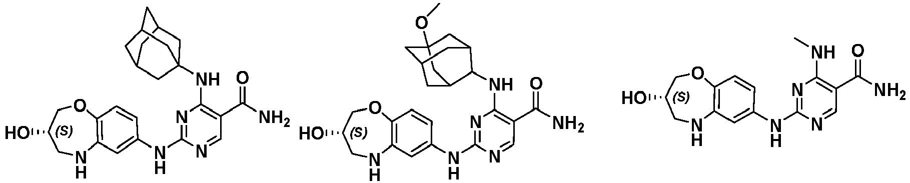

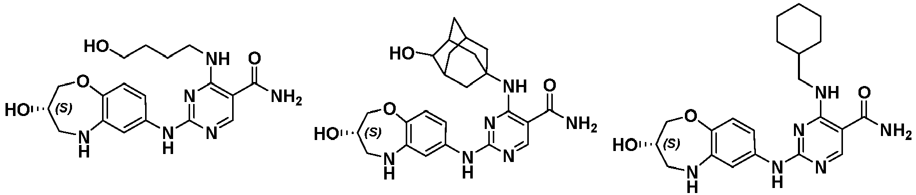

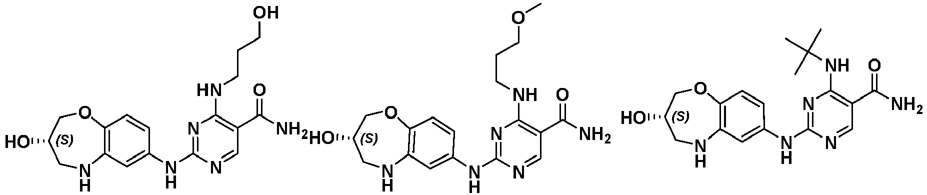

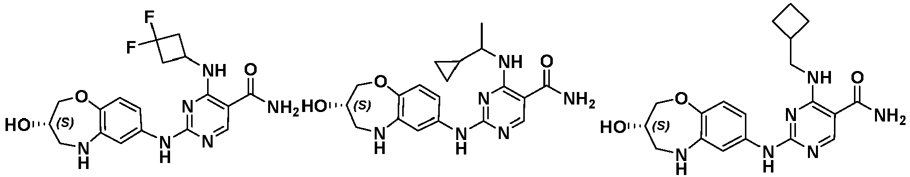

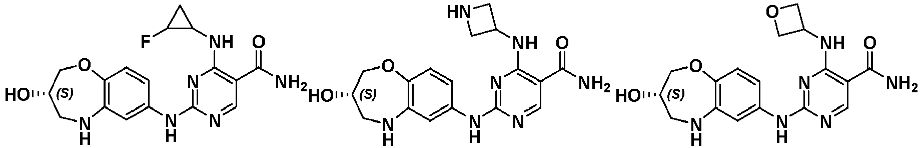

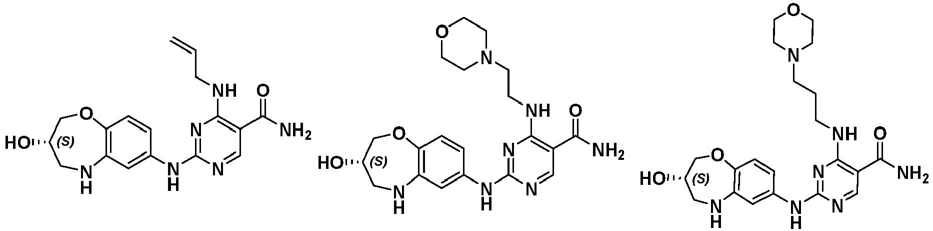

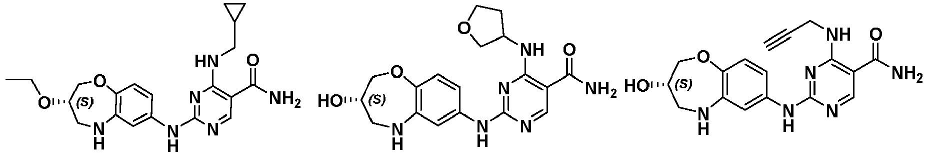

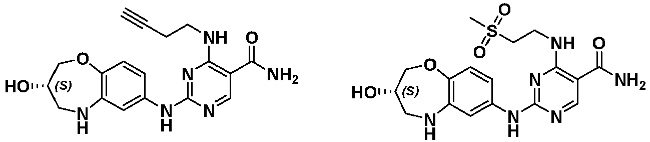

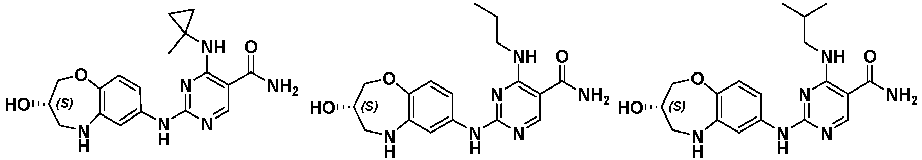

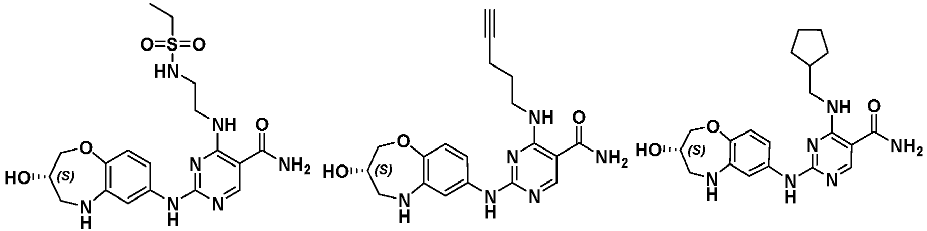

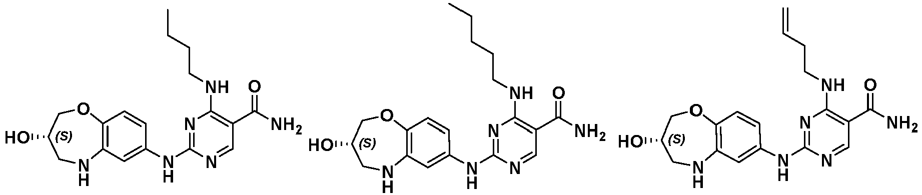

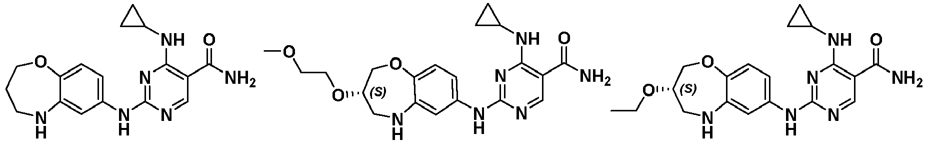

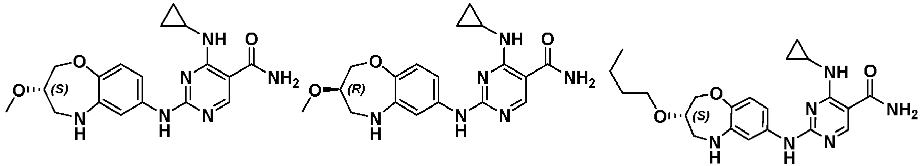

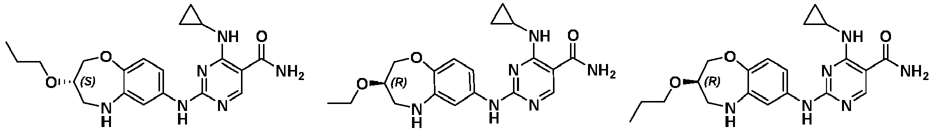

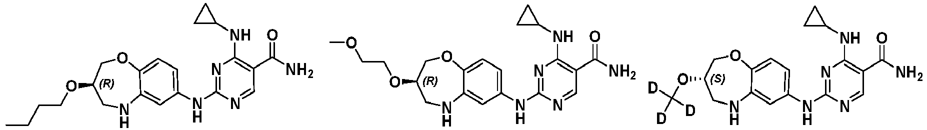

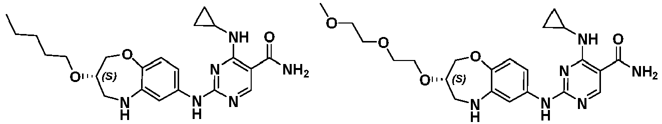

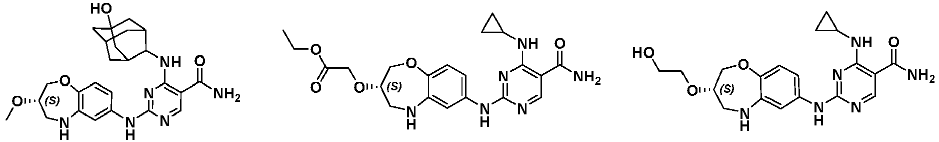

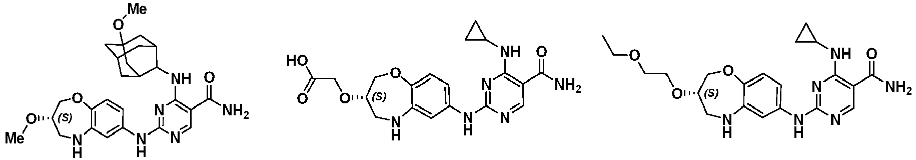

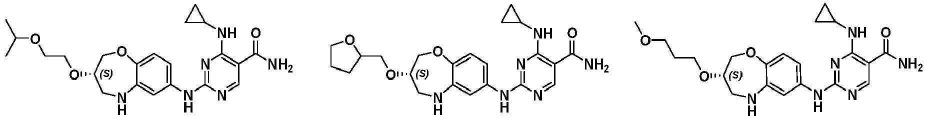

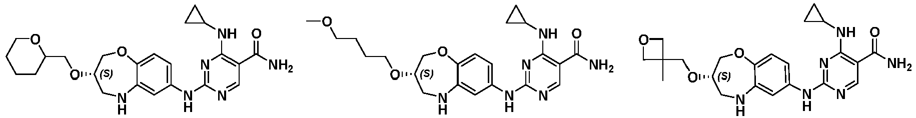

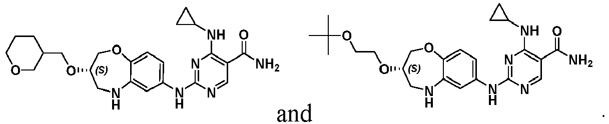

11. A pharmaceutical composition comprising a compound according to any one of claims 1 to 10 and pharmaceutically acceptable excipient(s).

12. The pharmaceutical composition according to claim 11, wherein the pharmaceutical composition further comprises at least one of anti-inflammatory drugs selected from non-steroidal anti-inflammatory drugs, non-selective cyclooxygenase-2 inhibitors, selective cyclooxygenase-2 inhibitors, corticosteroids, tumor necrosis factor receptor antagonists, salicylic esters or salts, immunosuppressants or methotrexate.

13. Use of a compound according to any one of claims 1 to 10 as an inhibitor of TYK2 kinase, JAK1 kinase, JAK2 kinase, JAK3 kinase, or SYK kinase.

14. Use of a compound according to any one of claims 1 to 10 in the manufacture of a medicament for prevention or treatment of a disease associated with TYK2 kinase, JAK1 kinase, JAK2 kinase, JAK3 kinase or SYK kinase.

15. A method for treating a disease by administering to a patient a compound according to any one of claims 1 to 10 or a pharmaceutical composition according to claim 13 or 14 to inhibit the activity of TYK2 kinase, JAK1 kinase, JAK2 kinase, JAK3 kinase, or SYK kinase.

16. The use according to claim 14 or the method according to claim 15, wherein the disease includes an autoimmune disease, an inflammatory disease and a cancer;
the autoimmune disease includes asthma, psoriasis, lupus, multiple sclerosis, allergic rhinitis, atopic dermatitis, contact dermatitis, and delayed allergic reaction;
the inflammatory disease includes inflammatory bowel disease, and rheumatoid arthritis, and the inflammatory bowel disease includes Crohn's disease and ulcerative colitis;
the cancer includes leukemias and solid tumor.
